# EUROPEAN PATENT SPECIFICATION

(11) **EP 4 141 090 B1**
(45) Date of publication and mention of the grant of the patent: **23.08.2023**
(21) Application number: 21194166.1
(22) Date of filing: 31.08.2021
(51) Int. Cl.: C10G 3/00, C10G 2/00, C10G 50/00, C10L 1/06, C10L 1/08, C07C 1/12, C07C 1/20, C07C 11/04, C07C 11/06, C07C 31/125, C07C 5/02, C07C 1/24, C07C 2/76, C07C 29/141, C07C 29/145, C07C 29/32, C07C 29/44, C07C 45/50, C07C 45/29

(54) **METHOD FOR PRODUCING MOTOR FUEL FROM ETHANOL**
VERFAHREN ZUR HERSTELLUNG VON MOTORTREIBSTOFF AUS ETHANOL
PROCÉDÉ DE PRODUCTION DE CARBURANT DE MOTEUR À PARTIR D'ÉTHANOL

(43) Date of publication of application: 01.03.2023
(73) Proprietor: Swedish Biofuels AB, 102 27 Stockholm (SE)
(72) Inventor: GOLUBKOV, Igor, 181 31 Lidingö (SE)
(74) Representative: Brann AB

(56) References cited:
- US-A1- 2005 112 739
- US-A1- 2007 287 873
- US-A1- 2016 362 612

## Description

### Field of the invention

The present invention relates to a method for producing motor fuel, and more particularly gasoline, kerosene, and diesel, from ethanol obtained from a feedstock of mainly plant origin. In addition, intermediate products and by-products from the inventive motor fuel synthesis, that is; alcohols, aldehydes, ketones, ethers, olefins, paraffins and aromatic compounds obtained from feedstock of mainly plant origin, can be used not only in the chemical industry to produce paints or polymers, but also in the pharmaceutical industry, or for the manufacture of cosmetic products.

### State of the art

The rapid growth in the consumption of motor fuel, on the one hand resulting in a significant reduction of oil and gas reserves of our planet, and on the other hand, raising concerns of society regarding deterioration of the environment, stimulates the search for alternative motor fuels. The challenges facing modern industry concerning reducing carbon dioxide emissions to the atmosphere, stimulate research to improve existing methods and create new ones for the manufacture of motor fuel from renewable feedstocks. Currently, the most attractive raw material for the manufacture of alternative motor fuels is biomass, which is produced directly from the carbon dioxide constantly coming to the atmosphere. Recently, a significant number of works have appeared on the methods of processing biomass into motor fuel and, first of all, on the methods of producing kerosene from raw materials of biological origin.

US 8193402 B2, US 8373012 B2, and US 8487149 B2 disclose microorganisms that, by means of fermentation, provide for conversion of carbohydrates isolated from plant biomass both into individual C₂-C₆ alcohols, and into a mixture of C₂-C₆ alcohols. The alcohols obtained, in turn, are dehydrated into the corresponding olefins. The C₂-C₆ olefins obtained both as individual olefins and as mixtures, are oligomerized, yielding C₆-C₂₁ olefins. Then, the C₆-C₂₁ olefins obtained are hydrogenated resulting in a product containing one or a few saturated C₆-C₂₁ alkanes.

WO 2018071905 and US 2020010767 A1 disclose methods and materials for oligomerization of lower olefins, for example C₂-C₈ olefins, into transport fuels, including diesel and/or jet fuel. In some embodiments, tungsten zirconium catalysts are used to perform the oligomerization.

US 10633320 B2 proposes a process for converting crude and/or refined fusel oil mixtures into renewable chemicals of a higher value by means of mixed oxide metal or zeolite catalysts. The patent discloses methods of directing a vaporized stream of crude and/or refined fusel oils through various mixed metal oxide catalysts, metal doped zeolites or non-metal doped zeolites, and/or metal oxides, obtaining products of higher value. The renewable chemicals produced using these catalysts include methyl isobutyl ketone (MIBK), diisobutyl ketone (DIBK), isoamylene, and isoprene.

US 2011245542 A1 discloses synthesis of liquid fuels from oxygenated hydrocarbons. Processes and reactor systems for converting oxygen-containing hydrocarbons into hydrocarbons, ketones and alcohols used as liquid fuels such as gasoline, jet fuel or diesel fuel, and industrial chemicals are provided. The process comprises conversion of mono-oxygenated hydrocarbons such as alcohols, ketones, aldehydes, furans, carboxylic acids, diols, triols and/or other polyols to C₄₊ hydrocarbons, alcohols and/or ketones by condensation. Oxidized hydrocarbons can originate from any source, but the preferred ones are those derived from biomass. The teaching of the patent differs in the way that there is a catalytic interaction of the oxygenate in the vapor phase with hydrogen in the presence of a basic condensation catalyst containing a component selected from the group consisting of Li, Na, K, Cs, B, Rb, Mg, Ca, Sr, Si, Ba, Al, Zn, Ce, La, Y, Sc, Y, Zr, Ti, hydrotalcite, phosphate, base treated aluminosilicate zeolite, zinc aluminate, base resin, base nitride, alloys or combinations thereof, at condensation temperatures ranging from about 80°C to 500°C and a condensation pressure of at least 0.1 atm, to obtain a C₄₊ compound, where the C₄₊ compound includes a member selected from the group consisting of C₄₊ alcohol, C₄₊ ketone, C₄₊ alkane, C₄₊ alkene, C₅₊ cycloalkane, C₅₊ cycloalkene, aryl, condensed aryl, and mixtures thereof.

Furthermore, US 2012198760 A1 and US 9228134 B1 disclose a method and systems for producing distillate fuel from biomass. The invention disclosed therein provides methods, reactor systems and catalysts for converting biomass derived feedstock to C_{8 +} hydrocarbons using heterogeneous catalysts. The product stream can be separated and further processed for use in the chemical industry, or as a clean fuel, or as a mixing component for aviation and diesel fuels, or as heavy oils for lubricants and/or liquid fuels.

US 9771533 B2 and US 9932531 B2 disclose systems and processes for the conversion of ethylene feedstocks into hydrocarbon fuels. Systems, processes, and catalysts for producing fuels and fuel mixtures containing selected ratios of open and closed chain fuel range hydrocarbons suitable for the production of alternative fuels, including gasolines, jet fuels, and diesel fuels are disclosed. Fuel range hydrocarbons can be produced from feedstocks containing ethylene and ethanol.

US 2020165176 A1 discloses a method for converting ethanol into 1-butene and 2-butene in a single reactor. The document describes simplified processes for producing chemicals in demand, such as butenes, from feedstocks containing ethanol. In one set of embodiments, this is accomplished in one step where the gas phase ethanol feed is passed over an acidic metal oxide catalyst having a transition metal dispersion of at least 5% on a metal oxide support. The ethanol content in the feed mix can range from 10% to 100% of the feed, and where it is not related to food, the feed ethanol can contain water. The method for the production of butene from a feedstock containing ethanol in one stage is disclosed. The method includes the stage of passing the feedstock containing ethanol in the gas phase over a catalyst 4 wt.% Ag / 4 wt.% ZrO₂ / SiO₂-SBA-16 having a dispersion of the transition metal Ag of not less than 30%, at a temperature of 325°C, a pressure of 7 bar and a flow rate of 0.23 h⁻¹ in the presence of a hydrogen carrier for the direct formation of butenes, with a selectivity equal to or larger than 13% of ethanol, wherein a relatively weak hydrogenation ability of Ag and weakly acidic materials of the carrier provides for maintaining it unchanged.

US 2013144094 A1, US 2013219778 A1, and US 2013237728 A1 disclose methods for direct conversion of oxygenates derived from biomass into hydrocarbons with a longer chain. The longer chain hydrocarbons are characterized by a higher content of naphthenes, which is very useful in distillate range fuels, or more specifically jet and diesel range fuels. Naphthenes help the biomass-derived hydrocarbons meet jet and diesel product specifications while really helping cold flow properties. One of the embodiments describes the hydrotreating processes providing for selective conversion of glycols into monoalcohols that can be blended as biofuels. The NiMo and CoMo catalysts are active in the reaction and the reaction conditions can also affect the selectivity of the monoalcohols. Oxygenate feedstocks derived from biomass are converted into a variety of fuels, including the hydrocarbons of gasoline, jet and diesel fuels. General methods are proposed including hydrolysis, dehydration, condensation, oligomerization, and hydrogenation.

US 2015376511 A1 and US 2016108323 A1 provide a method for producing jet fuel hydrocarbons from alcohols derived from cellulose and hemicellulose, such as pentanediol and hydroxymethyl tetrahydrofuran, which is also known as tetrahydrofurfuryl alcohol. Alcohols are spliced or dimerized using the Guerbet synthesis, in which longer chain organic molecules are formed using a heterogeneous alcohol condensation catalyst. Also disclosed is a method for converting molecules containing one or more functional groups of alcohol into larger molecules. Alcohols such as methanol, ethanol, propanol and hexanol, and diols/glycols such as propylene glycol and butanediol are fed to a supported metal catalyst, such as a noble metal or solid acid catalyst in the presence of hydrogen at elevated temperatures and pressures, yielding a mixture of hydrocarbon and oxygenate products.

US 8049048 B2 discloses renewable engine fuels. The disclosure provides a renewable engine fuel derived completely from biomass sources. One of the embodiments discloses fully renewable motor fuel comprised of one or more low carbon ethers, one or more furans, pentosan derivatives, one or more aromatic hydrocarbons, one or more C₄-C₁₀ straight chain alkanes derivable from polysaccharides and one or more bio-oils. In addition, the fuel may contain triethanolamine. Such a lower octane renewable fuel may be utilized in, for example, automobile fuel, 100 LL aviation fuel, and turbine engines. These fully renewable ethanol fuels can have a wide range of octane numbers and energies and can be effectively used to replace 100 LL aviation fuel (known as AvGas), as well as high octane, rocket, diesel and gas turbine fuel. In another embodiment, there is provided a synthetic, high octane aviation fuel containing isopentane and mesitylene and a process of producing the same from a biomass.

CA 2800057 A1 and US 8852296 B2 disclose renewable engine fuel and a method of producing the same. A non-petroleum, high octane fuel obtained from biomass sources and a method for its production are disclosed. The production method includes the reduction of the biomass feedstock to sugars, the fermentation of sugars using microorganisms or their mutagens to produce ethanol or acetic acid, the conversion of acetic acid or ethanol to acetone and the conversion of acetone to mesitylene and isopentane, the main components of the engine fuel. Trimerization of acetone can be carried out in the presence of a catalyst containing at least one metal selected from a group consisting of niobium, iron and manganese. The ethanol can be converted to mesitylene in a dehydration reaction in the presence of a catalyst of zinc oxide or calcium oxide, and unreacted ethanol and water separated from mesitylene by distillation. These fuels based on ethanol are renewable and may be formulated to have a wide range of octane values and energy, and may effectively be used to replace 100LL aviation fuel (known as AvGas), as well as high octane, rocket, diesel, turbine engine fuels, as well as two-cycle, spark ignition engine fuels. Ethanol can be converted to mesitylene in a dehydration reaction in the presence of a zinc oxide or calcium oxide catalyst, and unreacted ethanol and water are separated from mesitylene by distillation. These ethanol fuels based on biomass are fully renewable, can have a wide range of octane numbers and energies, and can be effectively used to replace 100 LL aviation fuel (known as AvGas), as well as high octane, rocket, diesel, gas turbine fuel, and also fuel for two stroke, spark ignition engines.

US 9447347 B2 teaches production of biofuels via hydrogenolysis condensation. The method disclosed comprises a providing a carbohydrate; reacting the carbohydrate directly with hydrogen in the presence of a hydrogenolysis catalyst to produce a reaction product comprising a polyol; and then processing at least a portion of the reaction product to form a fuel blend.

US 2012156742 A1 teaches a process to produce biofuels from biomass. The document teaches a method to produce biofuels from biomass by contacting the biomass with an aqueous media to form an extracted biomass, separating at least a portion of an aqueous liquor from the extracted biomass thereby providing the aqueous liquor stream comprising soluble carbohydrates; contacting the aqueous liquor stream with a purification substrate effective for removing sulfur compounds and nitrogen compounds thereby producing a treated carbohydrate stream having less than 35% of the sulfur content and less than 35% of the nitrogen content of the untreated aqueous liquor feed, based on the untreated aqueous liquor stream, then contacting the treated carbohydrate stream with an aqueous phase reforming catalyst to form a plurality of oxygenated intermediates; and processing at least a portion of the oxygenated intermediates to form a liquid fuel.

US 9862655 B2 discloses a method and systems for producing jet range hydrocarbons. Methods and systems for producing jet range hydrocarbons are disclosed. In an illustrative embodiment, a process for producing jet range hydrocarbons comprises the steps of combining a first stream of C₄ olefinic hydrocarbons and a second stream of C₅-C₈ olefinic hydrocarbons to produce a third stream of C₄-C₈ hydrocarbons, oligomerizing the third stream of C₄-C₈ olefinic hydrocarbons to produce a fourth stream of C₄-C₂₀ olefinic hydrocarbons and separating C₅-C₈ hydrocarbons from the fourth stream of C₄-C₂₀ olefinic hydrocarbons to produce a second stream of C₅-C₈ olefinic hydrocarbons and a fifth stream of olefinic hydrocarbons C₉-C₂₀. The method further includes the step of hydrogenating a fifth stream of C₉-C₂₀ olefinic hydrocarbons to produce a sixth stream of C₉-C₂₀ paraffinic hydrocarbons of the jet fuel range.

US 2016312131 A1, US 2016312134 A1 provide a process for producing jet-range hydrocarbons. The disclosed therein for producing hydrocarbons suitable for jet fuel, includes passing a renewable olefin feedstock comprising C₃ to C₈ olefins to an oligomerization reactor containing a zeolite catalyst to produce an oligomerized effluent, separating the oligomerized effluent into at least a light stream, and a heavy olefin stream. At least a first portion of the heavy olefin stream is recycled to the oligomerization reactor to dilute the renewable olefin feedstock. A portion of the heavy olefin stream may be hydrogenated and separated to provide a hydrocarbon product suitable for jet fuel. Also disclosed are methods and systems for producing jet-range hydrocarbons. In an illustrative embodiment, a process for producing jet-range hydrocarbons includes the steps of combining a first stream containing C₄ olefinic hydrocarbons and a second stream containing C₅-C₈ olefinic hydrocarbons to produce a third stream containing C₄-C₈ hydrocarbons. A step for oligomerizing a third stream containing C₄-C₈ olefinic hydrocarbons to produce a fourth stream containing C₄-C₂₀ olefinic hydrocarbons. C₅-C₈ hydrocarbons separated from the fourth stream containing C₄-C₂₀ olefinic hydrocarbons, are sent to the second stream, obtaining a fifth stream containing olefinic hydrocarbons C₉-C₂₀. The method further includes the step of hydrogenating a fifth stream containing C₉-C₂₀ olefinic hydrocarbons to obtain a sixth stream containing C₉-C₂₀ paraffinic hydrocarbons in the jet fuel range.

US 2010146843 A1 and US 2011126448 A1 disclose a process, plant, and biofuel for integrated biofuel production. This invention relates to a process, a plant, and a biofuel for integrated biofuel production, such as with butanol, biodiesel, and sugar products. The integrated process includes the step of removing hexose from a feedstock to form a lignocellulosic material. The process also includes the step of converting the hexose to butanol and/or a biodiesel fuel, and the step of depolymerizing lignocellulosic material to form pentose and a residue. The process also includes the step of converting the pentose to butanol and/or a biodiesel material. The process also includes the step of converting pentose to biogasoline and/or biodiesel fuel.

WO 2014154799 discloses production of middle distillate hydrocarbon composition. A process for the preparation of a middle distillate hydrocarbon composition from ethylene is claimed, wherein said process comprises the following steps: (a) feeding an ethylene composition in to an oligomerisation reaction zone containing a supported nickel oligomerisation catalyst to form an oligomerisation product A, wherein the oligomerisation reaction is operated at a temperature in the range of 30 to 300°C and a pressure of at least 10 bar; (b) separating a light products stream B, a middle distillate product stream C and a heavy products stream D from oligomerisation product A, wherein the light product stream B comprises the fraction of oligomerisation product A, which boils in the C₂-C₈ mono-olefin boiling range and a middle distillate product stream C comprising a fraction of oligomerisation product A, which boils above the boiling range of the light product stream B and below the boiling range of the heavy products stream D, and wherein the heavy product stream D comprises the fraction of oligomerisation product A, which boils above the C₂₂ mono-olefin boiling range; (c) recycling a portion of the light products stream B to the oligomerisation reaction zone of step (a) of the process; (d) feeding the heavy product stream D and a portion of the light products stream B in to an olefin metathesis reaction zone to produce a metathesis product stream E; (e) separating a middle distillate product F from the metathesis product stream E; and (f) hydrogenating the middle distillate product stream C, wherein the middle distillate hydrocarbon composition comprises at least a portion of the hydrogenated middle distillate product stream C and at least a portion of the middle distillate product F.

EP 2285972 A2 teaches a method for treating biomass by irradiation with an electron beam to obtain useful products, including fuel. Plant biomass, animal biomass and urban waste biomass exposed to electron beam irradiation are processed to produce useful products. Cellulosic and lignocellulosic materials, as well as materials containing starch or sugar, can be used as feedstock in the claimed method for processing biomass. The irradiated biomass is used to produce ethanol and butanol by fermentation.

WO 2019084518 A1 and US 2019233751 A1 teach a method of preparing cellulosic ethanol having 100% biogenic carbon content as determined by ASTM 6866-18, which includes treating ground corn cobs with electron beam radiation and saccharifying the irradiated ground corn cob to produce sugars. The method also includes fermenting the sugars with a microorganism. The disclosures describe an unblended gasoline derived from cellulosic biomass with a research octane number higher than about 87 as determined by ASTM D2699. At the same time, it is indicated that the unblended gasoline is obtained as a result of catalytic treatment of ethanol obtained from cellulosic biomass. In addition, a jet fuel derived from cellulosic biomass comprising about 25% of aromatic hydrocarbons, about 2.5% of alkenes, about 41% of alkanes, and about 8.5% of oxygenated compounds (wt./wt.) is disclosed.

US 2012271081 A1 discloses a method for the production of C₁₀₊ hydrocarbons from heteroatomic organic compounds. The invention relates to a method for producing C_{10 +} hydrocarbons from heteroatomic organic compounds comprising at least one heteroatom chosen from oxygen, sulfur and halogen, alone or in combination. The method for producing a distillate from organic raw materials containing at least one heteroatom includes the first step of converting the starting organic compounds into olefins. At the second step of conversion, the olefins are oligomerized in the presence of at least 0.5% by weight of oxygen-containing compounds. By virtue of the presence of oxygenates during the oligomerization, this process makes it possible to improve the yield of distillate.

Furthermore, US 2012271085 A1 teaches a method for producing a distillate from a hydrocarbon feed, comprising alcohol condensation. The invention relates to a method for oligomerizing C₃-C₁₀ olefins into a distillate containing C₁₀₊ molecules. According to the disclosure, the oligomerization of C₃-C₁₀ olefins in the presence of at least one alcohol containing at least two carbon atoms provides for the production of C₁₀₊ hydrocarbons.

US 9790444 discloses methods to produce fuels. The disclosure generally relates to the production of fuels, and more specifically, to the catalytic conversion of alcohols into hydrocarbon ketones suitable for use as components in fuels. More specifically, the disclosure refers to the catalytic conversion of an isopropanol + butanol + ethanol (IBE) or acetone + butanol + ethanol (ABE) mixture to ketones suitable for use as a fuel. Blends of ABE or IBE can be obtained by fermentation of biomass or sugars. A method for producing a mixture of hydrocarbon ketones, comprising contacting acetone and at least two or more primary alcohols with a catalyst and, optionally, a base to obtain a mixture of hydrocarbon ketones, wherein the catalyst comprises: (i) one or more metals, and (ii) hydrotalcite (HT), lanthanum oxide (La₂O₃), titanium dioxide (TiO₂), or magnesium oxide (MgO), or any combination thereof.

US 9914672 B2 teaches a method for converting alcohols to distillate fuels. A process for the production of jet and other heavy fuels from alcohols and mixture of alcohols is disclosed. The process may include contacting in a reaction zone at least one C₂ to C₁₁ alcohol with a solid catalyst having activity for the simultaneous dehydration of the alcohols to form olefins, isomerization of the olefins to form internal olefins, and oligomerization of the olefins produced in situ via dehydration to form an effluent comprising mono-olefinic hydrocarbons. Preferably, the alcohol feed is a mixture of alcohols, such as C₂ to C₇ alcohols or C₄ and C₆ alcohols, enabling the production of a mixture of branched hydrocarbons that may be used directly as a jet fuel without blending. The disclosed method for the production of jet and other heavy fuel comprises: (i) in the reaction zone, bringing a mixture of two or more alcohols from C₂ to C₁₁, including at least one secondary alcohol, into contact with a solid catalyst having activity for dehydrating alcohols to yield olefins and water; (ii) oligomerization of olefins obtained in situ from the dehydration reaction; and (iii) isomerization of the resulting olefin oligomers and olefins to yield internal olefins, to obtain monoolefin hydrocarbons. In addition, it is taught that C₂ to C₁₁ alcohols are obtained by biomass fermentation or biomass gasification to synthesis gas followed by a modified Fischer-Tropsch synthesis.

US 9688590 B2 teaches production of jet and other heavy fuels from isobutanol.

US 8329970 B2 teaches a method for the deoxygenation of materials of biological origin. The disclosure relates to a method for the deoxygenation of materials of biological origin and particularly to the removal of oxygen from organic compounds derived from biomass with carbon monoxide, to yield linear and branched hydrocarbons suitable as biofuels or as blending stocks or components for biofuels, such as gas, gasoline, diesel fuel and aviation fuel, as well as solvents. The method comprises contacting a feedstock with carbon monoxide in the presence of a catalyst comprising a metal selected from a group consisting of ruthenium, manganese, rhodium, rhenium, osmium, iridium, molybdenum, copper, zinc, palladium, platinum and cobalt, in the presence of water, under alkaline conditions at a temperature from 150 to 350°C. and under a pressure from 0.1 to 150 bar, to produce hydrocarbons.

WO 2020093127 A1 teaches a process for producing a renewable isoparaffin compound, and use of the renewable isoparaffin compound. The claimed invention relates to a process for producing a renewable isoparaffin compound with a high octane rating, comprising a step of Guerbet reaction between an initial C₅ alcohol load, obtained from renewable raw material, and methanol to produce a branched renewable C₆ alcohol; dehydration of the branched renewable C₆ alcohol into a C₆ olefin; and hydrogenation of the C₆ olefin into renewable isoparaffin. A renewable isoparaffin compound with a high octane rating, comprising at least 50% carbon of renewable natural origin in its composition, and use of said renewable paraffin in gasolines in general and in special high-performance gasolines, such as aviation gasoline, are also described.

Besides the above-mentioned disclosures, the following documents mentioned below have relevance to the claimed invention.

There is a number of patent disclosures dealing with hydroformylation of light olefins, including ethylene and propylene.

GB 1086100 teaches a method for producing aldehydes by olefin hydroformylation: ethylene or propylene is reacted with a mixture of carbon monoxide and hydrogen, as well as an inert gas, in the presence of catalysts containing cobalt at a pressure not exceeding 20 MPa and a temperature of 135°C. The hydrogen content in the total amount of gas in the reactor is 30 to 45 mol.%, and the inert gas is 5 to 15 mol.%. Methane, ethane or nitrogen can be used as the inert gas. In the disclosed hydroformylation process, standard cobalt-containing hydroformylation catalysts such as cobalt acetate, cobalt oxide, cobalt naphthenate, cobalt formate, cobalt carbonyl or hydrocarbonyl, cobalt oleate and cobalt carbonate can be used.

JP 2006160746 discloses a method of hydroformylation, characterized by the addition of an aldehyde to the synthesis stage. The implementation of hydroformylation using an unmodified cobalt complex for a catalyst, as well as the presence of aldehydes in the feedstock, increases the selectivity of the formation of the target product in the range from 0.5 to 20 mol%. The method relates to the hydroformylation of olefins having from 7 to 25 carbon atoms.

CN 1168129 discloses a method providing for hydroformylation of a hydrocarbon stream comprising a stream containing 27.5 to 75 wt. % ethylene and a total content of olefin up to 80 wt. % based on the total hydrocarbon content. Then, in the presence of a rhodium-containing catalyst, it is contacted with synthesis gas, and the hydroformylation product is recovered. The catalyst was prepared in a 500 ml autoclave equipped with a continuous gas supply system with back pressure control. 201 g of tetraglyme, 15.6 g of triphenylphosphine and 0.70 mg of rhodium were mixed under nitrogen. The catalyst solution was transferred to the autoclave under nitrogen, and the autoclave was purged with nitrogen, and then the gas stream was introduced into the boiler as indicated in Table 1. Then the pressure was set to 1000 kPa (absolute pressure) and the back pressure control was turned on, after which the autoclave and its contents were heated up to 100°C. At a catalyst content of 85 ppm, the conversion of olefins in the hydroformylation reaction reached 65%.

CN 101768062 A teaches a hydroformylation process using a water-soluble complex based on rhodium phosphine. The process is carried out in a static mixing reactor and involves the simultaneous formation of propanal and butanal. The invention is a continuous process, the aqueous catalyst solution is recirculated by a pump, the space velocity of the aqueous catalyst solution in the static mixing reactor is from 0.2 to 1.2 m/s. The residence time of the reaction liquid in the circulation tank is 10-20 seconds. The molar ratio of olefin to hydrogen gas and carbon monoxide in the feed mixture is from 1:(1.0-1.1):(1.0-1.1). The reaction pressure of the hydroformylation is from1.4 MPa to 2.5 MPa, and the reaction temperature is 70 to 110°C. Since hydroformylation is a strongly exothermic reaction, heat removal from the reactor is expected. The degree of olefin conversion according to the claimed hydroformylation method is 96.2%, and the selectivity is 95%.

CN 102115433 discloses a method for the synthesis of propanal on a catalyst that is rhodium triphenylphosphine. In this catalyst, the rhodium concentration was 100 ppm and the triphenylphosphine concentration was 2.0%. The synthesis of propanal was carried out at a pressure of 1.5 MPa and a temperature of 80°C. The yield of propanal reached 99.6%.

RU 2354642 C2 teaches a method of hydroformylation of C₂-C₂₀ olefins, characterized by olefins being hydroformylated in the presence of a catalytic system containing rhodium, a polyphosphite ligand and a promoting ligand containing phosphorus. General formulas of polyphosphite ligand and the promoting ligand are given.

RU 2561171 C1 discloses a method of continuous, two-stage hydroformylation of C₃-C₄ olefins and a process unit for producing C₄-C₅ aldehydes. Hydroformylation of olefins is carried out using a recirculating catalyst solution containing a rhodium complex, organophosphorus ligands, product aldehydes, and heavy by-products.

Moreover, RU 2585285 C1 teaches a method for continuous hydroformylation of C₂-C₈ olefins using nanofiltration separation of heavy products from a recirculating catalyst solution containing a rhodium complex with a phosphite ligand. The disclosed method makes it possible to reduce the loss of the catalytically active rhodium complex and organophosphorus ligand when removing heavy condensation products of aldehydes.

Also, RU 2602239 C1 teaches a method of C₆-C₉ olefin hydroformylation into C₇-C₁₀ alcohols. Hydroformylation of C₆-C₉ olefins uses a catalytic system, consisting of a cobalt compound at a concentration of 0.15 to 0.40 wt. % and organophosphorus ligand, which is a triphenylphosphine in a molar ratio to cobalt in the range of 1 to 1.2. The alcohols are produced at a temperature of 170 to 190°C and a synthesis gas pressure of 5 to 10 MPa. The conversion of C₆-C₉ olefins is up to 99%, and the yield of C₇-C₁₀ alcohols is at least 90%.

RU 2051734 C1 discloses a catalyst for the conversion of ethanol into acetone and carbon dioxide, using a catalyst containing zink oxide ZnO 96.5-97.9% and cerium oxide CeO₂ 2.1-3.42%.

RU 0002619951 teaches a two-stage method for propionic aldehyde production. The method includes the stage of carbon dioxide hydrogenation into synthesis gas in the presence of a catalyst containing metallic cobalt supported by organometallic framework structure. Metallic rhodium supported by a carrier was used as a catalyst for the hydroformylation. The process was carried out in a two-shelf flow-through reactor at a pressure of 20 to 40 atm by contacting a stationary layer of a catalyst containing cobalt, placed on the upper shelf of the reactor and heated to a temperature of 500°C, with a raw material mixture of H₂ and CO₂ at a volumetric flow rate of gas feed supply of 500-1000 h⁻¹. After that, the reaction gases obtained, heated to a temperature of 500-520°C, containing a mixture of CO + H₂ + CO₂, were mixed with cold ethylene supplied to the inter-shelf space. The resulting gas mixture in a ratio of CO:H₂:C₂H₄ = 1:(1-2):1 was supplied to the lower shelf of the reactor at a temperature of 170 to 230°C for interaction with the rhodium-containing catalyst placed there. The proposed method provides for increasing the selectivity of the target product formation up to 58.1%, and the yield up to 20.1%, ensuring the utilization of the greenhouse gas, CO₂.

It should be mentioned that the largest scale and most industrially developed method of processing biomass into alcohols is currently the process of ethanol production.

Ethanol is currently used in gasoline, both individually and in the form of ethers to increase the octane number. However, the wider use of ethanol in gasoline is limited by the oxygen content limit, which is regulated by the relevant standards.

At the same time, it is known that ethanol, as well as C₃-C₅ alcohols, can be obtained by biosynthesis and processed into a motor fuel free of oxygen, see e.g. "Conversions of mixtures of C₂-C₈ olefins to jet fuel and/or diesel fuel in high yield from bio-based alcohols" WO 2018071905 A1, US 2020010767 A1. The method consists of the production of C₈-C₁₆ paraffins by processing C₂-C₅ alcohols obtained by fermentation of carbohydrates, extracted from biomass. The paraffins thus obtained can be used to produce motor fuel. The method demonstrates the possibility of obtaining hydrocarbons that can be used as components of motor fuel. Moreover, said method directly indicates the need to use C₂-C₅ alcohols obtained from biomass in the production of motor fuel. Also, the document proposes to use the oligomerization of C₂-C₈ olefins to obtain higher olefins C₁₂-C₁₆. Also, said document proposes to use the hydrogenation of C₈-C₁₆ olefins to obtain C₈-C₁₆ paraffins.

US2007/287873 discloses a method for producing motor fuel from ethanol.

### Objects of the invention

The tasks to be solved by the present invention are the following:
- development of methods for converting ethyl alcohol into higher alcohols, including C₃-C₈ alcohols;
- development of methods for producing C₃-C₈ alcohols, primarily branched alcohols;
- improvement of the technology of C₂-C₃ olefin hydroformylation, including the goal of increasing the yield of C₄-C₈ branched aldehydes;
- development of technology for lower olefin oligomerization into higher olefins, providing for producing fractions of higher branched olefins, with a linear molecule content of not higher than 5%, and free of aromatic and cyclic compounds;
- development of methods for producing C₆-C₂₄ branched paraffins, in which the number of the number of the various compounds is not less than 50 and preferably more than 100, and the content of linear molecules does not exceed 5%, and free of aromatic and cyclic compounds;
- development of methods for producing C₇-C₁₆ ethers of branched structure;
- development of production methods and compositions of gasoline with RON octane number of at least 95 and free of aromatic compounds;
- development of production methods and compositions of kerosene hydrocarbon fractions of primarily branched structure, free from aromatic compounds;
- development of production methods and compositions of kerosene, containing hydrocarbon fractions of mainly branched structure, and with aromatic compounds content in the range from 8% vol. to 25% vol.;
- development of production methods of diesel and diesel compositions that do not contain aromatic compounds;
- development of technology for the manufacture of gasoline, kerosene and diesel using raw materials of biological origin.

### Summary of the invention

In order to accomplish one or more of the above objects the method for producing from ethanol a motor fuel selected from gasoline, kerosene, and diesel, of claim 1 is provided, which method comprises the interconnected steps 1.1-1.13 as specified in claim 1.

A method for producing motor fuel from ethanol has been developed by the present inventor, wherein the technological process of ethanol conversion into motor fuel is carried out as follows:
Ethanol is converted into C₃-C₈ alcohols by two different routes.

In one of the routes, a mixture of ethanol and water at 500-515°C is contacted with a heterogeneous catalyst consisting of the following metal oxides: ZnO 60 to 63% mass; CeO₂ 1 to 6% mass; MgO 12 to 18% mass; Al₂O₃ 13 to 23% mass, with the proportions calculated in terms of metal oxide, yielding a liquid reaction medium, comprised mainly by water and acetone, as well as acetaldedyde and diethylketone. Furthermore, a gaseous reaction medium is obtained, comprised mainly by carbon dioxide and hydrogen, as well as C₂-C₄ olefins and C₁-C₄ paraffins. The acetone and diethylketone are then hydrogenated to yield isopropanol and 3 pentanol alcohol. Acetaldehyde is then used in aldol condensation with propanal to obtain 2-methylbutenal, which is the hydrogenated into 2 methyl butyl alcohol. A mixture of C₂-C₄ olefins and C₁-C₄ paraffins is used in the process of aromatization to obtain aromatic compounds. Carbon dioxide and hydrogen are converted into synthesis gas in the presence of Cu or Ni catalysts.

After that, ethanol and isopropanol in the presence of ditretamyl peroxide interact with ethylene to form sec-butanol, as well as tertiary C₅-C₈ alcohols.

In another route, C₃-C₈ alcohols are obtained by hydroformylation of ethylene or propylene, obtained by dehydration of ethanol or propanol in the presence of a gamma Al₂O₃ catalyst, by synthesis gas produced from carbon dioxide and hydrogen. Hydroformylation of ethylene or propylene is carried out in a heterogeneous reaction medium using a water-soluble Rhodium catalyst. In this case, the concentration of Rhodium in the aqueous phase is from 30 to 50 ppm. Triphenylphosphine-sulfonic acid sodium salts are used as a ligand: from triphenylphosphine-3-sulfonic acid sodium salt to triphenylphosphine-3,3',3"-trisulfonic acid trisodium salt. The process of obtaining propionic or butyl aldehydes is carried out at a temperature of 45 to 90°C and a pressure of 1.0 to 3.0 MPa. The obtained acetaldehyde, propionic and n-butyl aldehydes are treated by cross aldol condensation in the presence of a heterogeneous granular catalyst, containing at least 93% ZSM-5 modified by 3.5 to 7.0% Zn at a temperature of 100 to 150°C and a pressure of 0.5 to 1.0 MPa. The resulting mixture of aldehydes and C₃-C₈ aldoles is then hydrogenated to yield C₃-C₈ alcohols.

Moreover, it was found that the hydroformylation of ethylene or propylene obtained in the dehydration of ethanol or propanol by synthesis gas produced from carbon dioxide and hydrogen can be performed in a heterogeneous reaction medium using a water-soluble cobalt catalyst. When using a cobalt catalyst, the concentration of the metal in the aqueous phase is from 0.1% to 1.0%. In this case, triphenylphosphine-sulfonic acid sodium salts are used as a ligand: from triphenylphosphine-3-sulfonic acid sodium salt to triphenylphosphine-3,3',3"-trisulfonic acid trisodium salt. Hydroformylation of ethylene or propylene is carried out at a temperature of 120 to 145°C and a pressure of 3.0 to 5.0 MPa. It was demonstrated, that besides formation of the propionic or butyl aldehydes, the condensation of said aldehydes occurs in the reaction medium to yield 2-methyl pentenal or 2-ethylhexenal. Hydrogenation of the resulting aldehyde and aldol mixtures provide for producing C₃-C₈ alcohol mixtures.

C₂-C₈ olefins obtained by dehydration of primary and secondary C₂-C₈ alcohols are oligomerized in the presence of a heterogeneous catalyst, containing at least 93% ZSM-5 modified by 3.5 to 7.0% Zn, or in the presence of a heterogeneous catalyst containing at least 95% ZSM-5 modified by 3.5 to 5.0% Zn and 0.1 to 1.5% Ce, at a temperature of 250-350°C and a pressure of 2.0-5.0 MPa to obtain C₆-C₂₄ olefins. The unreacted C₂-C₅ olefins from the oligomerization reaction are used as a raw material for producing aromatic C₇-C₁₂ compounds in the presence of a heterogeneous catalyst, containing at least 93% ZSM-5 modified by 3.5 to 7.0% Zn at a temperature of 350 to 450°C and a pressure of 0.5 to 5.0 MPa.

C₅-C₈ olefins, obtained by dehydration of tertiary C₅-C₈ alcohols, are oligomerized in the presence of a heterogeneous catalyst, which is an ion exchange resin in the form of a cation exchanger, for example Amberlite15, at a temperature of 70-120°C and a pressure of 1.0 to 2.0 MPa to yield C₁₀-C₂₄ olefins. The unreacted C₅-C₈ olefins from the oligomerization reaction and a proportional part of the primary and secondary C₅-C₈ alcohols are used as a source material for producing C₇-C₁₆ ethers in the presence of an ion-exchange resin in the form of, for example, a cation exchanger, Amberlite15, at a temperature of 70 to 120°C and a pressure of 1.0 to 2.0 MPa.

C₆-C₂₄ and C₁₀-C₂₄ olefins are hydrogenated in the presence of a heterogeneous catalyst, containing oxides NiO, CuO and Cr₂O₃ in a molar ratio of 1:1:1, at a temperature of 150 to 200°C and a pressure of 4.5 to 5.0 MPa to obtain C₆-C₂₄ and C₁₀-C₂₄ paraffins. After that, C₆-C₂₄ and C₁₀-C₂₄ paraffins are directed to rectification to obtain C₆-C₁₀ gasoline, C₁₁-C₁₈ kerosene and C₁₉-C₂₄ diesel fractions. The isolated gasoline fraction of C₆-C₁₀ paraffins is blended with C₇-C₁₀ ethers and/or C₇-C₈ aromatic hydrocarbons to obtain gasoline complying with the current standard EN228, however, with a RON octane number of at least 100 and MON of at least 93. The separated kerosene fraction of C₁₁-C₁₈ paraffins is blended with C₉-C₁₂ aromatic hydrocarbons to obtain kerosene complying with the current standard for Jet A-1. The isolated diesel fraction of C₁₉-C₂₄ paraffins is blended with C₁-C₁₆ ethers to obtain diesel fuel complying with the current standards.

The method developed for producing motor fuel from ethanol, as well as the proposed schematic diagram of the technological process for converting ethanol, provide for establishing industrial production of environmentally friendly kerosene, as well as gasoline and diesel.

### Brief description of the attached drawing

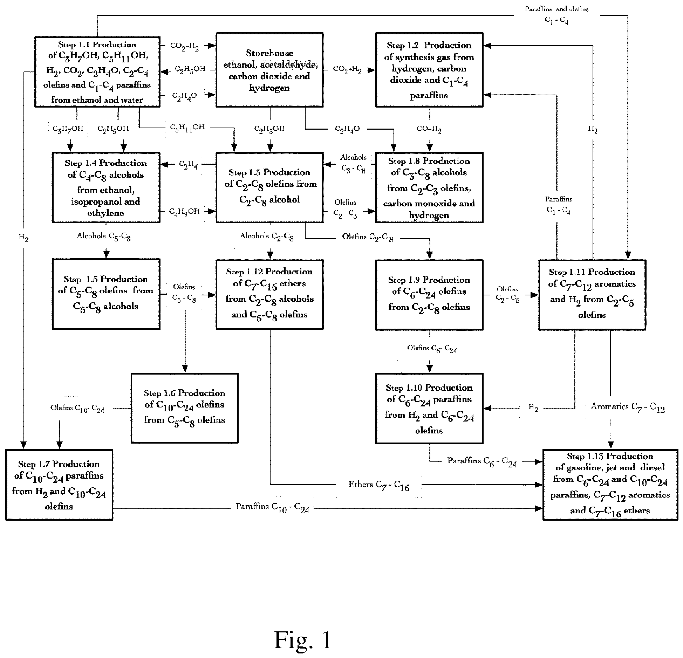

Figure 1 shows a block scheme of the inventive process.

### Detailed description of the invention

Figure 1 shows the inventive scheme developed for conversion of ethanol into motor fuels and, particularly, into kerosene.

The feedstock used for the inventive production of kerosene and other motor fuels is ethanol. Preferred ethanol of the inventive method for the motor fuel production is produced from wood or wastes from humans and their activities. To increase the economic efficiency of our proposed method for converting ethanol into motor fuel, fusel oils can be used as an additional raw material along with carbon dioxide and methane obtained in the production of ethanol. Also, according to the invention, it is recommended to use electricity obtained from environmentally friendly sources and wastes of plant biomass as the energy resource for the method of the present invention of converting ethanol into motor fuel.

It is known in the prior art, that acetone can be obtained from ethyl alcohol with a high yield, that is, 95% of theoretical yield. The vapors of ethyl alcohol mixed with steam are directed at 400-425°C through the catalyst containing 54% of iron and chromium oxides, 8% of copper oxide and 38% of calcium carbonate. The total reaction is described by the equation:

2 C₂H₅OH + H₂O catalysis, t° → CH₃-CO-CH₃ + CO₂↑ +4H₂↑

As disclosed in one of the prior art references above, RU 2051734 C1, acetone is nearly the only liquid product of the reaction in the presence of catalyst consisting of ZnO, 96.5 to 97.9%, and CeO₂, 2.1 to 3.42%.

At step 1.1 of the inventive method for producing motor fuel, an aqueous solution of ethanol is converted into a mixture of acetone, acetaldehyde, diethylketone and and C₃-C₅ alcohols in the presence of a specially designed catalyst at a temperature of 500 to 515°C and a pressure of 0.5 to 1.5 MPa. The composition of the catalyst is as follows: ZnO, 60 to 63% mass; CeO₂, 1 to 6% mass; MgO, 12 to 18% mass; Al₂O₃, 13 to 23% mass. Furthermore, part of the ethanol is converted, under these conditions, into carbon dioxide, hydrogen and a mixture of C₂-C₄ olefins and C₁-C₄ paraffins.

Conversion of ethanol into a mixture of the products above, in the presence of the catalyst used, is unexpected and opens up new possibilities for the production of hydrocarbons that can be used in kerosene.

In the inventive method, a mixture of ethanol and water, the content of which is from 25% to 35% of the total volume of the mixture, contacts at a pressure of 0.5-1.5 MPa and a temperature of 500-515°C with a heterogeneous catalyst consisting of the following metal oxides; ZnO 60-63% mass., CeO₂ 1-6% mass., MgO 12-18% mass., Al₂O₃ 13-23% mass., with the proportions calculated in terms of metal oxide, wherein a mixture of ethanol with water is supplied to the catalyst at a rate of 0.5-0.9 liters per 1 liter of catalyst per hour. Acetone and diethyl ketone, obtained in the process, are isolated from the reaction mixture, hydrogenated at a temperature of 100-150°C and a pressure of 0.5-0.9 MPa in the presence of catalyst containing oxides CuO and Cr₂O₃ in a molar ratio of 1:1 by hydrogen, which is also obtained from a mixture of ethanol and water. This yields isopropanol and 3-pentanol. A mixture of C₃-C₅ alcohols is used for producing the corresponding olefins by dehydration in the presence of catalyst, gamma Al₂O₃. Isopropanol, isolated from the reaction medium, is used to obtain tertiary alcohols C₅-C₇ and C₇₊. Moreover, contacting the catalyst consisting of the following metal oxides: ZnO 60-63% mass., CeO₂ 1-6% mass., MgO 12-18% mass., Al₂O₃ 13-23% mass., with the proportions calculated in terms of metal oxide, with a mixture of ethanol and water yields acetaldehyde, which is isolated from the reaction medium and used in the aldol condensation with propanal to obtain 2-methyl butenal. The 2-methyl butenal is then hydrogenated to yield 2-methyl butanol. Particulary important for the inventive method is the possibility of converting a mixture of ethanol and water into carbon dioxide and hydrogen upon contact with the catalyst consisting of the following metal oxides: ZnO 60-63% mass., CeO₂ 1-6% mass., MgO 12-18% mass., Al₂O₃ 13-23% mass., with the proportions calculated in terms of metal oxide. In step 1.2 of the inventive method said carbon dioxide and hydrogen are converted into water and carbon monoxide in the presence of a catalyst containing 5-10% Cu supported by Al₂O₃, at a temperature of 550-600°C and a pressure of P = 1.0-5.0 MPa, or, using a catalyst containing oxides NiO, CuO and Cr₂O₃ in a molar ratio 1:1:1, are converted at a temperature of 900-1000°C and a pressure of P = 0.1-0.5 MPa into a mixture of carbon monoxide and hydrogen. The carbon monoxide and hydrogen are used in step 1.2 of the inventive process to produce synthesis gas.

Dehydration of ethanol is carried out at the step 1.3 of the inventive method for olefin production in the presence of a gamma Al₂O₃ catalyst in a continuous flow reactor at 350 to 450°C and a pressure of 0.5 to 3.5 MPa.

Ethylene and water are formed in the dehydration reactor. The reaction mixture from the dehydration reactor enters the rectification column, where ethylene is separated from the obtained water. Water is directed to the production of an aqueous solution of ethanol in the process of acetone production.

Ethylene obtained by dehydration of ethanol is directed to step 1.4 of the inventive process for performing the reaction of telomerization or to step 1.8 of the inventive process for performing the reaction of hydroformylation.

Propylene, obtained by dehydration of n-propanol or isopropanol, is directed to step 1.8 of the inventive method to perform the reaction of hydroformylation. C₃-C₈ alcohols obtained in the inventive method for producing motor fuel, are processed at step 1.3 of the inventive method into the corresponding olefins C₃-C₈ in a similar way by using gamma Al₂O₃ as a catalyst. The C₂-C₈ olefins obtained at step 1.3 of the inventive method are sent to step 1.9 of the inventive method to carry out the reaction of oligomerization into C₆-C₂₄ olefins.

Hydroformylation of ethylene, obtained from the dehydration of ethanol at step 1.3 of the inventive method, is carried out at step 1.8 of the inventive method in the presence of either a rhodium or cobalt catalyst. The process of ethylene hydroformylation is carried out in a heterogeneous reaction medium using a water-soluble catalyst. When using a rhodium catalyst, the concentration of the metal with respect to the liquid phase is between 30 and 50 ppm. In this case, triphenylphosphine-sulfonic acid sodium salts are used as a ligand: from triphenylphosphine-3-sulfonic acid sodium salt to triphenylphosphine-3,3',3"-trisulfonic acid trisodium salt in a ratio of 30:1 to metallic rhodium. The process is carried out in the reactor with a stirring device at a temperature of 45 to 90°C and a pressure of 1.0 to 3.0 MPa. The molar ratio of the source gases C₂H₄:CO:H₂ = (1 to 1.1):1:1 is maintained by flow meters.

In a preferred embodiment of the inventive method, in order to reduce catalyst losses, the organic phase, obtained in the process of ethylene hydroformylation, is separated from the aqueous phase, containing dissolved Rh catalysis, using a highly efficient centrifugal separator.

The resulting reaction mass enters a highly efficient centrifugal separator, where it is separated into three phases: a gas phase containing unreacted ethylene, carbon monoxide and hydrogen, a liquid organic phase containing mainly propanal and a liquid aqueous phase containing a water-soluble rhodium catalyst. The gas phase and the liquid aqueous phase are returned to the hydroformylation reactor by the metering devices.

The liquid organic phase, consisting mainly of propanal, is directed to the reactor filled with a granular catalyst containing at least 93% of ZSM-5 zeolite modified by 3.5 to 7.0% Zn, or a granular catalyst containing at least 95% of ZSM-5 zeolite modified by 3.5 to 5.0% Zn and 0.1-1.5% Ce, where the propanal is converted into 2-methyl-pentenal at a temperature of 100 to 150°C and a pressure of 0.5 to 1.0 MPa.

The liquid organic phase, containing mainly 2-methyl-pentenal as well as propanal, is sent to the reactor filled with a granular catalyst comprising NiO, CuO and Cr₂O₃ oxides in a molar ratio of 1:1:1, where 2-methyl-pentenal and propanal are at a temperature of 150 to 200°C and a pressure of 4.5 to 5.0 MPa converted into 2-methyl-pentanol and propanol.

Further, the propanal, obtained by hydroformylation, is mixed with acetaldehyde, which is obtained by the inventive process for ethanol conversion into acetone, and is used to produce 2-methyl-butenal. For this, the mixture of acetaldehyde and propanal, in a molar ratio of 1:1, is directed to a reactor filled with a granular catalyst, containing at least 93% of ZSM-5 zeolite modified by 3.5 to 7.0% Zn, or a granular catalyst containing at least 95% of ZSM-5 zeolite modified by 3.5 to 5.0% Zn and 0.1 to 1.5% Ce, where the mixture of source aldehydes is at a temperature of 100 to 150°C and a pressure of 0.5 to 1.0 MPa converted into 2-methyl-butenal. The reaction mass containing 2-methyl-butenal is directed to the reactor filled with a granular catalyst consisting of the oxides NiO, CuO and Cr₂O₃ in a molar ratio of 1:1:1, where 2-methyl-butenal is converted into 2-methyl-butanol at a temperature of 100 to 200°C and a pressure of 5.0 to 9.0 MPa.

Hydroformylation of propylene, obtained in step 1.3 of the inventive method by dehydration of n-propanol or isopropanol in the presence of a rhodium catalyst is preferably performed as follows. Propanol, obtained by hydrogenation of propanal, or isopropanol, obtained by hydrogenation of acetone, are dehydrated. The dehydration of propyl alcohols is carried out using a heterogeneous gamma Al₂O₃ catalyst at a temperature of 350 to 450°C and a pressure of 0.1 to 1.0 MPa. Propylene, obtained by dehydration of propyl or isopropyl alcohols, is separated from water and sent to hydroformylation.

Hydroformylation of propylene is carried out in a heterogeneous reaction medium using a water-soluble rhodium catalyst. When a rhodium catalyst is used for propylene hydroformylation the metal concentration with respect to the liquid phase is from 30 to 50 ppm. In this case, triphenylphosphine-sulfonic acid sodium salts are used as a ligand: from triphenylphosphine-3-sulfonic acid sodium salt to triphenylphosphine-3,3',3"-trisulfonic acid trisodium salt in a ratio of 30:1 with metallic rhodium. The process is performed in the reactor with a stirring device at a temperature of 70 to 90°C and a pressure of 3.0 to 5.0 MPa. The ratio of the source gases in moles C₃H₆:CO:H₂ = (1 - 1.1):1:1 is maintained by flow meters.

To increase the rate of the propylene hydroformylation reaction and to increase the yield of isobutanal, C₂-C₃ alcohols are added to the water-soluble Rh catalyst in the volume ratio H₂O: (C₂-C₃) = (0.95-0.65) :( 0.05-0.35).

Additionally, in order to reduce catalyst losses, the organic phase obtained in the hydroformylation of propylene is separated from the aqueous phase, containing dissolved Rh catalyst, using a highly efficient centrifugal separator.

The resulting reaction mass is directed to a highly efficient centrifugal separator to separate the reaction mass into three phases: (i) a gas phase containing unreacted propylene, carbon monoxide and hydrogen, (ii) a liquid organic phase containing mainly butanals and (iii) a liquid aqueous phase containing a water-soluble rhodium catalyst. The gas phase and the liquid aqueous phase are returned to the hydroformylation reactor by metering devices.

The liquid organic phase containing n-butanal and isobutanal in the weight ratio (2-3):1 is transferred to the reactor filled with a granular catalyst, containing at least 93% of ZSM-5 zeolite modified by 3.5 to 7.0% Zn, or a granular catalyst containing at least 95% of ZSM-5 zeolite modified by 3.5 to 5.0% Zn and 0.1 to 1.5% Ce, where at a temperature of 100-150°C and a pressure of 0.5-1.0 MPa, butanal is converted into 2-ethyl-hexenal.

The liquid organic phase, containing mainly 2-ethyl-hexenal, as well as isobutanal, is sent to a reactor filled with a granular catalyst consisting of the oxides NiO, CuO and Cr₂O₃ in a molar ratio of 1:1:1, where, at a temperature of 100 to 200°C and a pressure of 5 to 10 MPa, 2-ethyl-hexenal and isobutanal are converted into 2-ethyl hexanol and isobutanol.

Moreover, the inventive method, in a preferred embodiment, provides for obtaining C₅-C₈ alcohols by cross-aldol condensation of n-butanal, acetaldehyde and propanal in the presence of a heterogeneous catalyst containing at least 93% of ZSM-5 zeolite modified by 3.5-7.0% Zn, or a granular catalyst containing at least 93% of ZSM-5 zeolite modified by 3.5-5.0% Zn and 0.1-1.5% Ce. The obtained 2-methyl-butenal, 2-methyl-pentenal, 2-methyl-hexenal and 2-ethyl-hexenal are then hydrogenated in the presence of a heterogeneous catalyst consisting of the oxides NiO, CuO and Cr₂O₃ in a molar ratio of 1:1:1 at a temperature of 150-200°C and a pressure P = 4.5-5.0 MPa to yield 2-methyl-butanol, 2-methyl-pentanol, 2-methyl hexanol and 2-ethyl-hexanol.

The fact that a water-soluble cobalt catalyst can be used for hydroformylation of lower C₂-C₃ olefins was unexpected. When using a cobalt catalyst, the concentration of the metal with respect to the liquid phase is between 0.1% and 1.0%. Triphenylphosphine-sulfonic acid sodium salts are used as a ligand: triphenylphosphine-3-sulfonic acid sodium salt to triphenylphosphine-3,3',3"-trisulfonic acid trisodium salt in a ratio (1-30):1 to metallic cobalt. The hydroformylation of ethylene is carried out in a reactor with a stirring device at a temperature of 120 to 140°C and a pressure of 3.0 to 5.0 MPa. The ratio of the source gases in moles: C₂H₄:CO:H₂ is from 1:1:1 to 1:1.5:2.5 and is maintained by flow meters.

In order to reduce catalyst losses, the organic phase formed during ethylene hydroformylation is separated from the aqueous phase, in which the Co catalyst is dissolved, using a highly efficient centrifugal separator. The resulting reaction mass is transferred to a highly efficient centrifugal separator for separation into three phases: a gas phase containing unreacted ethylene, carbon monoxide and hydrogen, a liquid organic phase containing mainly propanal, 2-methylpentenal, and also 2-methylpentanal, and a liquid aqueous phase containing a water-soluble cobalt catalyst. The gas phase and the liquid aqueous phase are returned to the hydroformylation reactor by metering devices. The fact that 2-methylpentenal and 2-methylpentanal were obtained directly at the hydroformylation step was unexpected.

The liquid organic phase, containing mainly propanal, as well as 2-methylpentenal and 2-methylpentanal, is transferred to the reactor filled with a granular catalyst, containing oxides of NiO, CuO and Cr₂O₃ in a molar ratio of 1:1:1, where at a temperature of 150 to 200°C and a pressure of 4.5 to 5.0 MPa, the mixture of aldehydes is converted into a mixture of the corresponding alcohols, that is n-propanol and 2-methylpentanol.

Hydroformylation of propylene is carried out in the reactor with a stirring device at a temperature of 135 to 140°C and a pressure of 3.0 to 5.0 MPa. The molar ratio of the feed gases: C₃H₆:CO:H₂ is from 1:1:1 to 1:1.5:2.5 and is maintained by flow meters. C₂-C₃ alcohols are added to the water-soluble cobalt catalyst in the volume ratio H₂O:(C₂-C₃) = (0.95-0.5):(0.05-0.5) in order to increase the rate of the reaction of propylene hydroformylation and to increase the yield of isobutanal. In order to reduce catalyst losses, the organic phase formed during hydroformylation of propylene is separated from the aqueous phase, in which the Co catalyst is dissolved, using a highly efficient centrifugal separator. The resulting reaction mass is transferred to a highly efficient centrifugal separator for separation into three phases: a gas phase containing unreacted propylene, carbon monoxide and hydrogen, a liquid organic phase, containing n-butanal, isobutanal, 2-ethylhexenal and 2-ethylhexanal, as well as some amounts of the corresponding alcohols, and a liquid aqueous phase containing a water-soluble cobalt catalyst. The gas phase and the liquid aqueous phase are returned to the hydroformylation reactor by metering devices. The fact that 2-ethylhexenal and 2-ethylhexanal and also butyl alcohols and 2-ethyl hexanol were obtained directly in the hydroformylation step was unexpected.

A mixture of n-butanal and isobutanal is separated by rectification from the liquid organic phase containing n-butanal and isobutanal in a mass ratio of (2-3):1, as well as 2-ethylhexenal and 2-ethylhexanal.

The mixture of n-butanal and isobutanal is directed to the reactor filled with a granular catalyst containing at least 93% of ZSM-5 zeolite modified by 3.5 to 7.0% Zn, or a granular catalyst containing at least 95% of ZSM-5 zeolite modified by 3.5 to 5.0% Zn and 0.1 to 1.5% Ce, where, at a temperature of 100 to 150°C and a pressure of 0.5 to 1.0 MPa, n-butanal is converted into 2-ethylhexenal.

The aldehydes thus obtained are then combined into a general mixture containing isobutanal, 2-ethylhexenal and 2-ethylhexanal. This mixture of aldehydes is sent to the reactor filled with a granular catalyst consisting of the oxides NiO, CuO and Cr₂O₃ in a molar ratio of 1:1:1, where at a temperature of 150 to 200°C and a pressure of 3.0 to 5.0 MPa, isobutanal, 2-ethylhexenal and 2-ethylhexanal are converted into a mixture of the corresponding alcohols, that is: isobutanol and 2-ethylhexanol.

Moreover, in a preferred embodiment, the inventive method provides for obtaining C₃-C₆ alcohols as follows. To obtain C₅ alcohol, namely: 2-methyl butanol, acetaldehyde along with gaseous feed is supplied at a temperature of 120-140°C and a pressure of P = 2.0-5.0 MPa in a molar ratio (C₂H₄:CO:H₂):(C₂H₄O) = 1: 0.5 to a water-soluble Co catalyst during the reaction of ethylene hydroformylation by synthesis gas. This yields propanal, 2-methylpentenal and 2-methylpentanal, as well as 2-methylbutenal, which is formed due to the cross aldol condensation of acetaldehyde with propanal. Then the resulting mixture of propanal and aldols C₅-C₆ is hydrogenated in the presence of a heterogeneous catalyst consisting of the oxides NiO, CuO and Cr₂O₃ in a molar ratio of 1:1:1 at a temperature of 150-200°C and a pressure of P = 4.5-5.0 MPa to obtain a mixture of C₃-C₆ alcohols containing 2-methyl butanol.

In a similar way, in order to obtain C₇ alcohols, namely 2-methyl hexanol and 2-ethyl pentanol, propanal along with a gaseous feed is supplied, during reaction of propylene hydroformylation by synthesis gas, at a temperature of 135-140°C and a pressure of P = 2, 0-5.0 MPa in a molar ratio (C₃H₆.CO:H₂):(C₃H₆O) = 1:(0.5-1) to a water-soluble Co catalyst. This yields isobutanal, 2-methylpentenal, 2-methylpentanal, 2-ethyl hexenal, and 2-ethyl hexanal, as well as 2-methyl hexenal and 2-ethyl pentenal, which are formed due to the cross-aldol condensation of n-butanal with propanal. Then the resulting mixture of isobutanal and aldoles C₆-C₈ is hydrogenated in the presence of a heterogeneous catalyst consisting of the oxides NiO, CuO and Cr₂O₃ in a molar ratio of 1:1:1, at a temperature of 150-200°C and a pressure of P = 4.5-5.0 MPa yielding a mixture of C₄-C₈ alcohols containing, inter alia, 2-methyl hexanol and 2-ethyl pentanol.

Hydroformylation of ethylene and propylene uses synthesis gas obtained by the inventive technology. For the production of synthesis gas, carbon dioxide and hydrogen obtained during conversion of ethanol into acetone at the step 1.1 of the inventive method are used. The synthesis gas production process is performed by two methods. In the first method, a mixture of carbon dioxide and hydrogen in a molar ratio of CO₂:H₂ = 1:1 is supplied into the reactor, filled with a granular catalyst 5 to 10% Cu supported by Al₂O₃, at a temperature of 550 to 600°C and a pressure of 3.0 to 5.0 MPa. The carbon monoxide thus obtained is cooled, isolated from water and directed to step 1.8 of the inventive method, to the hydroformylation reactor. Simultaneously with carbon monoxide, hydrogen is fed into the hydroformylation reactor in a molar ratio of CO:H₂ = 1:1.

In the second method, a mixture of carbon dioxide and hydrogen, in a molar ratio of CO₂:H₂ = 1:(2-3), is supplied into a reactor filled with a granular catalyst consisting of the oxides NiO, CuO and Cr₂O₃ in a molar ratio of 1:1:1, at a temperature of 950 to 1000°C and a pressure of 0.1 to 0.5 MPa. The mixture of carbon monoxide and hydrogen thus obtained is cooled, isolated from water, carbon dioxide and methane, and then transferred to the synthesis gas collecting tank for the adjustment of the composition. Simultaneously with a mixture of carbon monoxide and hydrogen, hydrogen is fed into the synthesis gas collecting tank to adjust the molar ratio to CO:H₂ = 1:1. From the synthesis gas collecting tank the mixture of carbon monoxide and hydrogen is directed to the hydroformylation reactor at step 1.8 of the inventive method by a compressor at a pressure of 3.0 to 5.0 MPa.

The C₂-C₆ alcohols synthesized by the inventive processes can be converted into motor fuel both individually and as mixtures. The main pathway for the conversion of C₂-C₈ alcohols into higher paraffins is based on the oligomerization of C₂-C₈ olefins, which, in turn, are obtained by dehydration of the corresponding alcohols.

According to the invention, zeolites modified by Zn and Ce, that is a zeolite-containing catalyst, containing at least 93% ZSM-5 modified by 3.5 to 7.0% Zn, or a zeolite-containing catalyst, comprising at least 95% ZSM-5 modified by 3.5 to 5.5% Zn and 0.1 to 1.5% Ce are used as catalysts for C₂-C₈ olefin oligomerization.

The inventive method for producing motor fuel provides for using a mixture of primary and secondary C₂-C₅ alcohols, which include C₃-C₅ alcohols synthesized from ethanol, as a feedstock. For this, primary and secondary C₂-C₅ alcohols are dehydrated using a catalyst gamma Al₂O₃ in a continuous flow reactor at a temperature of 350-450°C and a pressure of 0.5-3.5 MPa to yield C₂-C₅ olefins. C₂-C₅ olefins thus obtained are then oligomerized in the presence of a heterogeneous granular catalyst containing at least 93% of ZSM-5 zeolite modified by 3.5-7.0% Zn, or a granular catalyst containing no less than 95% of ZSM-5 zeolite modified by 3.5-5.0% Zn and 0.1-1.5% Ce at a temperature of 250-350°C and a pressure of P = 2.5-5.0 MPa.

The C₆-C₂₀ olefins formed in the process of oligomerization are hydrogenated in the presence of a heterogeneous catalyst consisting of the oxides NiO, CuO and Cr₂O₃ in a molar ratio of 1:1:1, at a temperature of 150-200°C and a pressure of P = 4.5-5.0 MPa to obtain a mixture of C₆-C₂₀ paraffins, which are suitable for isolation of, specifically, gasoline and kerosene fractions of motor fuel.

The oligomerization of the individual olefins in the presence of a zeolite-containing catalyst, comprising at least 93% ZSM-5 modified by 3.5 to 7.0% Zn, or a zeolite-containing catalyst, comprising at least 95% ZSM-5 modified by 3.5 to 5.5 % Zn and 0.1 to 1.5% Ce, differs significantly from the process where the Amberlite 15 is used. For example, when the catalyst, comprising at least 93% ZSM-5 modified by 3.5 to 7.0% Zn catalyst is used in the oligomerization of propylene, obtained from propyl alcohol, olefins in the range C₆H₁₂, C₇H₁₄, C₈H₁₆, C₉H₁₈, C₁₀H₂₀, and so on up to C₂₄H₄₈ are obtained. Hydrogenation of the resulting oligomerization products leads to the production of the corresponding paraffins.

The mixture of primary and secondary C₂-C₈ alcohols, obtained at the step 1.3 of the inventive method, is sent for dehydration to obtain the corresponding mixture of unsaturated C₂-C₈ hydrocarbons. In turn, unsaturated C₂-C₈ hydrocarbons are used at the step 1.9 of the inventive method as a feedstock for the oligomerization process to obtain higher unsaturated C₆-C₂₄ hydrocarbons or for the aromatization process to obtain C₇-C₁₂ aromatic hydrocarbons. Moreover, a mixture of primary and secondary C₂-C₈ alcohols, from step 1.3 of the inventive method, is supplied to step 1.12 of the inventive method to obtain C₇-C₁₆ ethers.

Primary and secondary C₂-C₈ alcohols are dehydrated in the presence of a heterogeneous catalyst, gamma Al₂O₃, in a continuous-flow reactor at a temperature of 350 to 450°C and a pressure of 0.5 to 3.5, MPa.

Unsaturated C₂-C₈ hydrocarbons and water are formed in the dehydration reactor. The reaction mixture from the reactor is fed to a separator to separate the unsaturated C₂-C₈ hydrocarbons from the water formed. The water is sent for utilization, and unsaturated C₂-C₈ hydrocarbons are collected in the collecting tank for further processing. Then the mixture of unsaturated C₂-C₈ hydrocarbons from the collecting tank is directed to the stage of oligomerization.

The unsaturated C₂-C₈ hydrocarbons obtained at step three of the inventive method by dehydration of the corresponding C₂-C₈ alcohols are directed to the oligomerization reactor at step nine of the inventive method. Oligomerization of the unsaturated hydrocarbons is performed at a temperature of 250 to 350°C and a pressure of 2.0 to 5.0 MPa in the presence of a heterogeneous, zeolite-containing catalyst, comprising at least 93% ZSM-5 modified by 3.5 to 7.0% Zn, or a zeolite-containing catalyst comprising at least 95% ZSM-5 modified by 3.5 to 5.5% Zn and 0.1 to 1.5% Ce.

Following laboratory tests, it was found that the selected heterogeneous zeolite-containing catalyst, comprising at least 93% ZSM-5 modified by 3.5 to 7.0% Zn, or a zeolite-containing catalyst, comprising at least 95% ZSM-5 modified by 3.5 to 5.5% Zn and 0.1 to 1.5% Ce are most active in the oligomerization of C₂-C₈ olefins obtained from primary and secondary C₂-C₈ alcohols. In the course of oligomerization, the unsaturated C₂-C₈ hydrocarbons are converted into C₆-C₂₄ unsaturated hydrocarbons.

The unsaturated C₆-C₂₄ hydrocarbons are then hydrogenated in the presence of the catalyst consisting of the oxides NiO, CuO and Cr₂O₃ in a molar ratio of 1:1:1, at a temperature of 150 to 200°C and a pressure of 3.0 to 5.0 MPa to yield a mixture of C₆-C₂₄ paraffins. C₆-C₂₄ paraffins obtained at step 1.9 of the inventive method are a feedstock for the production, at step 1.13 of the inventive method, of motor fuel, primarily kerosene, as well as gasoline and diesel.

It should be noted that the kerosene, obtained by the inventive method, has a number of unique properties. Studies performed by the inventor, which included mass spectroscopy, demonstrate that the composition of the kerosene, obtained solely from propylene, contains more than 150 different C₈-C₁₆ isomers and the linear hydrocarbons content is less than 5.0% mass. Such a kerosene, obtained solely from propylene, does not freeze at a temperature of minus 85°C, and also has a smoke point of more than 30 millimeters.

The unreacted at step 1.9 unsaturated C₂-C₅ hydrocarbons are used as a feedstock at step 1.11 of the inventive method in the process of aromatization to obtain C₇-C₁₂ aromatic compounds.

The unsaturated C₂-C₅ hydrocarbons obtained by rectification of the unsaturated C₆-C₂₄ hydrocarbons are supplied to step 1.11 of the inventive method to the aromatization reactor. Furthermore, a mixture of olefins and paraffins C₁-C₄, obtained at step 1.1 of the inventive method, is fed to the source reaction mixture at step 1.11. Aromatization of the unsaturated C₂-C₅ hydrocarbons is performed at a temperature of 350 to 450°C and a pressure of 0.5 to 2.0 MPa in the presence of a heterogeneous zeolite-containing catalyst, comprising at least 93% ZSM-5 modified by 3.5 to 7.0% Zn in a continuous-flow reactor. The laboratory tests have established that the selected catalyst is most active in the aromatization of primary C₄-C₅ olefins. In the process of aromatization, the unsaturated C₂-C₅ hydrocarbons are converted into aromatic C₇-C₁₂ hydrocarbons.

C₇-C₁₂ aromatic hydrocarbons of step 1.11 are sent to step 1.13 of the inventive method for the production of motor fuel. Furthermore, a mixture of gaseous products is produced in the process of aromatization of the unsaturated hydrocarbons C₂-C₅. This mixture of gaseous products, comprised by hydrogen and C₁-C₄ paraffins, is separated from the liquid phase and sent to step 1.2 of the inventive method for producing synthesis gas.

Yet another way of ethanol conversion into motor fuel, developed by the inventor, is to use the reaction of telomerization for producing secondary and tertiary alcohols C₃-C₈. The main source material for this process is ethanol along with secondary alcohols such as iso-propanol or sec-butanol obtained from ethanol. Ethanol and isopropanol are supplied from step 1.1 of the inventive method to step 1.4 for telomerization. Furthermore, this process uses, as a raw material, ethylene obtained by dehydration of ethanol at step 1.3 of the inventive method.

The catalyst used in the reaction of telomerization is peroxides of tertiary alcohols, including tert-butyl or tert-amyl alcohols. The ratio of the catalyst: tert-butyl or tert-amyl alcohol peroxide to ethanol or secondary alcohols by mass is (1 to 2)% mass of tert-butyl or tert-amyl peroxide to (98 to 99)% of ethanol or secondary alcohol.

The process is carried out while stirring a liquid mixture of tert-butyl or tert-amyl alcohol peroxide and ethanol or isopropanol or a mixture of these alcohols under a pressure of gaseous ethylene P = 1.0-5.0 MPa at a temperature of 100-130°C. Under these conditions, isopropanol is converted into tertiary C₅-C₉ alcohols, while ethanol is converted into sec-butanol and tertiary alcohols C₆-C₁₀. Conversion of ethanol into tertiary alcohols in this process was unexpected.

The obtained tertiary C₅-C₈ alcohols are isolated from the reaction mixture by rectification. In the process of rectification, individual tertiary alcohols or tertiary alcohol fractions are isolated. The yield of tertiary C₅-C₈ alcohols in terms of the reacted source alcohol reached more than 98% with a conversion rate of the initial alcohol higher than 60%. The yield of the obtained tertiary C₈₊ alcohols did not exceed 2% mass. The unreacted source alcohols from telomerization were separated at the rectification stage and returned to the telomerization process or were used in the process of producing ethers. The resulting C₈₊ tertiary alcohols can be used as oxygen-containing additives in the production of gasoline or diesel.

The mixture of tertiary C₅-C₈ alcohols obtained in the processes described above, is sent to step 1.5 of the inventive method for dehydration to obtain the corresponding mixture of unsaturated C₅-C₈ hydrocarbons. Dehydration of tertiary C₅-C₈ alcohols is carried out in the presence of a new or regenerated heterogeneous catalyst, gamma Al₂O₃ at a temperature of 100 to 150°C and a pressure of 0.5 to 1.5 MPa until reaching the yield of C₅-C₈ olefins of at least 99% of the source C₅-C₈ alcohols. Then the temperature is increased to 350-450°C and primary and/or secondary alcohols C₂-C₈ are dehydrated, wherein the process is continued in the presence of the same catalyst at a temperature not exceeding 450°C until reaching the yield of C₂-C₈ olefins of not less than 99% of the source primary and/or secondary C₂-C₈ alcohols.

Upon completion of the dehydration of primary and/or secondary C₂-C₈ alcohols, the catalyst is regenerated.

C₅-C₈ unsaturated hydrocarbons and water are obtained in the dehydration reactor. The reaction mixture from the reactor is directed to the separator for separation of the mixture of unsaturated C₅-C₈ hydrocarbons from the water obtained. The water is sent for utilization and the unsaturated C₅-C₈ hydrocarbons are collected in the collecting tank for further processing. Further, the mixture of unsaturated C₅-C₈ hydrocarbons from the collecting tank is directed to step 1.6 of the inventive method for oligomerization.

The inventor recommends using ion-exchange resins in the form of cation exchangers, for example, Amberlite15, as catalysts for oligomerization of C₃-C₈ olefins. Studies performed by the inventor have established that the use of Amberlite 15, as a catalyst in the process of oligomerization, obtains higher unsaturated compounds by way of condensation of the source olefin in the form of dimers, trimers, and so on. That is, higher olefins with a molecular weight as a multiple of the initial unsaturated compound appear. For example, oligomerization of propylene in the presence of a cation exchanger Amberlite 15 yields olefins C₆H₁₂, C₉H₁₈, C₁₂H₂₄, and so on up to C₂₄H₄₈. Hydrogenation of the resulting oligomerization products yields the corresponding paraffins.

At the same time the use of Amberlite 15 at the stage of oligomerization along with a mixture of at least two olefins: propylene and isobutylene, obtained from the corresponding alcohols, results in obtaining a mixture of higher olefins. Hydrogenation of said mixture of higher olefins yields a mixture of higher paraffins in the range C₆H₁₄, C₇H₁₆, C₈H₁₈, C₉H₂₀, C₁₀H₂₂ and so on up to C₂₄H₅₀.

Moreover, the unsaturated C₅-C₈ hydrocarbons obtained at step 1.5 by dehydration of tertiary alcohols C₅-C₈ are used at step 1.6 of the inventive method as a raw material in the reaction of oligomerization to obtain unsaturated hydrocarbons C₁₀-C₂₄.

Oligomerization of the unsaturated hydrocarbons C₅-C₈ is carried out in the presence of catalyst, ion-exchange resins in the form of cation exchangers, for example Amberlite15, in a continuous flow reactor at a temperature of 70-120°C and a pressure of 1-2 MPa. Oligomerization of the unsaturated C₅-C₈ hydrocarbons yields a mixture of higher unsaturated hydrocarbons C₁₀-C₂₄.

The unsaturated C₁₀-C₂₄ hydrocarbons of step 1.6 of the inventive method are then hydrogenated at step 1.7 in the presence of a catalyst consisting of the oxides NiO, CuO and Cr₂O₃ in a molar ratio of 1:1:1, at a temperature of 150 to 200°C and a pressure of 3.0 to 5.0 MPa, to obtain a mixture of C₁₀-C₂₄ paraffins.

C₁₀-C₂₄ paraffins obtained at step 1.7 are the source material for the production of motor fuel, first of all, kerosene, as well as gasoline and diesel at step 1.13 of the inventive method. The iso-structure of these hydrocarbons obtains kerosene and diesel with unique characteristics, as well as gasoline free of aromatic compounds with octane numbers of at least 95 RON and 91 MON. Studies carried out by the inventor, which included chromatomass spectroscopy, demonstrate that the composition of kerosene, obtained from tertamyl alcohol, contains more than 60 different C₁₀-C₁₅ isomers and there are no linear hydrocarbons. This kerosene, obtained from tertamyl alcohol, does not freeze at a temperature of minus 85°C, and also has a smoke point of more than 30 mm.

The inventive method for producing motor fuel provides for using tertiary C₅-C₈ alcohols, synthesized on the basis of ethanol, as a feedstock. For this purpose, C₅-C₆ olefins are isolated by rectification from the mixture of C₅-C₈ olefins of step 1.5, obtained by dehydration of tertiary alcohols C₅-C₈, and oligomerized at step 1.6 of the inventive method at a temperature of 70-120°C and a pressure of P = 1.5-2.0 MPa in the presence of catalyst, ion exchange resins in the form of cation exchangers, for example Amberlite15.

C₁₀-C₁₈ olefins obtained in the process of oligomerization are hydrogenated in a presence of a heterogeneous catalyst consisting of the oxides NiO, CuO and Cr₂O₃ in a molar ratio of 1:1:1, at a temperature of 150-200°C and a pressure of P = 4.5-5.0 MPa to obtain a mixture of C₁₀-C₁₈ paraffins, which almost entirely is a kerosene fraction of motor fuel.

Furthermore, C₇-C₈ olefins isolated from the mixture of C₅-C₈ olefins, obtained by dehydration of tertiary C₅-C₈ alcohols, are oligomerized at a temperature of 70-120°C and a pressure P = 1.5-2.0 MPa by using, as catalyst, ion-exchange resins in the form of cation exchangers, for example, Amberlite15.

C₁₄-C₂₄ olefins obtained as a result of oligomerization are hydrogenated in the presence of a heterogeneous catalyst consisting of the oxides NiO, CuO and Cr₂O₃ in a molar ratio of 1:1:1, at a temperature of 150-200°C and a pressure of P = 4.5-5.0 MPa to obtain C₁₄-C₂₄ paraffins. Subsequently, kerosene and diesel fractions of the motor fuel are isolated from said hydrocarbon mixture by rectification.

The inventive method for producing motor fuel from ethanol makes it possible to produce and use oxygen-containing additives, which, when used in gasoline and diesel compositions, improve significantly the quality of said compositions. The main oxygen-containing additives proposed herein to be used to improve properties of gasoline and diesel are ethers.

It is proposed to obtain these ethers by using primary of secondary C₂-C₈ alcohols, obtained by the inventive method of the motor fuel production. Furthermore, the unsaturated C₅-C₈ hydrocarbons, obtained by dehydration of tertiary alcohols C₅-C₈, can be used for producing said ethers.

The process for producing ethers is carried out at step 1.12 of the inventive method, in the presence of a catalyst, the ion-exchange resins in the form of cation exchangers, for example Amberlite15, in a continuous-flow reactor at a temperature of 50 to 100°C and a pressure of 0.5 to 1.5 MPa. C₂-C₈ primary and secondary alcohols of step 1.3 of the inventive method, that are supplied to step 1.12, interact with C₅-C₈ olefins, of step 1.5 of the inventive method, that are supplied to step 1.12, to yield a mixture of C₇-C₁₆ ethers. The mixture of C₇-C₁₆ ethers is directed to step 1.13 of the inventive method for producing motor fuel.

As described above in the detailed description of the inventive method for ethanol conversion into motor fuel, the inventive technology provides for obtaining mixtures of paraffins in the C₆-C₂₄ range, consisting mainly of branched hydrocarbons. These paraffin mixtures are separated by rectification into fractions C₆-C₁₀, C₁₁-C₁₈, and C₁₉-C₂₄, suitable for producing gasoline, kerosene and diesel. In addition, the inventive technology for the production of aromatic compounds from ethanol provides for obtaining mixtures of aromatic hydrocarbons in the range C₇-C₁₂. These mixtures of aromatic hydrocarbons are separated by rectification into fractions C₇-C₈ and C₉-C₁₂, suitable for producing gasoline and kerosene. Mixing the gasoline fraction of C₆-C₁₀ paraffins with the gasoline fraction of C₇-C₈ aromatic hydrocarbons provides for obtaining gasoline meeting all the requirements of the EN 228 standard. Mixing the kerosene fraction of C₁₁-C₁₈ paraffins with the kerosene fraction of aromatic C₉-C₁₂ hydrocarbons provides for obtaining kerosene meeting all the requirements of the Jet A-1 standard.

At the same time, the inventive technology for the production of ethers from ethanol provides for obtaining mixtures of ethers in the range C₇-C₁₆. These mixtures of C₇-C₁₆ ethers are separated by rectification into fractions C₇-C₁₀ and C₁₁-C₁₆, suitable for producing gasoline and diesel. Mixing the gasoline fraction of C₆-C₁₀ paraffins with the gasoline fraction of C₇-C₁₀ ethers provides for obtaining gasoline meeting requirements of the EN 228 standard and free of aromatic compounds, and with octane ratings RON of at least 100 and MON of at least 93. Mixing the diesel fraction of C₁₉-C₂₄ paraffins with the diesel fraction of C₁₁-C₁₆ ethers provides for obtaining diesel meeting requirements of the EN 590 standard and completely free of aromatic hydrocarbons.

C₆-C₂₄ paraffins obtained from primary and secondary C₂-C₈ alcohols at steps 1.8, 1.9 and 1.10 of the inventive method for conversion of ethanol into motor fuel, and C₁₀-C₂₄ paraffins obtained from tertiary C₅-C₈ alcohols at steps 1.5, 1.6 and 1.7 of the inventive method, are supplied to step 1.13 for rectification to obtain C₆-C₁₀ gasoline, C₁₁-C₁₈ kerosene, and C₁₉-C₂₄ diesel fraction of the motor fuel. Moreover, aromatic C₇-C₁₂ hydrocarbons obtained from primary and secondary C₂-C₅ alcohols at steps 1.3, 1.9 and 1.11 of the inventive method are supplied to step 1.13 for rectification to yield C₇-C₈ gasoline and C₉-C₁₂ kerosene fractions of the motor fuel. C₇-C₈ aromatic hydrocarbons are used to produce gasoline meeting all requirements of the EN 228 standard. C₉-C₁₂ aromatic hydrocarbons are used to produce kerosene meeting all the requirements of the Jet A-1 standard.

Also, C₇-C₁₆ ethers, obtained at steps 1.3, 1.4, 1.5 and 1.12 from primary and secondary C₂-C₈ alcohols, and C₅-C₈ olefins, obtained from tertiary C₅-C₈ alcohols, are supplied to step 1.13 for rectification, and are separated into a mixture of C₇-C₁₀ ethers and a mixture of C₁₁-C₁₆ ethers. C₇-C₁₀ ethers are used to produce a high-quality gasoline, while C₁₁-C₁₆ ethers are used to produce a high-quality diesel.

The use of the ethers obtained in the inventive process in the compositions of gasoline provides for excluding aromatic compounds from compositions of gasoline and for reducing the harmful effects of the gasoline combustion products on the environment. Up to 22% of ethers by volume are allowed in gasoline according to the current standards. However, it is necessary to comply with the oxygen content limit, which for various standards can be not higher that 2.7% by weight or 3.2% by weight.

The compositions of gasoline obtained by the invention with an ether content of not more than 20% by volume provide for achieving RON ratings of at least 100 and MON of at least 93, without violating the above requirements for oxygen content.

A distinguishing feature of the inventivew method for obtaining motor fuel from C₆-C₂₄ paraffins, synthesized from ethanol, is that in order to obtain gasoline fully meeting the requirements of the existing EN 228 standard, the C₆-C₁₀ paraffin fraction isolated from a mixture of C₆-C₂₄ paraffins is mixed with a fraction of C₇-C₁₀ ethers isolated from a mixture of C₇-C₁₆ ethers, synthesized from ethanol, and/or with a fraction of C₇-C₈ aromatic hydrocarbons, isolated from a mixture of C₇-C₁₆ aromatic hydrocarbons, which are also synthesized from ethanol. Wherein the C₇-C₁₆ ethers required for mixing are obtained by etherification of primary or secondary C₂-C₈ alcohols with C₅-C₈ olefins obtained from tertiary C₅-C₈ alcohols.

The esterification process is carried out in the presence of catalyst, which is an ion-exchange resin in the form of cation exchanger, for example Amberlite15, at a temperature of 70-120°C and a pressure of P = 1.5-2.0 MPa. For the production of C₇-C₁₆ ethers are used primary or secondary C₂-C₈ alcohols, obtained from tertiary C₅-C₈ alcohols, and C₅-C₈ olefins, wherein all raw material for the production of C₇-C₁₆ ethers is obtained from ethanol.

According to one preferred embodiment of the inventive method, in order for obtaining motor fuel from C₆-C₂₄ paraffins, synthesized from ethanol, in order to produce diesel fuel that meets all requirements of the existing EN 590 standard, the C₁₉-C₂₄ paraffin fraction isolated from a mixture of C₆-C₂₄ paraffins, is mixed with the fraction of C₁₁-C₁₆ ethers, isolated from a mixture of C₇-C₁₆ ethers, said C₇-C₁₆ ethers are also synthesized from ethanol. Wherein, the C₇-C₁₆ ethers required for the mixing are produced by etherification of primary or secondary C₂-C₈ alcohols by C₅-C₈ olefins, obtained from tertiary C₅-C₈ alcohols.

The esterification is performed in the presence of catalysts, the ion-exchange resin in the form of cation exchangers, for example Amberlite15, at a temperature of 70-120°C and a pressure of P = 1.5-2.0 MPa. For the production of C₇-C₁₆ ethers are used primary or secondary C₂-C₈ alcohols, obtained from tertiary C₅-C₈ alcohols, and C₅-C₈ olefins, wherein all raw material for the production of C₇-C₁₆ ethers is obtained from ethanol.

According to a preferred embodiment of the inventive method, in order to obtain kerosene meeting all the requirements of the existing standard Jet A-1 from C₆-C₂₄ paraffins (obtained at steps: 1.3, 1.8, 1.9 and 1.10 from primary and secondary alcohols C₂-C₈), from C₁₀-C₂₄ paraffins (obtained at steps: 1.4, 1.5, 1.6 and 1.7 from tertiary C₅-C₈ alcohols), and from aromatic C₇-C₁₂ hydrocarbons (obtained at steps: 1.3, 1.9 and 1.11 from the primary and secondary C₂-C₅ alcohols), the C₁₁-C₁₈ paraffins required in the kerosene composition, obtained at step 1.10 and isolated from the mixture of C₆-C₂₄ paraffins at step 1.13, the C₁₁-C₁₈ paraffins, obtained at step 1.7 and isolated from the mixture of C₁₀-C₂₄ paraffins at step 1.13, and the aromatic C₉-C₁₂ hydrocarbons, obtained at step 1.11 and isolated from the mixture of aromatic C₇-C₁₂ hydrocarbons, are mixed so that the concentration of aromatic C₉-C₁₂ compounds is in the range of 8-25% vol, while the resulting kerosene composition comprises at least 100 different hydrocarbons, and preferably 150 different hydrocarbons, and has a smoke point of min 30 mm and a freezing point of max minus 80°C.

It should be noted that the inventive method for the production of motor fuel provides for using not only ethanol containing a significant amount of water, but also ethanol containing a large amount of fusel oils. Also, the inventive method for ethanol convertion into motor fuel can use carbon dioxide and methane, obtained during the production of ethanol, as an additional raw material.

Moreover, the inventive method is not limited only to the motor fuel production industry. The inventive method for converting ethanol into C₃-C₈ containing aldehydes, ketones, alcohols: primary, secondary and tertiary, olefins, as well as C₆-C₂₄ containing olefins and branched paraffins may be in demand in the chemical and cosmetic industries.

The implementation of the inventive method for producing motor fuels is demonstrated by the following examples.

### Example 1. Conversion of ethanol into a mixture of higher alcohols and hydrocarbons, as well as into synthesis gas.

The process of converting ethanol into a mixture of higher alcohols and hydrocarbons, as well as into synthesis gas, was carried out in a cascade of reactors filled with appropriate heterogeneous catalysts. At the first step of the process, an aqueous solution of ethanol was supplied into a one liter volume continuous flow reactor, where the catalyst was loaded. The catalyst consists of the following metal oxides: ZnO 60 to 63% mass.; CeO₂ 1 to 6% mass.; MgO 12 to 18% mass.; Al₂O₃ 13-23 % mass, with the proportions calculated in terms of metal oxide. The technological parameters of the process varied within the following ranges: temperature 500 to 515°C, pressure 0.9 to 1.1 MPa, ethanol concentration in water 59 to 63% wt, load 0.45 to 0.77 kg of ethanol solution in water per 1 liter of catalyst. The gaseous reaction mass obtained as a result of the interaction of ethanol with water was directed to step 1.2 of the process while the liquid reaction mass was directed to step 1.3 of the process.

The gaseous reaction mass, which is a mixture of hydrogen, carbon dioxide, saturated C₁-C₄ and unsaturated C₂-C₄ hydrocarbons, was supplied to step 1.2 of the process. This gaseous reaction mass was directed into a one liter volume continuous flow reactor filled with a heterogeneous zeolite-containing catalyst, comprising at least 93% ZSM-5 modified by 3.5 to 7.0% Zn catalyst. The technological parameters of the process varied within the following limits: temperature 350 to 450°C, pressure 0.5 to 1.0 MPa. Under these conditions, the C₂-C₄ olefins contained in the gaseous reaction mass were converted into aromatic compounds, and the obtained gas mixture was directed to the absorber to extract carbon dioxide. The gas mixture, free from carbon dioxide, with a hydrogen content exceeding 95%, was directed to step 1.3 of the process.

At step 1.3 of the process, acetaldehyde was isolated from the liquid reaction mass obtained at step 1.1 of the process. After that the remaining liquid reaction mass was separated into organic and aqueous phases. The aqueous phase, containing unreacted ethanol, is returned to the process to produce the aqueous ethanol solution. The organic phase, which is a mixture of C₃ and C₅ ketones and C₃ and C₅ alcohols, was directed to the step of hydrogenation. To do this, the mixture of oxygen-containing products of ethanol conversion was supplied into a 1-liter continuous flow reactor, equipped with the catalyst consisting of the oxides CuO and Cr₂O₃ in a molar ratio of 1:1. The technological parameters of the process varied within the following limits: temperature 100 to 150°C, pressure 0.5 to 0.9 MPa. The gas mixture obtained at the second step of this process was supplied into the reactor loaded with the catalyst, comprixed by oxides CuO and Cr₂O₃ in a molar ratio of 1:1, along with the mixture of the liquid oxygen-containing products of the ethanol conversion. Under these conditions, the C₃ and C₅ ketones contained in the liquid organic mixture were converted into the corresponding C₃ and C₅ alcohols. The residual unsaturated compounds were removed from the gas mixture obtained in the presence of the catalyst, comprised by oxides CuO and Cr₂O₃ in a molar ratio of 1:1, by hydrogenation. This gas mixture is an excellent raw material for synthesis gas production. Table 1 shows the results of a few experiments carried out in accordance with the described technological process for converting a mixture of ethanol and water into a mixture of alcohols, aromatic hydrocarbons, and also into synthesis gas.

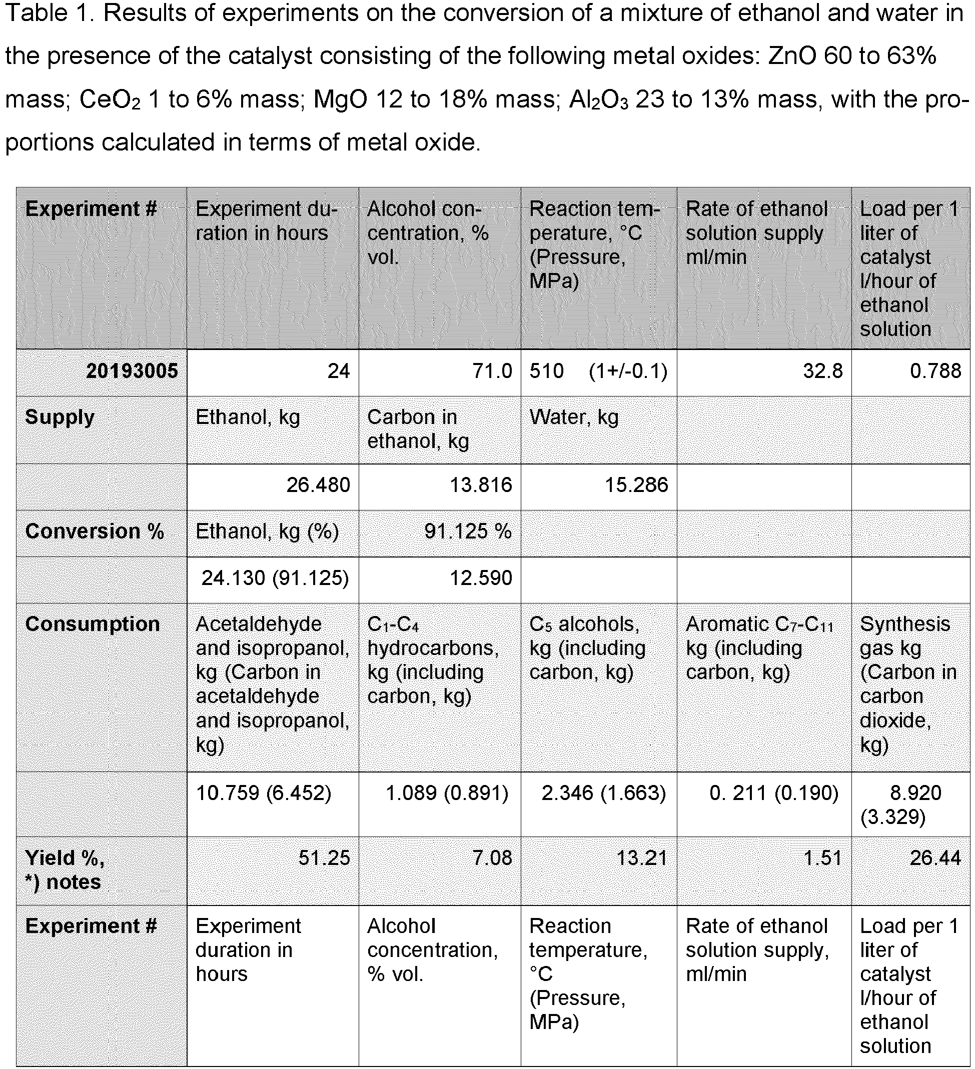

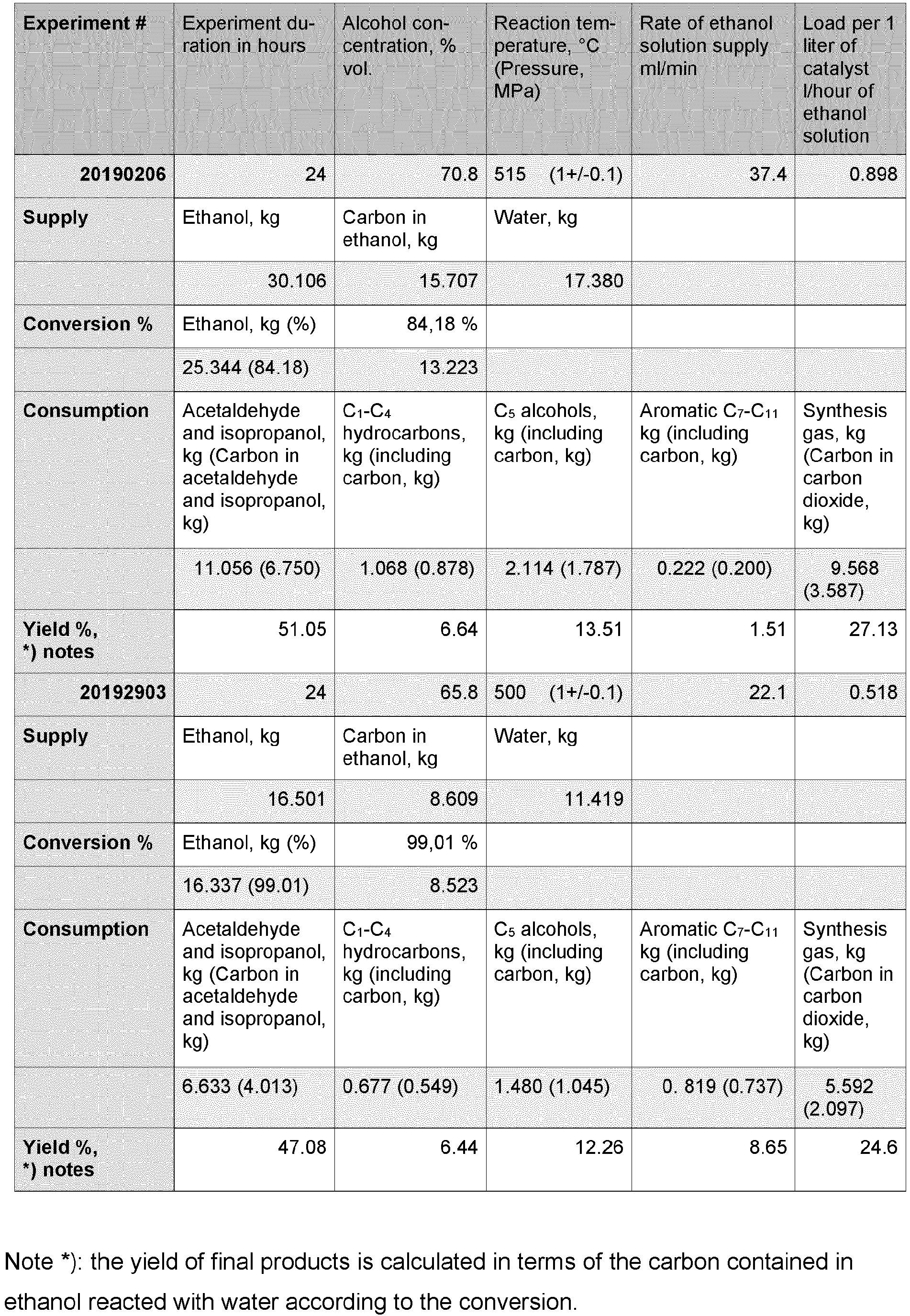

### Example 2. Synthesis gas generation in the presence of copper and nickel catalysts.

Synthesis gas, obtained on the basis of the inventive technology, is used in the inventive process of hydroformylation of ethylene and propylene.

For the production of synthesis gas, carbon dioxide and hydrogen obtained in the conversion of ethanol to acetone are used. Carbon dioxide is distilled off from the liquid reaction mass in the process of acetaldehyde distillation. In addition, carbon dioxide is extracted from the absorbent solution obtained during the purification of hydrogen, which is supplied to the stage of hydrogenation of acetone and other oxygen-containing compounds. The gas mixture leaving the stage of hydrogenation contains more than 90% hydrogen and is an excellent raw material for the production of synthesis gas by the inventive technology. The synthesis gas thus obtained is used in the process of ethylene and propylene hydroformylation.

The synthesis gas production by the inventive technology is carried out by two methods.

In the first method, carbon dioxide and mixture of 90% hydrogen in a molar ratio of CO₂:H₂ = 1:(1.1-1.5) are fed into a flow reactor filled with a granular catalyst of 5 to 10% Cu supported on Al₂O₃ at a temperature of 550 to 600°C and a pressure of 3.0 to 5.0 MPa. The carbon monoxide obtained in this process is cooled and isolated from water and a mixture of C₁-C₄ paraffins. The carbon monoxide, free of water, and the mixture of C₁-C₄ paraffins is directed to the hydroformylation reactor. Simultaneously with carbon monoxide, hydrogen is fed into the hydroformylation reactor in a molar ratio of CO:H₂ = 1:1.

In the second method, carbon dioxide and hydrogen in a molar ratio of CO₂:H₂ = 1:(2-3) are fed into a flow reactor filled with a granular catalyst consisting of the oxides NiO, CuO and Cr₂O₃ in a molar ratio of 1:1:1, at a temperature of 950 to 1000°C and a pressure of 0.1 to 0.5 MPa. The mixture of carbon monoxide and hydrogen obtained in this process is cooled, isolated from water, carbon dioxide and methane, and then sent to the synthesis gas collecting vessel to adjust the composition. Simultaneously with the mixture of carbon monoxide and hydrogen, hydrogen is fed into the synthesis gas collecting vessel to adjust the molar ratio of CO:H₂ = 1:1. From the synthesis gas collecting vessel the mixture of carbon monoxide and hydrogen in a molar ratio of CO:H₂ = 1:1 is sent to the hydroformylation reactor by a compressor at a pressure of 3.0 to 5.0 MPa.

### Example 3. Obtaining di tert-amyl peroxide.

Under vigorous stirring, tert-amyl alcohol is added to an aqueous solution of 70% sulfuric acid, cooled to a temperature of 0 to 5°C, in a molar ratio of H₂SO₄:(CH₃)₂C(OH)CH₂CH₃ = 1:1. After adding the entire amount of tert-amyl alcohol the resulting reaction mass is stirred at a temperature of 0 to 5°C for 10 to 15 minutes.

Then, an aqueous solution of 27% hydrogen peroxide is added to the resulting tertamyl sulfate at a temperature of 0 to 5°C, with vigorous stirring, in a molar ratio of tertamyl sulfate to hydrogen peroxide: (CH₃)₂C(HSO₄)CH₂CH₃:H₂O₂ = 1:0.5. After adding the entire amount of 27% hydrogen peroxide, the reaction mixture is further stirred at a temperature of 5 to 20°C for 60 minutes.

After the stirring is finished the reaction mass is divided into two layers. The upper layer, containing the formed di-tert-amyl peroxide is separated from the aqueous acidic layer and washed under stirring by a 10% aqueous solution of potassium carbonate and then by water to pH of 7. The crude di-tert-amyl peroxide washed from acid and alkali to neutral reaction is distilled under vacuum, at a pressure of 10 to 15 mm Hg, to collect the fraction boiling at 40 to 45°C. The yield of di tert-amyl peroxide after distillation is not less than 65%.

### Example 4. Radical addition of ethylene to isopropanol.

In a 2000 ml volume reactor, a steel autoclave of type "PARR", were placed 470 ml of isopropyl alcohol (369 g, 6.15 mol) and 4 g of di-tert-amyl peroxide. The reactor was purged 3 times by nitrogen at 5 atm and 2 times by ethylene at 5 atm. After purging, 12 to 15 liters of ethylene was fed into the reactor and the internal temperature was increased to 130 to 135°C. Absorbtion of ethylene begins at this temperature. While ethylene was being absorbed an additional amount of ethylene was fed in at a rate of 0.1 to 0.2 liters per minute to maintain the pressure in the system at 12 to 14 atm. About 30 to 32 liters of ethylene were absorbed during 4 hours of reaction. Then the reactor was cooled and an additional 4 g of di-tert-amyl peroxide was added. the autoclave was reheated to 130 to 135°C while maintaining the pressure in the system at 12 to 14 atm by feeding in ethylene. During 4 hours of reaction, an additional 20 to 22 liters of ethylene were absorbed. After cooling the reactor, 4 g of di-tert-amyl peroxide were added again and the experiment was repeated. During the entire experiment 84 liters, (105 g) of ethylene were consumed and 600 ml (480 g) of the product were obtained.

The mixture of alcohols in the amount of 600 ml thus obtained in the reaction of telomerization was subjected to distillation. After distillation at atmospheric pressure and a temperature of 80 to 90°C, 250 ml of isopropyl alcohol were obtained. The subsequent distillation yielded 340 ml of tertiary alcohols boiling at 100 to 250°C and 10 ml of tertiary alcohols boiling at a higher temperature, which remained in the bottoms. The yield of tertiary alcohols obtained from the reacted isopropyl alcohol, ethylene and tert-amyl peroxide was more than 99%.

The yield of tertiary C₅-C₇ alcohols was about 80%, and the yield of tertiary C₉-C₁₁ alcohols was about 17.1%. According to GLC analysis, the following was obtained: tert-amyl alcohol 57.4%; tert-heptyl alcohol 22.6%; tert-nonyl alcohol 11.4%, tertundecyl alcohol 5.7% and tert-C₁₁₊ alcohols 2.9%. The conversion of isopropyl alcohol reached about 50%. These experiments were carried out with a "deficiency" of ethylene in order to avoid formation of heavier telomeres.

### Example 5. Radical addition of ethylene to ethyl alcohol.

408 ml (322 g, 7.0 mol) of ethyl alcohol and 45 ml (37 g, 0.21 mol) of di tert-amyl peroxide were placed in a 2000 ml volume reactor, a steel autoclave of type "PARR". The reactor was purged 3 times by nitrogen at 5 atm and 2 times by ethylene at 5 atm. Then, 12 liter (0.5 mol) of ethylene was fed into the reactor and the internal temperature was increased to 130 to 135°C. At this temperature absorbtion of ethylene begins. While ethylene was being absorbed at 130 to 135°C, an additional amount of ethylene was fed in at a rate of 0.1 to 0.3 liters per minute to maintain the pressure in the system at 12 to 14 atm. During 8 hours of the reaction, about 95 liters, (119 g, 4.25 mol) of ethylene were absorbed. Then the reactor was cooled, the reaction mixture was unloaded and transferred to distillation. The above experiment was repeated twice. In total, 1224 ml, (966 g, 21 mol) of ethyl alcohol and 135 ml, (111 g, 0.64 mol,) of di tert-amyl peroxide were consumed to perform the reaction of telomerisation, while 285 liter (356 g, 12.71 mol) of ethylene were absorbed.

In total, 1793 ml (1433 g) of alcohol mixture was obtained.

The mixture of alcohols in the amount of 1793 ml obtained as a result of the telomerization reaction was distilled. Distillation at atmospheric pressure and a temperature of 80°C yielded 776 ml (612 g, 13.3 mol) of ethyl alcohol. Further distillation at atmospheric pressure and a temperature of 98°C yielded 450 ml (364 g, 4.95 mol) of sec-butyl alcohol. Further distillation at atmospheric pressure additionally yielded 528 ml (425 g) of tertiary C₅-C₈ alcohols boiling between 102 and 180°C, and 30 ml (25 g) of tertiary alcohols boiling at higher temperatures, which remained in the bottoms.

The yield of secondary butyl alcohol and tertiary alcohols obtained from the reacted ethyl alcohol, ethylene and di-tert-amyl peroxide was more than 99%. The yield of secondary butyl alcohol was 44.3%. The yield of tertiary C₅-C₈ alcohols was 51.8%, and the yield of tertiary C₁₀-C₁₂ alcohols was about 3.0%. The rate of ethyl alcohol conversion was about 37%.

### Example 6. Hydroformylation of ethylene in the presence of a rhodium catalyst.

The process of ethylene hydroformylation is carried out in a heterogeneous reaction medium using a water-soluble rhodium catalyst.

During hydroformylation of ethylene in the presence of a rhodium catalyst, the concentration of metal with respect to the aqueous phase is from 30 to 50 ppm. Triphenylphosphine-3-sulfonic acid sodium salt is used as a ligand in a ratio of 30:1 to metallic rhodium.

The process of obtaining propionic aldehyde is carried out at a temperature of 45 to 90°C and a pressure of 1.0 to 2.5 MPa. The ratio of the source gases: C₂H₄:CO:H₂ = (0.9-1):1:1 is maintained by flow meters. The resulting reaction mass is directed to a highly efficient separator where it is separated into three phases: a gas phase containing non-reacted ethylene, carbon monoxide and hydrogen, a liquid organic phase containing mainly propanal, and a liquid aqueous phase containing a water-soluble rhodium catalyst. The gas phase and the liquid aqueous phase are returned to the hydroformylation reactor by metering devices.

An aqueous solution of the catalyst was loaded into a 2000 ml volume reactor with a highly efficient stirring device. The catalyst was then activated as follows. Synthesis gas was supplied in the ratio CO:H₂ = 1:1 into the reactor whilst stirring the catalyst solution until a pressure of 1.0 MPa was reached, after which the heating was switched on and the temperature was increased to 60 to 70°C. The catalyst solution was stirred for one hour at this temperature. Upon completion of catalyst activation, an automatic mode of the reaction temperature and pressure limit control was switched on at the instrument panel, which terminates supply of the reaction gases to the reactor. To prevent heating of the reaction mixture to a temperature critical for catalyst operation, the reactor is equipped with a refrigerator providing for removal of the excess heat. Water is used as the refrigerant. The gas supply rates for ethylene, carbon monoxide, and hydrogen were set at the flow meters installed on the gas supply lines into the reactor, and after that the gas supply to the reactor was switched on. After reaching the required volume of liquid reaction mass in the reactor, the pump was turned on to provide continuous pumping of the excess reaction mass to the separator. The separator separated the vapour and liquid phases, as well as separated the liquid phase into an organic phase containing propionic aldehyde and an aqueous phase containing dissolved catalyst.

The optimum temperature for the process of hydroformylation is 45 to 90°C at a pressure of 1.0 to 2.5 MPa. With a decrease in temperature below 45°C, a significant decrease in the yield of the target aldehyde was observed in all experiments. With an increase of temperature above 90°C, a decrease in conversion was also observed, however it was less significant. The experiments were carried out in the range of pressures of 1.0-2.5 MPa. It should be noted that all experiments were performed using one and the same sample of catalyst developed by the inventor. During the study, the catalyst was repeatedly separated from the reaction products and returned to the process. Its total service life exceeded 9000 hours. The catalyst demonstrated high selectivity in the experiments on ethylene hydroformylation. Conversion of the reaction gases at the optimal technological parameters exceeded 99.0%, the selectivity for aldehyde was higher than 99.5%.

### Example 7. Aldol condensation of propanal and obtaining propyl and 2-methylpentyl alcohols.

Propanal obtained in the hydroformylation of ethylene by synthesis gas in the presence of a rhodium catalyst was supplied at a temperature of 100 to 150°C and a pressure of 0.5 to 1.0 MPa into a one-liter flow reactor loaded with a granular catalyst containing at least 93% of ZSM-5 zeolite modified by 3.5 to 7.0% Zn. These conditions provide for aldol condensation of propanal and obtaining 2-methyl pentenal and C₆₊ products of propanal condensation. The reaction mass obtained in the process of aldol condensation was supplied for hydrogenation into a flow reactor, where the catalyst containing oxides NiO, CuO and Cr₂O₃ in a molar ratio of 1:1:1 was loaded. Simultaneously with the reaction mass of the aldol condensation process, hydrogen is also supplied to the said reactor, equipped with catalyst consisting of the oxides NiO, CuO and Cr₂O₃ in a molar ratio of 1:1:1, at a temperature of 90 to 130°C and a pressure of 4.0 to 5.0 MPa.

By way of hydrogenation, 2-methyl pentenal as well as the unreacted propanal and the resulting C₆₊ products of condensation are converted into 2-methylpentanol, propyl alcohol, and C₆₊ alcohols, respectively.

The rate of propanal conversion into 2-methyl pentenal in the process of aldol condensation was at least 50%. The total yield of the corresponding alcohols, propyl, 2-methyl pentyl, and C₆₊ alcohols, in the hydrogenation of a mixture of propanal, 2-methyl pentenal, and C₆₊ products of propanal condensation, exceeded 99%.

### Example 8. Hydroformylation of ethylene using a cobalt catalyst.

The hydroformylation of ethylene is performed in a heterogeneous reaction medium in the presence of a water-soluble cobalt catalyst.

During hydroformylation of ethylene in the presence of a cobalt catalyst, the concentration of metal in relation to the aqueous phase is from 0.1% to 0.15%. Triphenylphosphine-3,3',3"-trisulfonic acid trisodium salt is used in the weight ratio 10:1 to metallic cobalt as a ligand.

The process of obtaining propionic aldehyde is performed at a temperature of 120 to 140°C and a pressure of 3.0 to 5.0 MPa. The ratio of the source gases: C₂H₄:CO:H₂ = (0.9-1):1:1 is maintained by flow meters. The resulting reaction mass is directed to a highly efficient separator for separation into three phases: a gas phase containing unreacted ethylene, carbon monoxide and hydrogen, a liquid organic phase containing resulting products of chemical conversions; propanal, 2-methyl pentenal, 2-methyl pentanal and C₆₊ products of propanal condensation, and a liquid aqueous phase containing a water-soluble cobalt catalyst. The liquid aqueous phase is returned to the hydroformylation reactor by metering pump. The gas phase is directed to the stage of synthesis gas production.

The liquid organic phase from the separator is directed to the rectification column for isolation of the reaction products: propanal, 2-methyl pentenal, 2-methyl pentanal and C₆₊ products of propanal condensation, as well as water dissolved in this organic phase.

The organic products isolated at the rectification column were sent to hydrogenation to obtain the corresponding alcohols. Isolated at the rectification column water, dissolved in organic matter, accumulates at the bottom of the column and is periodically pumped into the hydroformylation reactor by a pump.

An aqueous solution of the catalyst was put in a 2000 ml reactor with a highly efficient stirring device. The catalyst was then activated. The activation was done as follows. Synthesis gas was supplied into the reactor in the ratio CO:H₂ = 1:1, while stirring the catalyst solution, until a pressure of 2.0 MPa was reached. Then the heating was switched on and the temperature was increased to 120 to 130°C. The catalyst solution was stirred at this temperature for two hours. Upon completion of catalyst activation, an automatic mode of reaction temperature and pressure limit control was switched on at the instrument panel, which terminates supply of the reaction gases to the reactor. To prevent heating of the reaction mixture to the critical temperature of catalyst operation, the reactor is equipped with a refrigerator for removal of the excess heat. Water is used as a refrigerant. The gas supply rates for ethylene, carbon monoxide, and hydrogen were set at the flow meters installed on the gas supply lines into the reactor, and then the gas supply to the reactor was switched on. After reaching the required volume of liquid reaction mass in the reactor, the pump was turned on to provide continuous pumping of the excess reaction mass to the separator. The separator separated the vapor and liquid phases, and also separated the liquid phase into an organic phase containing propanal; 2-methyl pentenal; 2-methyl pentanal and C₆₊ products of propanal condensation, and into water, in which the catalyst is dissolved.

The optimum temperature for the hydroformylation process is 120 to 130°C. A decrease in temperature below 120°C results in a significant decrease in the yield of the target products in all experiments. An increase of the temperature above 130°C also results in a decrease of the conversion rate, though less significant. The experiments were carried out over the pressure range of 3.0 to 5.0 MPa. It should be noted that all experiments were carried out on one and the same sample of the inventive catalyst. During the study, the catalyst was repeatedly isolated from the reaction products and returned back to the process. Its total service life exceeded 1200 hours. The catalyst demonstrated high selectivity in the experiments on ethylene hydroformylation. Conversion of the reaction gases at optimal technological parameters was higher than 98.0%, the selectivity for propanal reached 99.0%, and the selectivity for the totality of the products of propanal aldol condensation, that is 2-methyl pentenal, 2-methyl pentanal and the products of C₆₊ propanal condensation reached 50%.

### Example 9. Obtaining propyl, 2-methylpentyl and C₆₊ alcohols.

Propanal, 2-methyl pentenal, 2-methyl pentanal and the products of C₆₊ propanal condensation, obtained in the hydroformylation of ethylene by synthesis gas in the presence of a cobalt catalyst, were supplied at a temperature of 150 to 200°C and a pressure of 4.0 to 5.0 MPa into a continuous flow, one liter reactor where a granular catalyst consisting of the oxides NiO, CuO and Cr₂O₃ in a molar ratio of 1:1:1, is loaded. Simultaneously with propanal, 2-methyl pentenal, 2-methyl pentanal and the C₆₊ products of propanal condensation, hydrogen is also supplied into the reactor. By way of hydrogenation, propanal, 2-methyl pentenal, 2-methyl pentanal, and the C₆₊ propanal condensation products are converted into propyl, 2-methylpentyl, and C₆₊ alcohols, respectively. The total yield of the corresponding alcohols: propanol, 2-methyl-pentanol, and C₆₊ alcohols in the process of hydrogenation of a mixture of propanal, 2-methyl pentenal, 2-methyl pentanal, and C₆₊ propanal condensation products exceeded 99%.

### Example 10. Hydroformylation of propylene in the presence of a cobalt catalyst.

The process of propylene hydroformylation is performed in a heterogeneous reaction medium using a water-soluble cobalt catalyst. During hydroformylation of propylene in the presence of a cobalt catalyst, the concentration of metal in relation to the aqueous phase is from 0.25%mass. to 0.5% mass. Triphenylphosphine-3-sulfonic acid sodium salt is used as a ligand in a weight ratio of 7:1 to metallic cobalt. To increase the rate of the propylene hydroformylation, as well as to increase the yield of isobutanal, ethanol is added to the water-soluble cobalt catalyst in the volume ratio of H₂O:C₂H₅OH = (0.75-0.65):( 0.25-0.35).

The process of obtaining butyric aldehydes is carried out at a temperature of 135°C to 140°C and a pressure of 3.0 to 5.0 MPa. The ratio of the source gases: C₃H₆:CO:H₂ = (0.9:1):1:1 is maintained by flow meters. The resulting reaction mass is directed to a highly efficient separator where it is separated into three phases: vapor, liquid organic and liquid water phase. The vapor phase containing the unreacted propylene, carbon monoxide and hydrogen is sent to the stage of synthesis gas production.

The liquid organic phase obtained in the chemical conversion, containing butanal, isobutanal, 2-ethyl hexenal, 2-ethyl hexanal, and the C₈₊ products of butanal condensation, is transferred from the separator to the rectification column. The rectification column is used to separate the following reaction products: butanal, isobutanal, 2-ethyl hexenal, 2-ethyl hexanal, C₈₊ products of butanal condensation, and water dissolved in this organic phase.

The liquid aqueous phase containing the water-soluble cobalt catalyst is returned back to the hydroformylation reactor by metering pump.

Organic products isolated by rectification were sent to hydrogenation to obtain the corresponding alcohols. Isolated by the distillation column water, dissolved in organic matter, accumulated in the bottom of the column and was periodically supplied by the pump to the hydroformylation reactor.

The experiments were carried out as follows. An aqueous solution of the catalyst was loaded into a 2000 ml reactor with an integrated highly efficient stirring device. The catalyst was then activated, it was done as follows. Synthesis gas was supplied in the ratio CO:H₂ =1:1 into the reactor whilst stirring of the catalyst solution until a pressure of 2.0 MPa was reached, after which the heating was switched on and the temperature was increased to 140°C. The catalyst solution was stirred for two hours at this temperature. Upon completion of the catalyst activation, an automatic mode of control of reaction temperature and pressure limit was switched on at the instrument panel, which terminated supply of the reaction gases to the reactor. To prevent heating of the reaction mixture to the critical temperature of catalyst operation, the reactor is equipped with a refrigerator for removal of the excess heat. Water is used as a refrigerant. The gas supply rates for propylene, carbon monoxide, and hydrogen were set at the flow meters installed on the gas supply lines to the reactor and the gas supply was switched on. After reaching the required volume of liquid reaction mass in the reactor, the pump was turned on to provide continuous pumping of the excess reaction mass to the separator. The separator separated the vapor and liquid phases, as well as separated the liquid phase into an organic phase containing butanal, isobutanal, 2-ethyl hexenal, 2-ethyl hexanal, and the C₈₊ products of 2-ethyl hexanal condensation, and into an aqueous phase containing dissolved catalyst. It should be noted that the ratio of butanal and isobutanal formed in the reaction mass during the entire experiment was within the ratio (n-C₄H₈O):(iso-C₄H₈O) = (2-3):1.

The optimum temperature for the hydroformylation process is 138 to 140°C. The experiments were performed over the pressure range of 3.0 to 5.0 MPa. It should be noted that all experiments were performed by using one and the same sample of the developed catalyst. In the course of the studies performed by the inventor, the catalyst was repeatedly separated from the reaction products, and returned back to the process. Its total service life exceeded 240 hours. The catalyst demonstrated high selectivity in the experiments on the hydroformylation of propylene. Conversion of the reaction gases at the optimal technological parameters was above 98.0%, the selectivity for butyric aldehydes reached 99.0%. Moreover, the selectivity for the totality of the products of n-butanal aldol condensation: 2-ethyl hexenal, 2-ethyl hexanal, and the C₈₊ products of the 2-ethyl hexanal condensation exceeded 60%.

### Example 11. Obtaining butyl, isobutyl, and 2-ethylhexyl alcohols.

Butanal, isobutanal, 2-ethyl hexenal, and 2-ethyl hexanal, obtained in the hydroformylation of propylene by synthesis gas in the presence of a cobalt catalyst, were isolated from the reaction mixture by rectification. The mixture of oxygen-containing products thus obtained was supplied at a temperature of 90 to 130°C and a pressure of 4.0 to 5.0 MPa to a one-liter flow reactor where a granular catalyst, containing oxides NiO, CuO and Cr₂O₃ in a molar ratio of 1:1:1 was loaded. Hydrogen was also fed to the reactor simultaneously with butanal, isobutanal, 2-ethyl hexenal and 2-ethyl hexanal. In the course of hydrogenation, butanal, isobutanal, 2-ethyl hexenal, and 2-ethyl hexanal are converted to butyl, isobutyl, and 2-ethyl hexyl alcohols, respectively. The total yield of the corresponding alcohols, that is butyl, isobutyl, and 2-ethyl hexyl alcohol exceeded 99%.

### Example 12. Obtaining kerosene from propyl alcohol.

Propyl alcohol is dehydrated in contact with the gamma Al₂O₃ catalyst at a temperature of 350 to 400°C and a pressure of 1.0 to 2.0 MPa. The obtained propylene (C₃H₆) is separated from water and directed at a temperature of 250-350°C and a pressure of 3.0-5.0 MPa into a continuous flow reactor, equipped with the granular catalyst, containing at least 93% of ZSM-5 zeolite modified by 3.5 to 7.0% Zn, or a granular catalyst containing at least 95% of ZSM-5 zeolite modified by 3.5 to 5.0% Zn and 0.1 to 1.5% Ce. These conditions provide for oligomerization of propylene to yield C₆-C₂₄ olefins. Unreacted propylene is separated by distillation from the reaction mass of the oligomerization process and returned back to the process. The reaction mass, free of the source propylene, is directed for hydrogenation to a continuous flow reactor equipped with the granular catalyst consisting of the oxides NiO, CuO and Cr₂O₃ in a molar ratio of 1:1:1. Simultaneously with the products of oligomerization, hydrogen at 120-150°C and a pressure of 4.0-5.0 MPa is supplied to the hydrogenation. The process of hydrogenation provides for the conversion of the unsaturated C₆-C₂₄ compounds obtained at the stage of oligomerization into C₆-C₂₄ paraffins. The reaction mass obtained at the stage of hydrogenation goes to rectification for separation into kerosene, as well as gasoline and diesel fractions.

Table 2 shows the main properties of the kerosene fraction free of aromatic compounds.

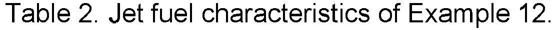

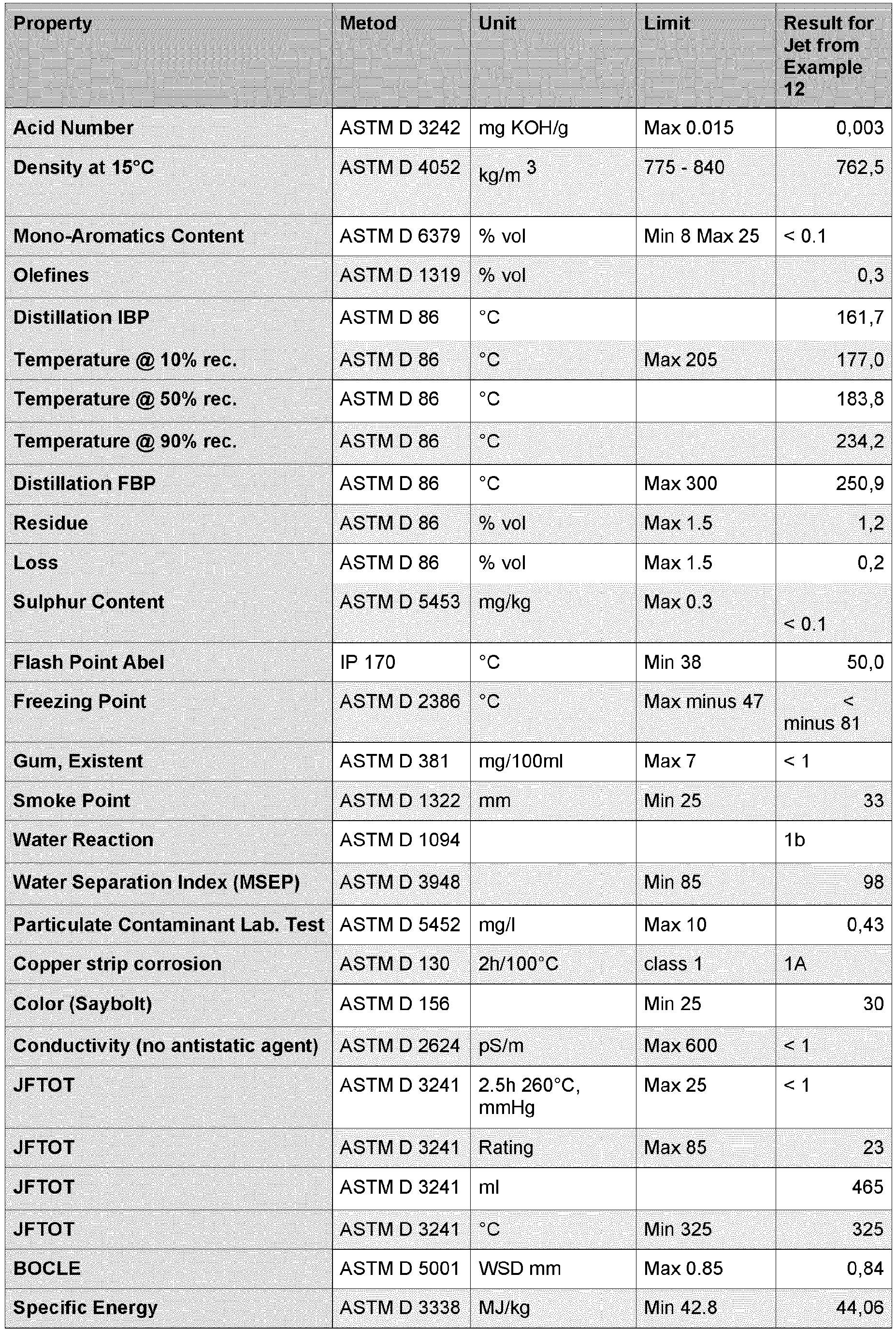

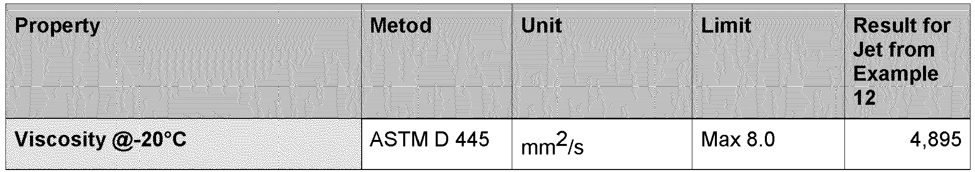

The study performed by the inventor using chromatography-mass spectroscopy demonstrates, that the composition of kerosene, obtained solely from propylene, contains more than 160 different C₈-C₁₆ isomers, wherein 4.7 wt. % of those are linear hydrocarbons.

### Example 13. Obtaining kerosene from isobutyl alcohol.

Isobutyl alcohol obtained by hydroformylation of propylene and subsequent hydrogenation of the resulting isobutyraldehyde is dehydrated in contact with the gamma Al₂O₃ catalyst at a temperature of 350 to 400°C and a pressure of 1.0 to 2.0 MPa. The isobutylene (C₄H₈) obtained is separated from water and supplied at a temperature of 250 to 350°C and a pressure of 3.0 to 5.0 MPa into a continuous flow reactor, equipped with granulated catalyst containing at least 95% zeolite ZSM-5 modified by 3.5-5.0% Zn and 0.1-1.5% Ce. These conditions provide for oligomerization of isobutylene to yield C₈-C₂₀ olefins. The unreacted isobutylene is distilled off the reaction mass obtained by oligomerization and returned back to the process. The reaction mass, free from the source isobutylene, is supplied for hydrogenation to the continuous flow reactor, equipped with catalyst consisting of the oxides NiO, CuO, and Cr₂O₃ in a molar ratio 1:1:1. Simultaneously with olefins obtained by oligomerization, hydrogen is supplied into the hydrogenation reactor at a temperature of 120 to 150°C and a pressure of 4.0 to 5.0 MPa. By means of hydrogenation, the unsaturated C₈-C₂₀ compounds obtained at the stage of oligomerization are converted into C₈-C₂₀ paraffins. The reaction mass obtained at the stage of hydrogenation is directed to rectification for separation into kerosene, as well as gasoline and diesel fractions.

### Example 14. Obtaining kerosene from tert-amyl alcohol.

Tert-amyl alcohol (2-methyl-2-butanol) is dehydrated in contact with gamma Al₂O₃ catalyst at a temperature of 100-150°C and a pressure of 1.0 to 2.0 MPa. The pentenes (C₅H₁₀) obtained are separated from water and supplied at a temperature of 100-150°C and a pressure of 1.0 to 2.0 MPa into a continuous flow reactor, equipped with an ion-exchange resin in the form of a cation exchanger Amberlite 15, as catalyst. These conditions provide for oligomerization of pentenes to yield C₁₀-C₂₀ olefins. The unreacted pentenes are distilled off the reaction mass obtained by oligomerization and returned back to the process. The reaction mass, free of the source pentenes, is supplied for hydrogenation into a continuous flow reactor, equipped with the catalyst comprised by oxides NiO, CuO, and Cr₂O₃ in a molar ratio 1:1:1. Simultaneously with the oligomerization products, hydrogen at a temperature of 120 to 150°C and a pressure of 4.0 to 5.0 MPa is supplied to the hydrogenation reactor. The unsaturated C₁₀-C₂₀ compounds obtained at the oligomerization stage are hydrogenated into C₁₀-C₂₀ paraffins. The yield of C₁₀-C₂₀ paraffins in terms of oligomerized pentenes is above 98%. The reaction mass obtained at the stage of hydrogenation is then separated by rectification into kerosene, as well as gasoline and diesel fractions, free of aromatic compounds. The iso- structure of the C₁₀-C₂₀ paraffins provides for producing kerosene and diesel demonstrating unique properties, as well as gasoline with octane number RON of at least 95 and an octane number MON of at least 91. Results of the studies by using chromatography-mass spectroscopy demonstrate, that the kerosene obtained from tert-amyl alcohol contains 45 different C₁₀-C₁₅ isomers and is free of linear hydrocarbons. Kerosene, obtained from tert-amyl alcohol, does not freeze at minus 85°C, and has a Smoke point of 33 millimeters.

### Example 15. Obtaining kerosene from 2-methyl-1-pentanol.

2-methyl pentyl alcohol is dehydrated in contact with the catalyst gamma Al₂O₃ at a temperature of 350 to 450°C and a pressure of 1.0 to 2.0 MPa. The hexenes (C₆H₁₂) obtained by dehydration of alcohol are separated from water and supplied at a temperature of 250 to 350°C and a pressure of 3.0 to 5.0 MPa into a continuous flow reactor, equipped with the catalyst containing at least 95% of ZSM-5 zeolite modified by 3.5 -5.0% Zn and 0.1-1.5% Ce. Oligomerization of the source hexenes yielding C₁₂-C₂₄ olefins is performed in the presence of the said catalyst at the specified process parameters. The unreacted hexenes are distilled off the reaction mixture and returned back to the process. The reaction mass, free of the source hexenes, is directed for hydrogenation to a continuous flow reactor, equipped with the catalyst consisting of the oxides NiO, CuO, and Cr₂O₃ in a molar ratio of 1:1:1. Simultaneously with the C₁₂-C₂₄ olefins, hydrogen at a temperature of 120 to 150°C and a pressure of 4.0 to 5.0 MPa is supplied to the hydrogenation reactor. The C₁₂-C₂₄ olefins obtained at the oligomerization stage are converted into C₁₂-C₂₄ paraffins. The yield of C₁₂-C₂₄ paraffins in terms of oligomerized hexenes is above 98%. The reaction mass obtained at the stage of hydrogenation is directed to rectification, where it is separated into kerosene and diesel fractions.

### Example 16. Obtaining kerosene from terthexyl alcohol.

Terthexyl alcohol (3-methyl-3-pentanol) is dehydrated in contact with the catalyst gamma Al₂O₃ at a temperature of 100-150°C and a pressure of 1.0 to 2.0 MPa. The hexenes (C₆H₁₂) formed are separated from water and supplied at a temperature of 100-150°C and a pressure of 1.0 to 2.0 MPa into a continuous flow reactor, equipped with an ion-exchange resin in the form of a cation exchanger Amberlite 15, as the catalyst. Oligomerization of the source hexenes yielding C₁₂-C₁₈ olefins is carried out in the presence of the ion-exchange resin in the form of a cation exchanger Amberlite 15 as the catalyst at the specified process parameters. Unreacted hexenes are distilled off the reaction mass obtained by oligomerization and returned back to the process. The reaction mass, free of the source hexenes, is supplied for hydrogenation to a continuous flow reactor, equipped with the catalyst consisting of the oxides NiO, CuO, and Cr₂O₃ in a molar ratio of 1:1:1. Simultaneously with the C₁₂-C₁₈ olefins, hydrogen is supplied to the hydrogenation reactor at a temperature of 120 to 150°C and a pressure of 4.0 to 5.0 MPa. The C₁₂-C₁₈ olefins obtained at the stage of oligomerization are hydrogenated into C₁₂-C₁₈ paraffins. The mixture of C₁₂-C₁₈ paraffins thus obtained is a kerosene fraction, free of aromatic compounds. The yield of C₁₂-C₁₈ paraffins, in terms of oligomerized hexenes, exceeds 98%. Results of the studies, performed by using chromatography-mass spectroscopy, demonstrate that kerosene, obtained from tert-hexyl alcohol, contains 48 different C₁₂-C₁₈ isomers and is free of linear hydrocarbons.

### Example 17. Obtaining kerosene from 3-ethyl-3-pentanol.

3-ethyl-3-pentanol is dehydrated in contact with the catalyst gamma Al₂O₃ at a temperature of 100-150°C and a pressure of 1.0 to 2.0 MPa. The heptenes (C₇H₁₄) obtained are separated from water and supplied at a temperature of 100-150°C and a pressure of 1.0 to 2.0 MPa into a continuous flow reactor, equipped with an ion-exchange resin in the form of a cation exchanger Amberlite 15, as catalyst. Oligomerization of the source heptenes is carried out in the presence of the ion-exchange resin in the form of a cation exchanger Amberlite 15 catalyst with the specified process parameters, to yield C₁₄-C₂₁ olefins. Unreacted heptenes are distilled off the reaction mass obtained by oligomerization, and returned back to the process. The reaction mass, free of the source heptenes, is supplied for hydrogenation in a continuous flow reactor, equipped with the catalyst consisting of the oxides NiO, CuO, and Cr₂O₃ in a molar ratio of 1:1:1. Simultaneously with the C₁₄-C₂₁ olefins, hydrogen is supplied to the hydrogenation reactor at a temperature of 120 to 150°C and a pressure of 4.0 to 5.0 MPa. The C₁₄-C₂₁ olefins obtained at the oligomerization stage are hydrogenated into C₁₄-C₂₁ paraffins. The reaction mass obtained at the stage of hydrogenation is directed to rectification, and is separated into kerosene and diesel fractions, free from aromatic compounds. The yield of C₁₂-C₁₈ paraffins, in terms of oligomerized hexenes, exceeds 98%. Results of the studies, performed by using chromatography-mass spectroscopy, demonstrate that kerosene, obtained from tert-hexyl alcohol, contains 48 different C₁₂-C₁₈ isomers and is free of linear hydrocarbons.

### Example 18. Obtaining kerosene from tert-heptyl alcohol.

2-Methyl-hexanol-2 (dimethyl butyl carbinol) is dehydrated in contact with the catalyst gamma Al₂O₃ at a temperature of 100-150°C and a pressure of 1.0 to 2.0 MPa. The obtained heptenes (C₇H₁₄) are separated from water and supplied at a temperature of 100-150°C and a pressure of 1.0 to 2.0 MPa into a continuous flow reactor, equipped with the catalyst, an ion-exchange resin in the form of a cation exchanger Amberlite 15. These conditions provide for oligomerization of heptenes yielding C₁₄-C₂₁ olefins. The unreacted heptenes are distilled off the reaction mass obtained in the process of oligomerization, and returned back to the process. The reaction mass, free from the source heptenes, is supplied for hydrogenation to a continuous flow reactor, equipped with the catalyst consisting of the oxides NiO, CuO, and Cr₂O₃ in a molar ratio of 1:1:1. Simultaneously with the oligomerization products, that is the C₁₄-C₂₁ olefins, hydrogen at a temperature of 120 to 150°C and a pressure of 4.0 to 5.0 MPa is supplied into the hydrogenation reactor. The C₁₄-C₂₁ olefins obtained at the oligomerization stage are converted into C₁₄-C₂₁ paraffins. The reaction mass obtained at the stage of hydrogenation is directed to rectification, and is separated into kerosene and diesel fractions, free of aromatic compounds. The yield of C₁₄-C₂₁ paraffins, in terms of oligomerized heptenes, exceeds 95%. Results of the studies, performed by using chromatography-mass spectroscopy, demonstrate that kerosene, obtained from tertheptyl alcohol, contains 28 different C₁₄-C₁₈ isomers and is free of linear hydrocarbons.

### Example 19. Obtaining kerosene from 2-ethyl-hexyl alcohol.

2-ethyl-hexyl alcohol (2-ethyl-hexanol-1) is dehydrated in contact with the catalyst gamma Al₂O₃ at a temperature of 350 to 400°C and a pressure of 0.5 to 2.0 MPa to yield octenes. The octenes (C₈H₁₆) are separated from water and supplied at a temperature of 150 to 200°C and a pressure of 1.0 to 2.0 MPa into a continuous flow reactor, equipped with the granular catalyst containing at least 95% of ZSM-5 zeolite modified by 3.5-5.0% Zn and 0.1-1.5% Ce. These conditions provide for oligomerization of octenes to yield C₁₆-C₂₄ olefins. Unreacted octenes are distilled off from the reaction mass obtained by oligomerization, and returned back to the process. The reaction mass, free of the source octenes, is supplied for hydrogenation to a continuous flow reactor, equipped with the catalyst consisting of the oxides NiO, CuO, and Cr₂O₃ in a molar ratio of 1:1:1. Simultaneously with the C₁₆-C₂₄ olefins, hydrogen is supplied into the hydrogenation reactor at a temperature of 250 to 350°C and a pressure of 4.0 to 5.0 MPa. The C₁₆-C₂₄ olefins obtained at the oligomerization stage are hydrogenated into C₁₆-C₂₄ paraffins. The yield of C₁₆-C₂₄ paraffins, in terms of oligomerized octenes, exceeds 95%. Then the reaction mass obtained at the stage of hydrogenation is separated by rectification into kerosene and diesel fractions. Aromatic compounds content in these fractions does not exceed 5.0% mass.

### Example 20. Obtaining kerosene from a mixture of tert-amyl and tert-hexyl alcohols.

A mixture of tert-amyl and tert-hexyl alcohols in a molar ratio of 1:1 is dehydrated in contact with the catalyst gamma Al₂O₃ at a temperature of 100-150°C and a pressure of 1.0 to 2.0 MPa. The resulting mixture of olefins (C₅H₁₀ + C₆H₁₂) is separated from water and supplied at a temperature of 100 to 150°C and a pressure of 1.0 to 2.0 MPa into a continuous flow reactor, equipped with the ion-exchange resin in the form of a cation exchanger Amberlite 15, as catalyst. These conditions provide for oligomerization of pentenes and hexenes to yield C₁₀-C₂₄ olefins. The unreacted pentenes and hexenes are distilled off from the reaction mass obtained by oligomerization and returned back to the process. The reaction mass, free of the source pentenes and hexenes, is supplied for hydrogenation to a continuous flow reactor, equipped with the catalyst consisting of the oxides NiO, CuO, and Cr₂O₃ in a molar ratio of 1:1:1. Simultaneously with the products of oligomerization, hydrogen at a temperature of 120 to 150°C and a pressure of 4.0 to 5.0 MPa is supplied to the hydrogenation reactor. The unsaturated C₁₀-C₂₄ compounds obtained at the oligomerization stage are hydrogenated into C₁₀-C₂₄ paraffins. After that the reaction mass obtained at the stage of hydrogenation is separated by rectification into kerosene, as well as gasoline and diesel fraction, free of aromatic compounds.

The yield of C₁₀-C₂₄ paraffins, in terms of oligomerized pentenes and hexenes, exceeds 99%. Results of the studies, performed by using chromatography-mass spectroscopy, demonstrate that kerosene, obtained from tert-amyl and tert-hexyl alcohols, contains 78 different C₁₀-C₂₀ isomers and is free of linear hydrocarbons.

### Example 21. Obtaining kerosene from a mixture of tert-amyl, tert-hexyl and tert-heptyl alcohols.

A mixture of tert-amyl, tert-hexyl and tert-heptyl alcohols in a molar ratio of 1:1:1 is dehydrated in contact with the catalyst gamma Al₂O₃ at a temperature of 100-150°C and a pressure of 1.0 to 2.0 MPa. The resulting mixture of olefins (C₅H₁₀ + C₆H₁₂ + C₇H₁₄) is separated from water and supplied at a temperature of 100 to 150°C and a pressure of 1.0 to 2.0 MPa into a continuous flow reactor, equipped with the ion-exchange resin in the form of a cation exchanger Amberlite 15, as catalyst. These conditions provide for oligomerization of pentenes, hexenes and heptenes to yield C₁₀-C₂₄ olefins. The unreacted pentenes, hexenes and heptenes are distilled off from the reaction mass obtained by oligomerization, and returned back to the process. The reaction mass, free of the source pentenes, hexenes and heptenes, is directed for hydrogenation to a continuous flow reactor, equipped with the catalyst consisting of the NiO, CuO, and Cr₂O₃ in a molar ratio of 1:1:1. Simultaneously with the products of oligomerization, hydrogen at a temperature of 120 to 150°C and a pressure of 4.0 to 5.0 MPa is supplied to the hydrogenation reactor. The unsaturated C₁₀-C₂₄ compounds obtained at the oligomerization stage are hydrogenated into C₁₀-C₂₄ paraffins. After that, the reaction mass obtained at the stage of hydrogenation is separated by rectification into C₁₁-C₁₈ kerosene, and C₁₉-C₂₄ diesel fractions, free of aromatic compounds. The yield of C₁₀-C₂₄ paraffins, in terms of oligomerized C₅-C₇ olefins, exceeds 99%. Results of the studies, performed by using chromatography-mass spectroscopy, demonstrate that kerosene, obtained from tert-amyl, tert-hexyl, and tert-heptyl alcohols, contains 101 different C₁₀-C₂₀ isomers and is free of linear hydrocarbons.

### Example 22. Obtaining kerosene from a mixture of propyl and 2-methyl pentyl alcohols.

A mixture of propyl and 2-methyl pentyl alcohols in a molar ratio (1.0-1.5):1 is dehydrated in contact with the catalyst gamma Al₂O₃ at a temperature of 350 to 450°C and a pressure of 1.0 to 2.0 MPa. The resulting mixture of olefins (C₃H₆ + C₆H₁₂) is separated from water and supplied at a temperature of 250 to 350°C and a pressure of 3.0 to 5.0 MPa into a continuous flow reactor, equipped with the granular catalyst containing at least 95% of ZSM-5 zeolite modified by 3.5 -5.0% Zn and 0.1-1.5% Ce. These conditions provide for oligomerization of propylene and hexenes to yield C₆-C₂₄ olefins. The unreacted propylene and hexenes are distilled off from the reaction mass obtained by oligomerization, and returned back to the process. The reaction mass, free from the source propylene and hexenes, is supplied for hydrogenation to a continuous flow reactor, equipped with the catalyst consisting of the NiO, CuO, and Cr₂O₃ in a molar ratio of 1:1:1. Simultaneously with the oligomerization products, hydrogen at a temperature of 120-150°C and a pressure of 4.0-5.0 MPa is supplied to the hydrogenation reactor. The unsaturated C₆-C₂₄ compounds obtained at the oligomerization stage are hydrogenated into C₆-C₂₄ paraffins. The reaction mass obtained at the stage of hydrogenation is then separated by rectification into C₁₁-C₁₈ kerosene, as well as C₆-C₁₀ gasoline and C₁₉-C₂₄ diesel fractions, with aromatic compounds content of not more than 5.0% mass.

### Example 23. Obtaining kerosene from a mixture of propyl, isobutyl, 2-methyl pentyl and 2-ethyl hexyl alcohols.

A mixture of propyl, isobutyl, 2-methyl pentyl and 2-ethyl hexyl alcohols in a molar ratio of (1.0-1.5): 1:1:(1.0-1.5) is dehydrated in contact with the catalyst gamma Al₂O₃ at a temperature of 350 to 450°C and a pressure of 1.0 to 2.0 MPa. The resulting mixture of olefins (C₃H₆ + C₄H₈ + C₆H₁₂ + C₈H₁₆) is separated from water and directed at a temperature of 250 to 350°C and a pressure of 3.0 to 5.0 MPa into a continuous flow reactor, equipped with the granular catalyst containing at least 95% of ZSM-5 zeolite modified by 3.5-5.0% Zn and 0.1-1.5% Ce. These conditions provide for oligomerization of propylene, isobutylene, isohexenes and isooctenes to yield C₆-C₂₄ olefins. The unreacted propylene and isobutylene are distilled off from the reaction mass obtained by oligomerization and returned back to the process. The reaction mass, free from the source propylene and isobutylene, is supplied for hydrogenation to a continuous flow reactor, equipped with the catalyst consisting of the oxides NiO, CuO, and Cr₂O₃ in a molar ratio of 1:1:1. Simultaneously with the oligomerization products, hydrogen at a temperature of 120 to 200°C and a pressure of 4.0 to 5.0 MPa is supplied to the hydrogenation reactor. The unsaturated C₆-C₂₄ compounds obtained at the oligomerization stage are hydrogenated into C₆-C₂₄ paraffins. Said C₆-C₂₄ paraffins are separated by rectification into C₁₁-C₁₈ kerosene, C₆-C₁₀ gasoline and C₁₉-C₂₄ diesel fractions. Tables 3, 4, and 5 demonstrate the properties of the hydrocarbon fractions obtained by the inventive process, and having an aromatic compound content of not more than 5% mass. The yield of C₆-C₂₄ paraffins, in terms of oligomerized C₃₋C₈ olefins, exceeds 99%.

Results of the studies, performed by using chromatography-mass spectroscopy, demonstrate that kerosene, obtained from propyl, isobutyl, 2-methyl pentyl and 2-ethyl hexyl alcohols, contains 134 different C₈-C₁₈ isomers, wherein 4.2% mass of those are linear hydrocarbons.

**Table 3. Properties of gasoline of Example 23.**

| **Property** | **Method** | **Unit** | **Limit** | **Result for gasoline of Example 23** |
|---|---|---|---|---|
| **Research octane number RON** | EN ISO 5164 | | Min 95 | 96.5 |
| **Motor octane number MON** | EN ISO 5163 | | Min 85 | 92.3 |
| **Distillation:** | | | | |
| **Initial Boiling Point IBP** | EN ISO 3405 | °C | Min 30 | 30.6 |
| **% vol recovered at 70C** | EN ISO 3405 | % vol | 20-52 | 22.6 |
| **% vol recovered at 100C** | EN ISO 3405 | % vol | 46-72 | 46.8 |
| **% vol recovered at 150C** | EN ISO 3405 | % vol | Min 75 | 85.7 |
| **Final Boiling Point FBP** | EN ISO 3405 | °C | Max 210 | 180.8 |
| **Residue** | EN ISO 3405 | % vol | Max 2.0 | 1.2 |
| **Saturated vapour pressure** | EN 13016 | kPa | Min 45 Max 100 | 56.7 |
| **Density at 15°C** | EN ISO 3675 | kg/m³ | 720 - 775 | 731.6 |
| **Olefins** | EN ISO 22854 | % vol | Max 18.0 | < 0.3 |
| **Benzene** | EN ISO 22854 | % vol | Max 1.0 | < 0.1 |
| **Aromatic hydrocarbon** | EN ISO 22854 | % vol | Max 35 | < 0.5 |
| **Oxygen** | EN ISO 22854 | % mass | Max 3.7 | < 0.1 |
| **Copper strip corrosion** | EN ISO 2160 | rating | class 1 | 1A |
| **Sulphur** | EN ISO 20846 | mg/kg | Max 10.0 | < 0.1 |
| **Gum, Existent Unwashed** | EN ISO 6246 | mg/100ml | Max 5 | 1.5 |
| **Gum, Existent Solvent Washed** | EN ISO 6246 | mg/100ml | Max 5 | < 1.0 |

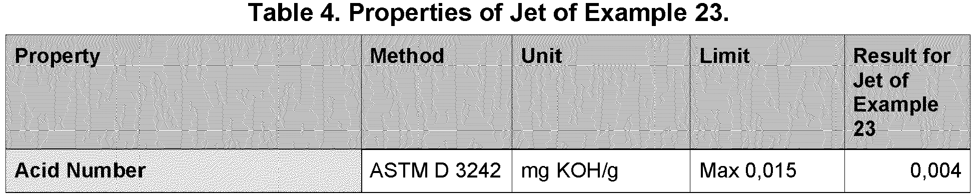

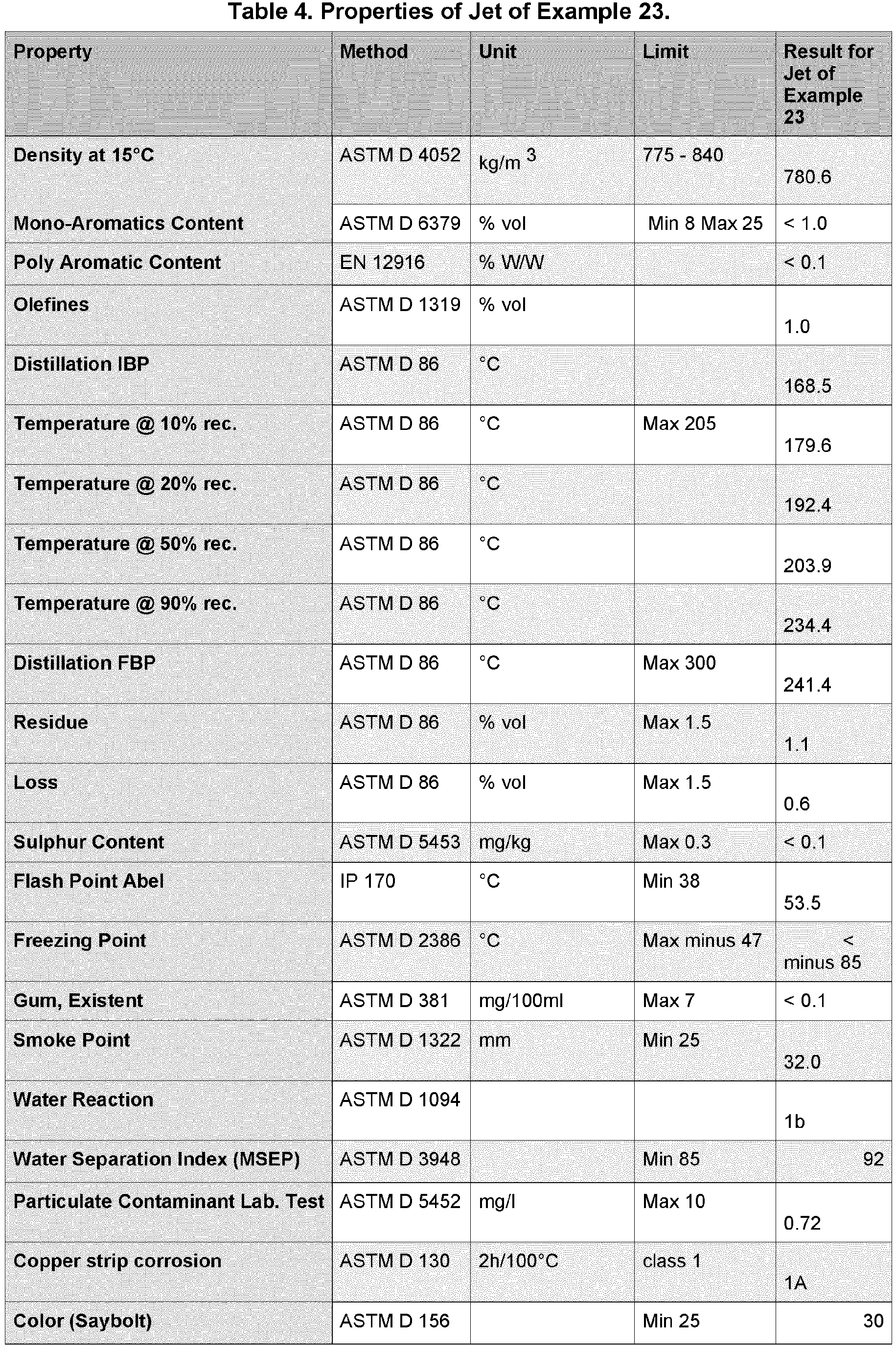

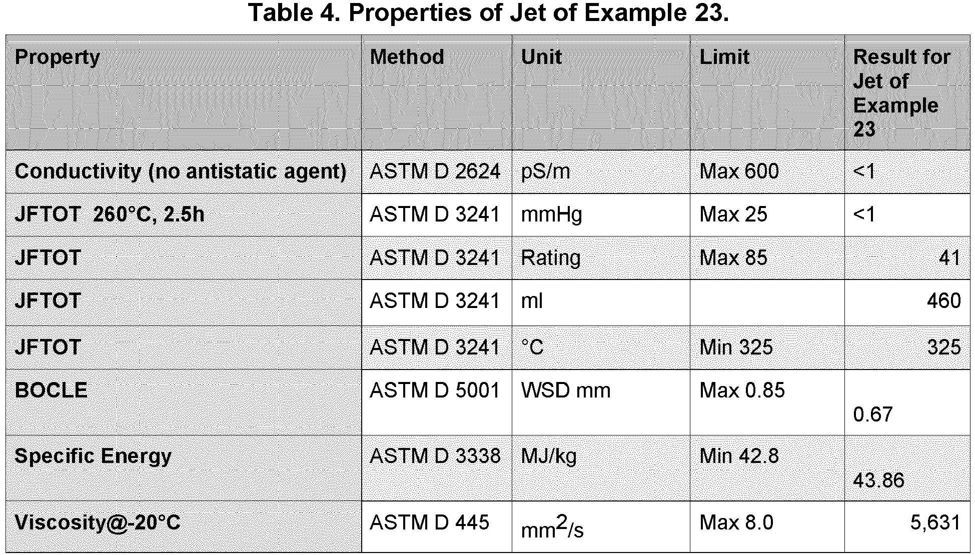

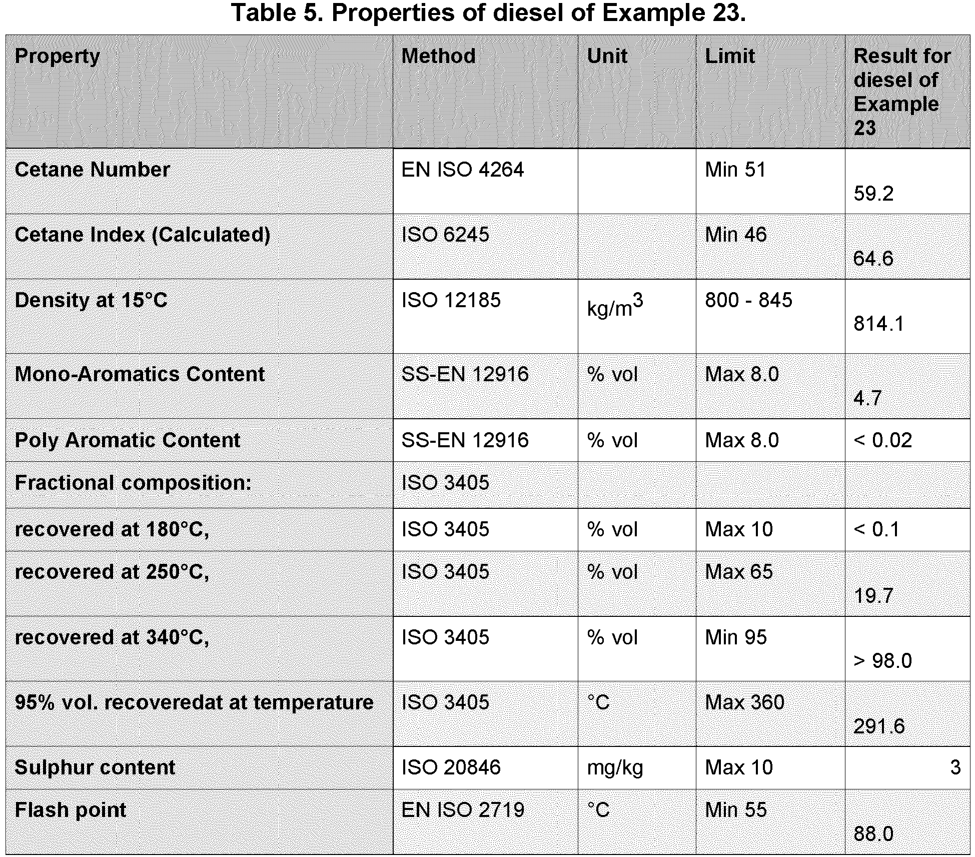

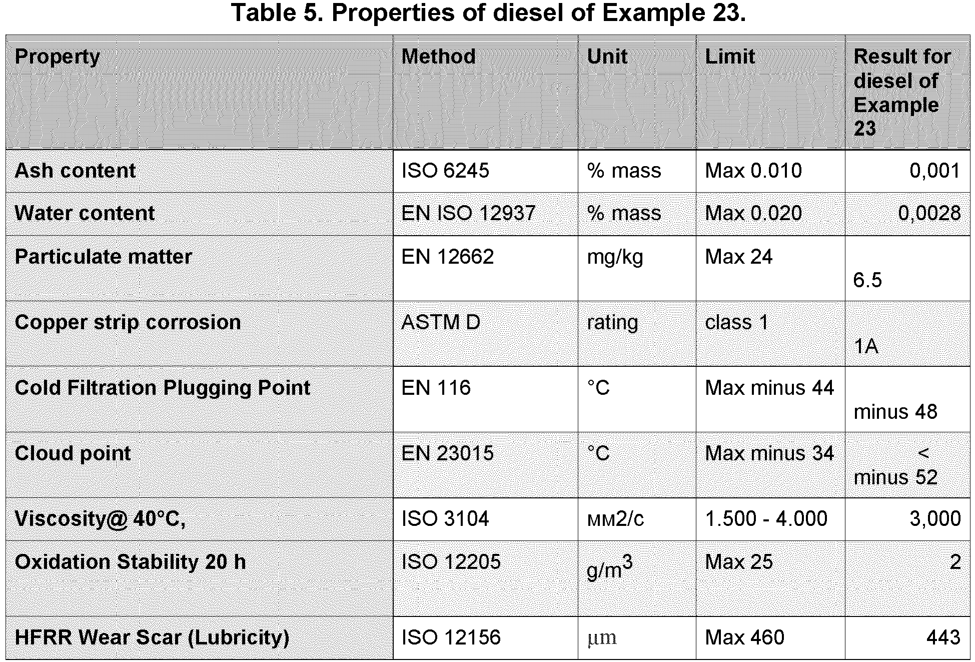

### Example 24. Obtaining tertamyl ethyl ether from tert-amyl and ethyl alcohols.

Tert-amyl alcohol (2-methyl-2-butanol) produced on the basis of the inventive technology is dehydrated in contact with the catalyst gamma Al₂O₃ at a temperature of 100-150°C and a pressure of 1.0 to 2.0 MPa. The obtained pentenes (C₅H₁₀) are separated from water, mixed with ethyl alcohol (C₂H₅OH) in a molar ratio of 1:(1.1-1.5) and supplied at a temperature of 50 to 100°C and a pressure of 0.5-1.0 MPa to a continuous flow reactor, equipped with the ion-exchange resin in the form of a cation exchanger Amberlite 15, as catalyst. The load on the catalyst is 0.5 to 1.0 liters of a solution of pentenes in ethyl alcohol per 1.0 liter of catalyst, the contact time of a solution of pentenes in ethyl alcohol with the ion-exchange resin in the form of a cation exchanger Amberlite 15, as catalyst is 30 to 60 minutes. The reaction mass obtained at the stage of etherification is directed to rectification, where ethyl alcohol is separated from ethyl tert-amyl ether. The rate of conversion of pentenes into ethyl tert-amyl ether exceeds 99%. The unreacted ethyl alcohol is returned back to the process to the stage of mixing with pentenes. Tert-amyl ethyl ether is used to obtain a standard gasoline free of aromatics.

### Example 25. Obtaining a standard gasoline containing ethers.

To obtain a standard gasoline free of aromatic compounds, the gasoline fraction of paraffins, produced on the basis of the inventive technology was used, and tertamyl ethyl ether, produced by the method of the previous example. The gasoline fraction of paraffins, containing C₃-C₁₀ hydrocarbons, is mixed with tertamyl ethyl ether in any volume ratio that does not violate the limits for oxygen content in gasoline prescribed by the relevant standards. Concentrations of 15%-22% by volume of tertamyl ethyl ether in the final mixture with C₃-C₁₀ paraffins are preferred for gasolines produced by the inventive technology. Table 6 shows the main properties of gasoline that is free of aromatic compounds. Gasoline prepared from the gasoline fraction of paraffins and tertamyl ethyl ether, produced on the basis of the inventive technology, is characterized by high octane numbers. A content of 20.0% by volume of tertamyl ethyl ether and 80.0% by volume of the gasoline fraction, as shown in the Example 23, provides the octane number RON = 100.9 MON = 93.8. The oxygen content of such gasoline is less than 3.0% wt.

**Table 6. Properties of gasoline of Example 25.**

| **Property** | **Metod** | **Unit** | **Limit gasoline Example 25** | **Result for gasoline of Example 25** |
|---|---|---|---|---|
| **Research octane number RON** | EN ISO 5164 | | Min 95 | 100.9 |
| **Motor octane number MON** | EN ISO 5163 | | Min 85 | 93.8 |
| **Distillation:** | | | | |
| **Initial Boiling Point IBP** | EN ISO 3405 | °C | Min 30 | 32.4 |
| **% vol recovered at 70C** | EN ISO 3405 | % vol | 10 - 50 | 25.4 |
| **% vol recovered at 100C** | EN ISO 3405 | % vol | 35 - 71 | 37.8 |
| **% vol recovered at 150C** | EN ISO 3405 | % vol | Min 60 | 83.3 |
| **Final Boiling Point FBP** | EN ISO 3405 | °C | Max 210 | 177.5 |
| **Residue** | EN ISO 3405 | % vol | Max 2.0 | 1.1 |
| **Saturated vapour pressure** | EN 13016 | kPa | Min 45 Max 100 | 59.0 |
| **Density at 15°C** | EN ISO 3675 | kg/m³ | 720 - 775 | 737.3 |
| **Olefins** | EN ISO 22854 | % vol | Max 18.0 | < 0.1 |
| **Benzene** | EN ISO 22854 | % vol | Max 1.0 | < 0.1 |
| **Aromatic hydrocarbon** | EN ISO 22854 | % vol | Max 35 | < 0.5 |
| **Oxygen** | EN ISO 22854 | % mass | Max 3.7 | 2.99 |
| **Copper strip corrosion** | EN ISO 2160 | rating | class 1 | 1A |
| **Sulphur** | EN ISO 20846 | mg/kg | Max 10.0 | < 0.1 |
| **Gum, Existent Unwashed** | EN ISO 6246 | mg/100ml | Max 5 | 0.6 |
| **Gum, Existent Solvent Washed** | EN ISO 6246 | mg/100ml | Max 5 | < 0.5 |

### Example 26. Obtaining ethers from tertiary C₅-C₆ alcohols and ethanol.

A mixture of tertiary C₅-C₈ alcohols, obtained by telomerization, were sent to rectification for isolation of tertiary C₅-C₆ alcohols. The C₅-C₆ tertiary alcohols were then dehydrated to yield the corresponding C₅-C₆ olefins. The obtained C₅-C₆ olefins were cooled and supplied at a temperature of 50 to 100°C and a pressure of 0.5-1.0 MPa into a continuous flow reactor, equipped with the ion-exchange resin in the form of a cation exchanger Amberlite 15, as catalyst. Along with C₅-C₆ olefins, ethyl alcohol was supplied to the reactor in a molar ratio (C₅H₁₀ + C₆H₁₂):C₂H₅OH = 1:(1.0-1.1). The contact time of the C₅-C₆ olefin solution in ethyl alcohol with the ion-exchange resin in the form of a cation exchanger Amberlite 15, as catalyst is 30 to 60 minutes. The reaction mass obtained at the stage of etherification is sent to rectification for separation of the unreacted ethyl alcohol from tertamyl ethyl and terthexyl ethyl ethers. The rate of conversion of C₅-C₆ olefins into ethyl tertamyl and ethyl terthexyl ethers exceeds 99%. The unreacted in the etherification reaction ethyl alcohol is returned back to the process to the stage of mixing with C₅-C₆ olefins. Ethyl tertamyl and ethyl terthexyl ethers are used for producing gasoline with an octane number of at least 100.

### Example 27. Obtaining gasoline with octane number of at least 100 and oxygen content of not more than 2.7% mass.

To produce gasoline with an octane number of at least 100 and an oxygen content of not higher than 2.7% mass, it is necessary to use C₃-C₁₀ gasoline fraction of paraffins, obtained by the inventive technology, as well as tertamyl ethyl and terthexyl ethyl ethers, obtained as described in the previous Example. Furthermore, to produce gasoline with an octane number of at least 100 and an oxygen content of not more than 2.7 wt%, it is necessary to use C₇-C₉ aromatic compounds obtained by the inventive technology. The gasoline fraction of paraffins, containing C₃-C₁₀ hydrocarbons, is mixed with tertamyl ethyl and terthexyl ethyl ethers in any volume ratio, which does not violate limits on oxygen content in gasoline stipulated by the relevant standard EN228. Preferred volumetric concentrations for gasolines with an octane number of at least 100 are 15%-25% of tertamyl ethyl and terthexyl ethyl ethers in the final composition with C₃-C₁₀ paraffins and C₇-C₉ aromatics.

Table 7 shows main properties of gasoline with an octane number of at least 100 and an oxygen content of not more than 2.7% mass.

**Table 7. Properties of gasoline of Example 27.**

| **Property** | **Method** | **Unit** | **Limit** | **Result for Example 25 gasoline of Example 27** |
|---|---|---|---|---|
| **Research octane number RON** | EN ISO 5164 | | Min 95 | 100.1 |
| **Motor octane number MON** | EN ISO 5163 | | Min 85 | 93.6 |
| **Distillation:** | | | | |
| **Initial Boiling Point IBP** | EN ISO 3405 | °C | Min 30 | 36.4 |
| **% vol recovered at 70C** | EN ISO 3405 | % vol | 10 - 50 | 17.9 |
| **% vol recovered at 100C** | EN ISO 3405 | % vol | 35 - 71 | 42.7 |
| **% vol recovered at 150C** | EN ISO 3405 | % vol | Min 60 | 84.1 |
| **Final Boiling Point FBP** | EN ISO 3405 | °C | Max 210 | 178.4 |
| **Residue** | EN ISO 3405 | % vol | Max 2.0 | 1.2 |
| **Saturated vapour pressure** | EN 13016 | kPa | 45 - 100 | 58.0 |
| **Density at 15°C** | EN ISO 3675 | kg/m³ | 720 - 775 | 757.6 |
| **Olefins** | EN ISO 22854 | % vol | Max 18.0 | < 0.1 |
| **Benzene** | EN ISO 22854 | % vol | Max 1.0 | < 0.1 |
| **Aromatic hydrocarbon** | EN ISO 22854 | % vol | Max 35 | 7.5 |
| **Oxygen** | EN ISO 22854 | % mass | Max 2.7 | 2.65 |
| **Copper strip corrosion** | EN ISO 2160 | rating | class 1 | 1A |
| **Sulphur** | EN ISO 20846 | mg/kg | | < 0.1 |
| **Gum, Existent Unwashed** | EN ISO 6246 | mg/100ml | Max 5 | 0.6 |
| **Gum, Existent Solvent Washed** | EN ISO 6246 | mg/100ml | Max 5 | < 0.5 |

### Example 28 Obtaining of ethers from tertiary C₇-C₈ alcohols and n-butyl alcohols

A mixture of tertiary C₅-C₈ alcohols, obtained by telomerization, was sent for rectification to isolate tertiary C₇-C₈ alcohols. The tertiary alcohols were then dehydrated to yield the corresponding C₇-C₈ olefins. The C₇-C₈ olefins thus obtained were cooled and supplied at a temperature of 50 to 100°C and a pressure of 0.5-1.0 MPa into a continuous flow reactor, where the ion-exchange resin in the form of a cation exchanger Amberlite 15, as catalyst was loaded. Along with the C₇-C₈ olefins, n-butyl alcohol, obtained by the inventive technology, was supplied into the reactor in a molar ratio (C₇H₁₄ + C₈H₁₆):C₄H₉OH = 1:(1.0-1.1). The contact time of the C₇-C₈ olefin solution in n-butyl alcohol with the ion-exchange resin in the form of a cation exchanger Amberlite 15, as catalyst is 30 to 60 minutes. The reaction mass obtained in the etherification process was directed to rectification, where n-butyl alcohol was separated from tert-heptyl butyl and tert-octyl butyl ethers. The rate of C₇-C₈ olefin conversion into tert-heptyl butyl and tert-octyl butyl ethers exceeds 99%. The unreacted in the etherification reaction butyl alcohol, is separated by rectification in the rectification column and returned back to the process to the stage of mixing with C₇-C₈ olefins. Tert-heptyl butyl and tert-octyl butyl ethers are used to produce diesel, containing oxygenated hydrocarbons.

### Example 29. Producing a standard diesel fuel containing oxygenated hydrocarbons.

To produce a standard diesel fuel comprising oxygen-containing hydrocarbons, it is necessary to use diesel fraction of C₁₉-C₂₄ paraffins, produced by the inventive technology. Furthermore, tert-heptyl butyl and tert-octyl butyl ethers shall be used as oxygen-containing hydrocarbons of this diesel fuel. The method for producing tert-heptyl butyl and tert-octyl butyl ethers is described in the previous example. The diesel fraction of paraffins, containing C₁₉-C₂₄ hydrocarbons, was mixed with tert-heptyl butyl and tert-octyl butyl ethers in any ratio that does not violate requirements of the relevant standards for diesel fuels. Preferred concentration for diesel fuels, produced by the invenitive technology, is from 5% to 15% by volume of the total of tert-heptyl butyl and tert-octyl butyl ethers in the final mixture with C₁₉-C₂₄ paraffins. Table 8 shows main properties of the diesel fuel containing a mixture of tert-heptyl butyl and tert-octyl butyl ethers in the amount of 10% by vol. of the final mixture with C₁₉-C₂₄ paraffins.

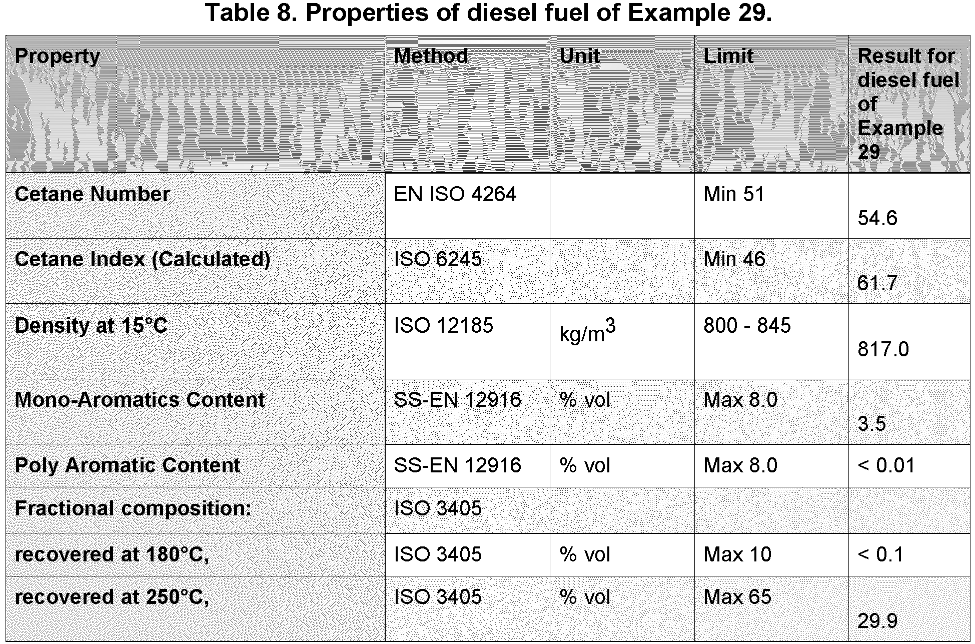

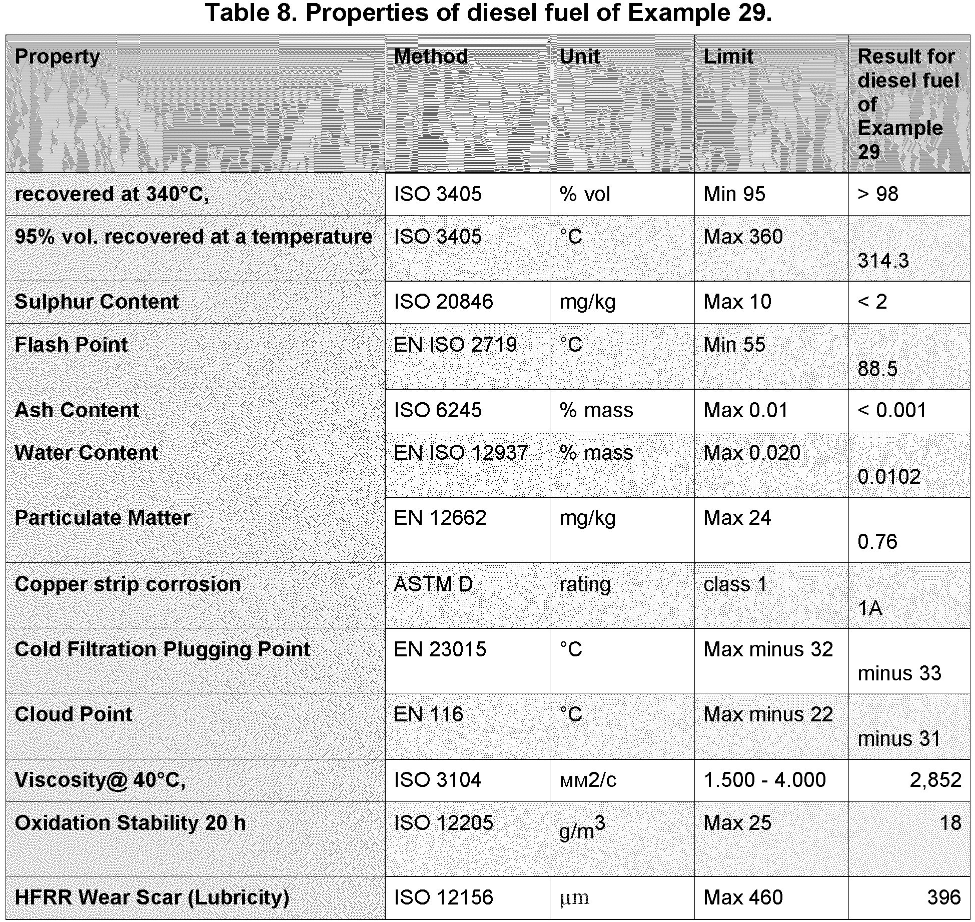

### Example 30. Producing ethers from tertiary C₇-C₈ alcohols and 2-ethyl hexyl alcohol.

A mixture of tertiary C₅-C₈ alcohols, obtained by telomerization, was sent to rectification to isolate the tertiary C₇-C₈ alcohols. The isolated alcohols were then dehydrated to obtain the corresponding C₇-C₈ olefins. The C₇-C₈ olefins obtained were cooled and supplied at a temperature of 50 to 100°C and a pressure of 0.5 to 1.0 MPa to a continuous flow reactor, where the catalyst, which is an ion exchange resin, in this case the cation exchanger Amberlite 15, was loaded. Along with the C₇-C₈ olefins, 2-ethyl hexyl alcohol, obtained by the inventive technology, was supplied to the reactor in a molar ratio (C₇H₁₄ + C₈H₁₆):C₈H₁₇OH = 1:(1.1-1.5). The contact time of the solution of C₇-C₈ olefins in 2-ethyl hexyl alcohol with the catalyst, which is an ion exchange resin, in this case the cation exchanger, is 45 to 60 minutes. The reaction mass obtained at the stage of etherification was directed to rectification, where 2-ethyl hexyl alcohol was separated from the obtained tert-heptyl 2-ethyl hexyl and tert-octyl 2-ethyl hexyl ethers. The rate of C₇-C₈ olefin conversion into tert-heptyl 2-ethyl hexyl and tert-octyl 2-ethyl hexyl ethers exceeds 90%. A mixture of 2-ethyl hexyl alcohol and unreacted C₇-C₈ olefins was isolated in the rectification column and returned back to the process to the etherification stage. Tert-heptyl 2-ethyl hexyl and tert-octyl 2-ethyl hexyl ethers are used to produce diesel fuel, comprising in the composition oxygen-containing hydrocarbons in an amount of not less than 20% vol.

### Example 31. Producing diesel fuel, comprising in the composition oxygen-containing hydrocarbons in the amount of not less than 20% vol.

To produce diesel fuel comprising in the composition oxygen-containing hydrocarbons in the amount of not less than 20% vol., it is necessary to use the C₁₉-C₂₄ paraffin fraction obtained by the inventive technology. Furthermore, to produce said diesel fuel, it is necessary to use tert-heptyl butyl and tert-octyl butyl ethers, as well as tert-heptyl 2-ethyl hexyl and tert-octyl 2-ethyl hexyl ethers. Methods for producing said ethers are described in the Examples 28 and 30.

The paraffin fraction, containing C₁₉-C₂₄ hydrocarbons, was mixed with tert-heptyl butyl and tert-octyl butyl ethers, as well as tert-heptyl 2-ethyl hexyl and tert-octyl 2-ethyl hexyl ethers. The mixing was performed in a manner providing for compliance of the properties of the resulting hydrocarbon mixtures with the requirements of the current standards for diesel motor fuel. Preferred concentration for diesel fuels produced by the inventive technology and comprising said ethers is from 10% to 20% by volume of the total of all ethers in the final mixture with C₁₉-C₂₄ paraffins. Table 9 shows the main properties of diesel fuel, containing not less than 20% vol. of oxygen-containing hydrocarbons.

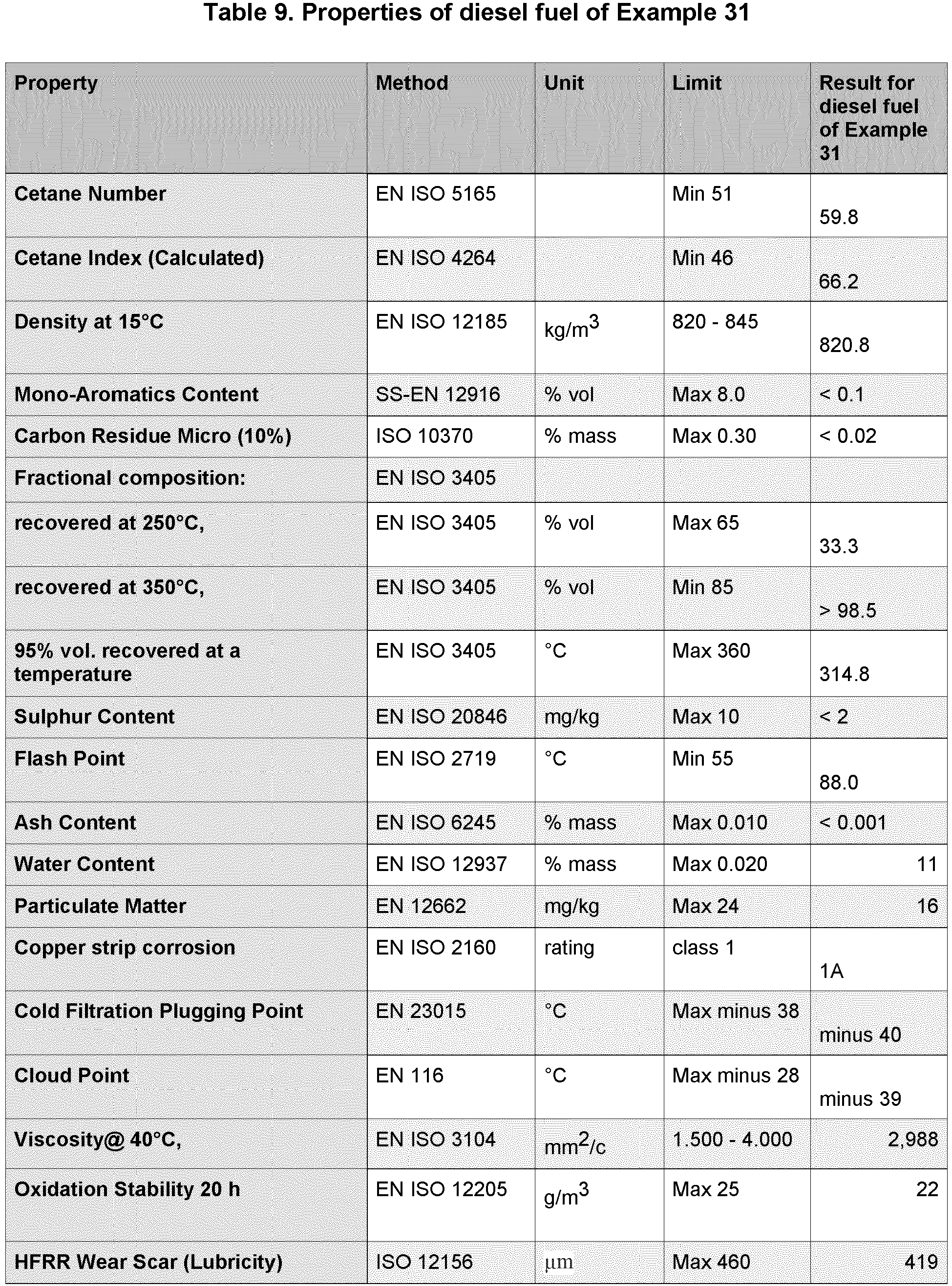

### Example 32. Obtaining a 100% biological kerosene from ethanol, in full compliance of all requirements of the current standard Jet A1.

A mixture of tert-amyl, tert-hexyl, tert-heptyl and tert-octyl alcohols in a molar ratio of 1:1:1:1 is dehydrated in contact with the gamma Al₂O₃ catalyst at a temperature of 100 to 150°C and a pressure of 1.0 to 2.0 MPa. The resulting mixture of olefins (C₅H₁₀ + C₆H₁₂ + C₇H₁₄ + C₈H₁₆) is separated from water and supplied at a temperature of 100 to 150°C and a pressure of 1.0 to 2.0 MPa to a continuous flow reactor, where the catalyst, which is an ion exchange resin, in this case the cation exchanger Amberlite 15, is loaded. Under these conditions, pentenes, hexenes, heptenes and octenes are oligomerized yielding C₁₀-C₂₄ olefins. The unreacted pentenes, hexenes, heptenes and octenes are distilled from the reaction mass obtained by oligomerization and returned back to the process. The reaction mass, free of the source pentenes, hexenes, heptenes and octenes, is directed for hydrogenation to a continuous flow reactor, where the catalyst consisting of the oxides NiO, CuO and Cr₂O₃ in a molar ratio of 1:1:1 is loaded. Along with the oligomerization products, hydrogen is supplied to the hydrogenation reactor at a temperature of 120 to 150°C and a pressure of 4.0 to 5.0 MPa. In the course of hydrogenation, the unsaturated C₁₀-C₂₄ compounds obtained at the oligomerization stage are converted into C₁₀-C₂₄ paraffins. The reaction mass obtained at the stage of hydrogenation is directed to the rectification, where it is separated into kerosene and diesel fractions, free of aromatic compounds.

The unsaturated C₂-C₅ hydrocarbons obtained at the stage of dehydration of the corresponding C₂-C₅ alcohols are supplied to the aromatization reactor. Aromatization of the unsaturated hydrocarbons is carried out at a temperature of 350 to 450°C and a pressure of 0.5 to 2.0 MPa in a continuous operation reactor in the presence of the inventive heterogeneous zeolite-containig catalyst, comprising at least 93% of ZSM-5 modified by 3.5 to 7.0% Zn. In the course of aromatization, the unsaturated C₂-C₅ hydrocarbons are converted into aromatic compounds C₇-C₁₂. The reaction mass obtained at the stage of aromatization is isolated from the gaseous products, which are a mixture of hydrogen and C₁-C₄ paraffins, and directed to rectification, where it is separated into two fractions: C₇-C₈ aromatic hydrocarbons and C₉-C₁₂ aromatic hydrocarbons. The C₇-C₈ aromatic hydrocarbons are used to produce gasoline, while the C₉-C₁₂ aromatic hydrocarbons are used to produce kerosene. The C₁₁-C₁₈ kerosene paraffins fraction isolated from C₁₀-C₂₄ paraffins is mixed with the aromatic compounds in such a way that the concentration of the aromatic hydrocarbons in the final composition of kerosene is in the range from 8% vol. to 25% vol. Table 10 shows the main properties of the 100% biological kerosene produced from ethanol, which is in full compliance with all requirements of the current standard Jet A1.

**Table 10. Properties of Jet of Example 32**

| **Property** | **Metod** | **Unit** | **Limit** | **Result for Jet of Example 32** |
|---|---|---|---|---|
| **Acid Number** | ASTM D 3242 | mg KOH/g | Max 0.015 | 0,007 |
| **Density at 15°C** | ASTM D 4052 | kg/m³ | 775 - 840 | 789.5 |
| **Mono-Aromatics Content** | ASTM D 1319 | % volume | Min 8 Max 25 | 9.8 |
| **Poly Aromatics Content** | EN12916 | %w/W | | < 0.1 |
| **Olefines** | ASTM D 1319 | % vol | | 1.8 |
| **Distillation IBP** | ASTM D 86 | °C | | 169,9 |
| **Temperature @ 10% rec.** | ASTM D 86 | °C | Max 205 | 183,1 |
| **Temperature @ 20% rec.** | ASTM D 86 | °C | | 187,2 |
| **Temperature @ 50% rec.** | ASTM D 86 | °C | | 201,4 |
| **Temperature @ 90% rec.** | ASTM D 86 | °C | | 223,0 |
| **Distillation FBP** | ASTM D 86 | °C | Max 300 | 235,3 |
| **Residue** | ASTM D 86 | % vol | Max 1.5 | 1,5 |
| **Loss** | ASTM D 86 | % vol | Max 1.5 | 0,2 |
| **Sulphur Content** | ASTM D 5453 | mg/kg | Max 0.3 | < 0.1 |
| **Flash Point Abel** | ASTM D 56 | °C | Min 38 | 51.0 |
| **Freezing Point** | ASTM D 2386 | °C | Max minus 47 | < minus 85 |
| **Gum, Existent** | ASTM D 381 | mg/100ml | Max 7 | < 1 |
| **Smoke Point** | ASTM D 1322 | mm | Min 25 | 35 |
| **Water Reaction** | ASTM D 1094 | | | 1b |
| **Water Separation Index (MSEP)** | ASTM D 3948 | | Min 85 | 97 |
| **Particulate Contaminant Lab. Test** | ASTM D 5452 | mg/l | Max 10 | 0.36 |
| **Copper strip corrosion** | ASTM D 130 | 2h/100°C | class 1 | 1A |

**Table 10. Properties of Jet of Example 32**

| **Property** | **Metod** | **Unit** | **Limit** | **Result for Jet of Example 32** |
|---|---|---|---|---|
| **Color (Saybolt)** | ASTM D 156 | | Min 25 | 30 |
| **Conductivity (no antistatic agent)** | ASTM D 2624 | pS/m | Max 600 | <1 |
| **JFTOT 2.5h 260°C** | ASTM D 3241 | mm Hg | Max 25 | <1 |
| **JFTOT Rating** | ASTM D 3241 | nm | Max 85 | 9 |
| **JFTOT** | ASTM D 3241 | ml | | 447 |
| **JFTOT** | ASTM D 3241 | °C | Min 325 | 325 |
| **BOCLE** | ASTM D 5001 | WSD mm | Max 0.85 | 0.69 |
| **Specific Energy** | ASTM D 3338 | MJ/kg | Min 42.8 | 43.75 |
| **Viscosity@-20°C** | ASTM D 445 | mm²/s | Max 8.0 | 4,893 |

## Claims

1. A method for producing from ethanol a motor fuel selected from gasoline, kerosene, and diesel, comprising the following interconnected steps:
step 1.1 converting a mixture of ethanol and 25-35 % by volume of water of said mixture into:
- isopropanol and 3-pentanol;
- acetaldehyde;
- a mixture of C₁-C₄ paraffins and C₂-C₄ olefins;
- a mixture of carbon dioxide and hydrogen;
wherein the mixture of ethanol and water at a pressure of 0.5-1.5 MPa and a temperature of 500-515°C is contacted with a heterogeneous catalyst consisting of the following metal oxides: ZnO 60-63% mass, CeO₂ 1-6% mass, MgO 12-18% mass; and Al₂O₃ 13-23% mass, wherein the mixture of ethanol and water is supplied to the catalyst at a space velocity of 0.5-0.9 hr⁻¹, thereby producing, acetone, and diethyl ketone,
wherein the acetone and the diethyl ketone obtained are isolated from the reaction mixture and hydrogenated at a temperature of 100-150°C and a pressure of 0.5-0.9 MPa in the presence of a catalyst comprising CuO and Cr₂O₃ in a molar ratio of 1:1, by hydrogen obtained from the mixture of ethanol and water, thereby resulting in the isopropanol and 3-pentanol;
step 1.2 converting the mixture of carbon dioxide and hydrogen, obtained in step 1.1, additional hydrogen, and a mixture of C₁-C₄ paraffins into synthesis gas;
wherein the mixture of carbon dioxide and hydrogen is converted into water and carbon monoxide in the presence of a catalyst containing 5-10% Cu supported by Al₂O₃, at a temperature of 550-600°C and a pressure of 1.0-5.0 MPa, or, into a mixture of carbon monoxide and hydrogen using a catalyst containing NiO, CuO and Cr₂O₃ in a molar ratio of 1:1:1, at a temperature of 900-1000°C and a pressure of 0.1-0.5 MPa;
step 1.3 converting by dehydration in the presence of gamma Al₂O₃ in a continuous flow reactor at a temperature of 350-450°C and a pressure of 0.5-3.5 MPa ethanol, and C₃-C₈ alcohols, including C₅ alcohol obtained from step 1.1, into: - C₂-C₆ olefins, including ethylene and propylene;
step 1.4 converting at a pressure of P = 1.0 -5.0 MPa and a temperature of 100-130°C a mixture of unreacted ethanol from step 1.1, isopropanol obtained in step 1.1, ethylene obtained in step 1.3, using a telomerization reaction into secondary butanol and tertiary C₅-C₈ alcohols, the tertiary C₅ and C₇ alcohols being obtained from the isopropanol, and the tertiary C₆, and C₈ alcohols being obtained from the ethanol, wherein the resulting secondary butanol is directed to step 1.3, and wherein the isopropanol reacts in the presence of ditertamyl peroxide with the ethylene and is thereby converted into the tertiary C₅ and C₇ alcohols;
step 1.5 converting the C₅-C₈ tertiary alcohols, obtained in step 1.4, by dehydration in the presence of a heterogeneous catalyst gamma Al₂O₃ at a temperature of 100-150°C and a pressure of 0.5-1.5 MPa until reaching the yield of C₅-C₈ olefins of at least 99% of the source C₅-C₈ alcohols into C₅-C₈ olefins;
step 1.6 converting a first portion of the C₅-C₈ olefins, obtained in step 1.5, by oligomerization into C₁₀-C₂₄ olefins, wherein the C₅-C₈ olefins are oligomerized using an ion-exchange resin in the form of a cation exchanger as a catalyst, at a temperature of 70-120°C and a pressure of 1.0-2.0 MPa;
step 1.7 converting the C₁₀-C₂₄ olefins, obtained in step 1.6, by hydrogenation using hydrogen obtained from step 1.1, into C₁₀-C₂₄ paraffins, wherein the C₁₀-C₂₄ olefins are hydrogenated in the presence of a heterogeneous catalyst containing NiO, CuO and Cr₂O₃ in a molar ratio of 1:1:1 at a temperature of 150-200°C and pressure of 4.5-5.0 MPa;
step 1.8 converting the synthesis gas, obtained in step 1.2, ethylene obtained in step 1.3, or propylene obtained in step 1.3, and the acetaldehyde, obtained in step 1.1, by hydroformylation and aldol condensation into a mixture of C₃-C₄ aldehydes and C₅-C₈ aldols, said mixture of C₃-C₄ aldehydes and C₅-C₈ aldols is thereafter hydrogenated to obtain C₃-C₈ alcohols, which alcohols are directed to step 1.3 to obtain C₃-C₈ olefins, wherein the acetaldehyde produces C₅ alcohol, the ethylene from step 1.3 produces C₃ and C₆ alcohols, and the propylene from step 1.3 produces C₄ and C₈ alcohols; and
wherein the ethylene and the propylene from step 1.3 produce C₇ alcohol;
wherein the ethylene or propylene is converted in the hydroformylation reaction at a temperature of 70-90°C and a pressure of 1.0-5.0 MPa in the presence of a water-soluble Rh catalyst, with a concentration of metal in relation to the aqueous phase of from 30 ppm to 50 ppm, into propanal or n-butanal and isobutanal,
thereafter a mixture of propanal, n-butanal, and acetaldehyde obtained in step 1.1, is converted by cross-aldol condensation at a temperature of 100-150°C and a pressure of 0.5-1.0 MPa in the presence of a heterogeneous catalyst containing at least 93% of ZSM-5 zeolite modified by 3.5-7.0% Zn, or a granular catalyst containing at least 95% of ZSM-5 zeolite modified by 3.5-5.0% Zn and 0.1-1.5% Ce into a mixture of C₃-C₄ aldehydes and C₅-C₈ aldols, thereafter the mixture of C₃-C₄ aldehydes and C₅-C₈ aldols is hydrogenated at a temperature of 150-200°C and a pressure of 4.5-5.0 MPa in the presence of a heterogeneous catalyst containing NiO, CuO and Cr₂O₃ in a molar ratio of 1:1:1 to obtain the mixture of C₃-C₈ alcohols,
wherein the ethylene or propylene is converted in the hydroformylation reaction at a temperature of 120-140°C and a pressure of 2.0-5.0 MPa in the presence of a water-soluble Co catalyst, with a concentration of Co metal in relation to the aqueous phase of from 0.1% to 1.0%, into propanal, 2-methylpentenal and 2-methylpentanal or into n-butanal, 2-ethylhexenal, 2-ethylhexanal and isobutanal, and
thereafter the mixture of C₃-C₄ aldehydes and C₆-C₈ aldols at a temperature of 150-200°C and a pressure of 4.5-5.0 MPa is in the presence of a heterogeneous catalyst containing NiO, CuO and Cr₂O₃ in a molar ratio of 1:1:1 hydrogenated to obtain the mixture of C₃-C₈ alcohols;
step 1.9 converting the C₂-C₆ olefins from step 1.3, by oligomerization into C₆-C₂₄ olefins in the presence of a heterogeneous catalyst containing at least 93% of ZSM-5 zeolite modified by 3.5-7.0% Zn, or a heterogeneous catalyst containing at least 95% of ZSM-5 zeolite modified by 3.5-5.0% Zn and 0.1-1.5% Ce at a temperature of 250-350°C and a pressure of 4.5-5.0 MPa to obtain the C₆-C₂₄ olefins, wherein unreacted C₂-C₅ olefins from the oligomerization reaction are separated by rectification from the C₆-C₂₄ olefins and directed to step 1.11;
step 1.10 converting the C₆-C₂₄ olefins, obtained in step 1.9, and hydrogen, by hydrogenation into C₆-C₂₄ paraffins;
wherein the hydrogenation of C₆-C₂₄ olefins is carried out in the presence of a heterogeneous catalyst containing NiO, CuO and Cr₂O₃ in a molar ratio of 1:1:1, at a temperature of 150-200°C and a pressure of 4.5-5.0 MPa to obtain the C₆-C₂₄ paraffins;
step 1.11 converting unreacted C₂-C₅ olefins from step 1.9, and the mixture of C₂-C₄ olefins and C₁-C₄ paraffins, obtained in step 1.1, by aromatization into C₇-C₁₂ aromatic hydrocarbons, hydrogen, and a mixture of C₁-C₄ paraffins, wherein the unreacted C₂-C₅ olefins are aromatized together in a mixture with C₂-C₄ olefins and C₁-C₄ paraffins, in the presence of a heterogeneous catalyst containing at least 93% of ZSM-5 zeolite modified by 3.5-7.0% Zn at a temperature of 350-450°C and a pressure of 0.5-5.0 MPa, wherein a first portion of the hydrogen produced is directed to step 1.10, and the remaining second portion of the hydrogen produced and the C₁-C₄ paraffins mixture are directed to step 1.2;
step 1.12 converting the remaining second portion of the mixture of C₅-C₈ olefins, obtained in step 1.5, and a portion of C₂-C₆ alcohols from step 1.3, into C₇-C₁₆ ethers by etherification carried out on a mixture of the C₅-C₈ olefins and the C₂-C₆ alcohols using as a catalyst an ion exchange resin in the form of a cation exchanger at a temperature of 70-120°C and pressure of 1.5-2.0 MPa to yield the C₇-C₁₆ ethers; and,
step 1.13 separating by rectification the C₁₀-C₂₄ paraffins, obtained in step 1.7, and the C₆-C₂₄ paraffins, obtained in step 1.10, into a C₆-C₁₀ paraffin fraction for producing gasoline, a C₁₁-C₁₈ paraffin fraction for producing kerosene, and a C₁₉-C₂₄ paraffin fraction for producing diesel;
separating by rectification the C₇-C₁₂ aromatic hydrocarbons, obtained in step 1.11, into a C₇-C₈ aromatic hydrocarbon fraction for producing gasoline and a C₉-C₁₂ aromatic hydrocarbon fraction for producing kerosene; and,
separating by rectification the C₇-C₁₆ ethers, obtained in step 1.12, into a C₇-C₁₀ ether fraction for producing gasoline and a C₁₁-C₁₆ ether fraction for producing diesel,
and mixing selected fractions thereof, into a motor fuel selected from gasoline, kerosene, and diesel;
wherein the C₆-C₂₄ paraffins are rectified to isolate the C₆-C₁₀ paraffin fraction for producing gasoline, the C₁₁-C₁₈ paraffin fraction for producing kerosene, and the C₁₉-C₂₄ paraffin fraction for producing diesel; and,
wherein the C₁₀-C₂₄ paraffins are rectified to isolate the C₁₁-C₁₈ paraffin fraction for producing kerosene, and the C₁₉-C₂₄ paraffin fraction for producing diesel, and the remaining C₁₀ paraffins are mixed with the C₆-C₁₀ paraffin fraction for producing gasoline, obtained in step 1.13 by rectification of the C₆-C₂₄ paraffins.

2. The method for producing motor fuel from ethanol in accordance with claim 1, wherein, in step 1.4, the unreacted ethanol from step 1.1 reacts in the presence of di-tert-butyl peroxide or di-tert-amyl peroxide under a pressure of P = 1.0-5.0 MPa and at a temperature of 100-130°C with the ethylene to obtain sec-butanol, which is then reacted with ethylene and is converted into the tertiary C₆ and C₈ alcohols.

3. The method for producing motor fuel from ethanol in accordance with claim 1, wherein Triphenylphosphine-sulfonic acid sodium salts, namely: from Triphenylphosphine-3-sulfonic acid sodium salt to Triphenylphosphine-3,3',3"-trisulfonic acid trisodium salt in a weight ratio (10-30):1 to metal Rh are used as a ligand in the step 1.8 in the hydroformylation of the ethylene or propylene for obtaining the water-soluble catalyst.

4. The method for producing motor fuel from ethanol in accordance with claim 3, wherein, in order to increase the reaction rate and the yield of isobutanal in step 1.8, the hydroformylation of propylene by synthesis gas is carried out in the presence of a water-soluble Rh catalyst prepared in accordance with claim 3; C₂-C₃ alcohols are added to said catalyst in a volume ratio H₂O:(C₂-C₃) = (0.95-0.65):(0.05-0.35), while the ratio of butanal and isobutanal obtained in the reaction medium is in the range (n-C₄H₈O):(iso-C₄H₈O) = (2-3):1.

5. The method for producing motor fuel from ethanol in accordance with claim 1, wherein, in step 1.8, Triphenylphosphine-sulfonic acid sodium salts, namely: from Triphenylphosphine-3-sulfonic acid sodium salt to Triphenylphosphine-3,3',3"-trisulfonic acid trisodium salt are used as a ligand in a weight ratio (1-30):1 to metal Co in the hydroformylation of ethylene or propylene for obtaining the water-soluble Co catalyst.

6. The method for producing motor fuel from ethanol in accordance with claim 5, wherein, in order to increase the reaction rate and the yield of isobutanal in step 1.8, in the hydroformylation of propylene, C₂-C₃ alcohols are added to the catalyst in a volume ratio H₂O:(C₂-C₃) = (0.95- 0.5):(0.05-0.5), wherein the ratio of butanal and isobutanal obtained in the reaction medium is in the range of (n-C₄H₈O):(iso-C₄H₈O) = (2-3):1.

7. The method for producing motor fuel from ethanol in accordance with any of the previous claims, wherein in step 1.6 the ion-exchange resin in the form of a cation exchanger is Amberlite 15.

8. The method for producing motor fuel from ethanol in accordance with claim 1, wherein, in step 1.13, in order to obtain kerosene fully complying with the requirements of the current Jet A-1 standard, C₁₁-C₁₈ paraffins are isolated from the C₆-C₂₄ paraffins mixture obtained from step 1.10, C₁₁-C₁₈ paraffins are isolated from the mixture of C₁₀-C₂₄ paraffins obtained in step 1.7, and aromatic C₉-C₁₂ hydrocarbons are isolated from the C₇-C₁₂ aromatic hydrocarbons mixture obtained in the step 1.11, and the isolated C₁₁-C₁₈ paraffins and the isolated aromatic C₉-C₁₂ hydrocarbons are mixed so that the concentration of aromatic C₉-C₁₂ hydrocarbons is in the range of 8-25% vol, and so that the resulting kerosene will contain at least 100 different hydrocarbons, and preferably 150 different hydrocarbons, have a Smoke Point of minimum 30 mm, and a Freezing Point of maximum minus 80°C.

## Patentansprüche

1. Ein Verfahren zur Herstellung eines Motorentreibstoffes aus Ethanol, ausgewählt aus Benzin, Kerosin und Diesel, umfassend die folgenden miteinander verbundenen Schritte:
Schritt 1.1 Umwandeln eines Gemisches aus Ethanol und 25 bis 35 Vol.-% Wasser des Gemischs in:
- Isopropanol und 3-Pentanol;
- Acetaldehyd;
- ein Gemisch aus C₁-C₄-Paraffinen und C₂-C₄-Olefinen;
- ein Gemisch aus Kohlendioxid und Wasserstoff;
wobei das Gemisch aus Ethanol und Wasser bei einem Druck von 0,5 bis 1,5 MPa und einer Temperatur von 500 bis 515°C mit einem heterogenen Katalysator, bestehend aus den folgenden Metalloxiden: ZnO 60 bis 63 Massen-%, CeO₂ 1 bis 6 Massen-%, MgO 12 bis 18 Massen-%; und Al₂O₃ 13 bis 23 Massen-%, in Kontakt gebracht wird, wobei das Gemisch aus Ethanol und Wasser dem Katalysator mit einer Raumgeschwindigkeit von 0,5 bis 0,9 h⁻¹ zugeführt wird, wodurch Aceton und Diethylketon hergestellt wird,
wobei das erhaltene Aceton und Diethylketon von dem Reaktionsgemisch isoliert werden und bei einer Temperatur von 100 bis 150°C und einem Druck von 0,5 bis 0,9 MPa in Gegenwart eines Katalysators, umfassend CuO und Cr₂O₃ in einem Molverhältnis von 1:1, durch Wasserstoff, welcher aus dem Gemisch von Ethanol und Wasser erhalten wurde, hydriert werden, wodurch das Isopropanol und 3-Pentanol erhalten werden;
Schritt 1.2 Umwandeln des in Schritt 1.1 erhaltenen Gemischs aus Kohlendioxid und Wasserstoff, zusätzlichem Wasserstoff und einem Gemisch aus C₁-C₄-Paraffinen in Synthesegas;
wobei das Gemisch aus Kohlendioxid und Wasserstoff in Wasser und Kohlenmonoxid in Gegenwart eines Katalysators, enthaltend 5 bis 10% Cu, welches auf Al₂O₃ geträgert ist, bei einer Temperatur von 550 bis 600°C und einem Druck von 1,0 bis 5,0 MPa oder ein Gemisch aus Kohlenmonoxid und Wasserstoff unter Verwendung eines Katalysators, welcher NiO, CuO und Cr₂O₃ in einem Molverhältnis von 1:1:1 enthält, bei einer Temperatur von 900 bis 1000°C und einem Druck von 0,1 bis 0,5 MPa umgewandelt wird;
Schritt 1.3 Umwandeln von Ethanol und C₃-C₈-Alkoholen, einschließlich C5-Alkohol, erhalten in Schritt 1.1 durch Dehydratisierung in Gegenwart von gamma-Al₂O₃ in einem Durchflussreaktor bei einer Temperatur von 350 bis 450°C und einem Druck von 0,5 bis 3,5 MPa, in:
- C₂-C₈-Olefine, einschließlich Ethylen und Propylen;
Schritt 1.4 Umwandeln eines Gemisches an nicht umgesetztem Ethanol aus Schritt 1.1, in Schritt 1.1 erhaltenem Isopropanol, in Schritt 1.3 erhaltenem Ethylen bei einem Druck von P = 1,0 bis 5,0 MPa und einer Temperatur von 100 bis 130°C unter Verwendung einer Telomerisationsreaktion in sekundäres Butanol und tertiäre C₅-C₈-Alkohole, wobei die tertiären C₅- und C₇-Alkohole aus dem Isopropanol erhalten werden und die tertiären C₆- und C₈-Alkohole aus dem Ethanol erhalten werden, wobei das resultierende sekundäre Butanol Schritt 1.3 zugeführt wird und wobei das Isopropanol in Gegenwart von Di-tert-amylperoxid mit dem Ethylen reagiert und dadurch in die tertiären C₅- und C₇-Alkohole umgewandelt wird;
Schritt 1.5 Umwandeln der in Schritt 1.4 erhaltenen tertiären C₅-C₈-Alkohole durch Dehydratisierung in Gegenwart eines heterogenen gamma-Al₂O₃-Katalysators bei einer Temperatur von 100 bis 150°C und einem Druck von 0,5 bis 1,5 MPa, bis zum Erreichen der Ausbeute an C₅-C₈-Olefinen von mindestens 99% der Ausgangs-Cs-Cs-Alkohole, in C₅-C₈-Olefine;
Schritt 1.6 Umwandeln eines ersten Teils der in Schritt 1.5 erhaltenen C₅-C₈-Olefine durch Oligomerisation in C₁₀-C₂₄-Olefine, wobei die C₅-C₈-Olefine unter Verwendung eines lonen-Austauscher-Harzes in Form eines Kationentauschers als ein Katalysator bei einer Temperatur von 70 bis 120°C und einem Druck von 1,0 bis 20 MPa oligomerisiert werden;
Schritt 1.7 Umwandeln der in Schritt 1.6 erhaltenen C₁₀-C₂₄-Olefine durch Hydrierung unter Verwendung des in Schritt 1.1 erhaltenen Wasserstoffs in C₁₀-C₂₄-Paraffine, wobei die C₁₀-C₂₄-Olefine in Gegenwart eines heterogenen Katalysators enthaltend NiO, CuO und Cr₂O₃ in einem Molverhältnis von 1:1:1 bei einer Temperatur von 150 bis 200°C und einem Druck von 4,5 bis 5,0 MPa hydriert werden;
Schritt 1.8 Umwandeln des in Schritt 1.2 erhaltenen Synthesegases, des in Schritt 1.3 erhaltenen Ethylens oder des in Schritt 1.3 erhaltenen Propylens und des in Schritt 1.1 erhaltenen Acetaldehyds durch Hydroformylierung und Aldol-Kondensation in ein Gemisch aus C₃-C₄-Aldehyden und Cs-Cs-Aldolen, wobei das Gemisch an C₃-C₄-Aldehyden und C₅-C₈-Aldolen danach hydriert wird, um C₃-C₈-Alkohole zu erhalten, wobei die Alkohole Schritt 1.3 zugeführt werden, um C₃-C₈-Olefine zu erhalten, wobei das Acetaldehyd Cs-Alkohol erzeugt, das Ethylen aus Schritt 1.3 C₃- und C₆-Alkohole erzeugt und das Propylen aus Schritt 1.3 C₄- und C₈-Alkohole erzeugt; und wobei das Ethylen und das Propylen aus Schritt 1.3 C₇-Alkohol erzeugen;
wobei das Ethylen oder Propylen in der Hydroformylierungsreaktion bei einer Temperatur von 70 bis 90°C und einem Druck von 1,0 bis 5,0 MPa in Gegenwart eines wasserlöslichen Rh-Katalysators mit einer Metallkonzentration, bezogen auf die wässrige Phase, von 30 ppm bis 50 ppm in Propanal oder n-Butanal und Isobutanal umgewandelt werden,
danach ein Gemisch aus Propanal, n-Butanal und in Schritt 1.1 erhaltenem Acetaldehyd durch Kreuz-Aldol-Kondensation bei einer Temperatur von 100 bis 150°C und einem Druck von 0,5 bis 1,0 MPa in Gegenwart eines heterogenen Katalysators enthaltend mindestens 93% an ZSM-5-Zeolith, modifiziert mit 3,5 bis 7,0% Zn, oder eines körnigen Katalysators enthaltend mindestens 95% an ZSM-5-Zeolith, modifiziert mit 3,5 bis 5,0% Zn und 0,1 bis 1,5% Ce, in ein Gemisch aus C₃-C₄-Aldehyden und C₅-C₈-Aldolen umgewandelt wird, danach das Gemisch aus C₃-C₄-Aldehyden und C₅-C₈-Aldolen bei einer Temperatur von 150 bis 200°C und einem Druck von 4,5 bis 5,0 MPa in Gegenwart eines heterogenen Katalysator enthaltend NiO, CuO und Cr₂O₃ in einem Molverhältnis von 1:1:1 hydriert wird, um das Gemisch aus C₃-C₈-Alkoholen zu erhalten,
wobei das Ethylen oder Propylen in der Hydroformylierungsreaktion bei einer Temperatur von 120 bis 140°C und einem Druck von 2,0 bis 5,0 MPa in Gegenwart eines wasserlöslichen Co-Katalysators mit einer Co-Metallkonzentration bezogen auf die wässrige Phase von 0,1% bis 1,0% in Propanal, 2-Methylpentenal und 2-Methylpentanal oder in n-Butanal, 2-Ethylhexenal, 2-Ethylhexanal und Isobutanal umgewandelt werden, und
danach das Gemisch aus C₃-C₄-Aldehyden und C₆-C₈-Aldolen bei einer Temperatur von 150 bis 200°C und einem Druck von 4,5 bis 5,0 MPa in Gegenwart eines heterogenen Katalysators enthaltend NiO, CuO und Cr₂O₃ in einem Molverhältnis von 1:1:1 hydriert wird, um das Gemisch aus C₃-C₈-Alkoholen zu erhalten;
Schritt 1.9 Umwandeln der C₂-C₈-Olefine aus Schritt 1.3 durch Oligomerisation in C₆-C₂₄-Olefine in Gegenwart eines heterogenen Katalysators enthaltend mindestens 93% an ZSM-5-Zeolith, modifiziert durch 3,5 bis 7,0% Zn, oder eines heterogenen Katalysators enthaltend mindestens 95% an ZSM-5-Zeolith, modifiziert durch 3,5 bis 5,0% Zn und 0,1 bis 1,5% Ce, bei einer Temperatur von 250 bis 350°C und einem Druck von 4,5 bis 5,0 MPa, um die C₆-C₂₄-Olefine zu erhalten, wobei nicht umgesetzte C₂-C₅-Olefine aus der Oligomerisationsreaktion durch Rektifikation von den C₆-C₂₄-Olefinen getrennt werden und Schritt 1.11 zugeführt werden;
Schritt 1.10 Umwandeln der in Schritt 1.9 erhaltenen C₆-C₂₄-Olefine und Wasserstoff durch Hydrierung in C₆-C₂₄-Paraffine;
wobei die Hydrierung der C₆-C₂₄-Olefine in Gegenwart eines heterogenen Katalysators enthaltend NiO, CuO und Cr₂O₃ in einem Molverhältnis von 1:1:1 bei einer Temperatur von 150 bis 200°C und einem Druck von 4,5 bis 5,0 MPa durchgeführt wird, um die C₆-C₂₄-Paraffine zu erhalten;
Schritt 1.11 Umwandeln von nicht umgesetzten C₂-C₅-Olefinen aus Schritt 1.9 und des in Schritt 1.1 erhaltenen Gemischs aus C₂-C₄-Olefinen und C₁-C₄-Paraffinen, durch Aromatisierung in aromatische C₇-C₁₂-Kohlenwasserstoffe, Wasserstoff und ein Gemisch aus C₁-C₄-Paraffinen, wobei die nicht umgesetzten C₂-C₅-Olefine zusammen in einem Gemisch mit C₂-C₄-Olefinen und C₁-C₄-Paraffinen in Gegenwart eines heterogenen Katalysators enthaltend mindestens 93% an ZSM-5-Zeolith, modifiziert durch 3,5 bis 7,0% Zn, bei einer Temperatur von 350 bis 450°C und einem Druck von 0,5 bis 5,0 MPa, aromatisiert werden, wobei ein erster Teil des erzeugten Wasserstoffs Schritt 1.10 zugeführt wird und der verbleibende zweite Teil des erzeugten Wasserstoffs und das C₁-C₄-Paraffingemisch Schritt 1.2 zugeführt werden;
Schritt 1.12 Umwandeln des verbleibenden zweiten Teils des in Schritt 1.5 erhaltenen Gemischs aus C₅-C₈-Olefinen und eines Teils der C₂-C₈-Alkohole aus Schritt 1.3 in C₇-C₁₆-Ether durch Veretherung, welche an einem Gemisch aus den C₅-C₈-Olefinen und den C₂-C₈-Alkoholen unter Verwendung eines lonen-Austauscher-Harzes in Form eines Kationentauschers als einen Katalysator bei einer Temperatur von 70 bis 120°C und einem Druck von 1,5-2,0 MPa durchgeführt wird, um die C₇-C₁₆-Ether zu erhalten; und
Schritt 1.13 Abtrennen der in Schritt 1.7 erhaltenen C₁₀-C₂₄-Paraffine und der in Schritt 1.10 erhaltenen C₆-C₂₄-Paraffine durch Rektifikation in eine C₆-C₁₀-Paraffinfraktion zur Herstellung von Benzin, eine C₁₁-C₁₈-Paraffinfraktion zur Herstellung von Kerosin und eine C₁₉-C₂₄-Paraffinfraktion zur Herstellung von Diesel;
Abtrennen der in Schritt 1.11 erhaltenen aromatischen C₇-C₁₂-Kohlenwasserstoffe durch Rektifikation in eine aromatische C₇-C₈-Kohlenwasserstofffraktion zur Herstellung von Benzin und eine aromatische C₉-C₁₂-Kohlenwasserstofffraktion zur Herstellung von Kerosin; und
Abtrennen der in Schritt 1.12 erhaltenen C₇-C₁₆-Ether durch Rektifikation in eine C₇-C₁₀-Etherfraktion zur Herstellung von Benzin und eine C₁₁-C₁₆-Etherfraktion zur Herstellung von Diesel,
und Mischen ausgewählter Fraktionen davon in einen Motorentreibstoff, ausgewählt aus Benzin, Kerosin und Diesel;
wobei die C₆-C₂₄-Paraffine rektifiziert werden, um die C₆-C₁₀-Paraffinfraktion zur Herstellung von Benzin, die C₁₁-C₁₈-Paraffinfraktion zur Herstellung von Kerosin und die C₁₉-C₂₄-Paraffinfraktion zur Herstellung von Diesel zu isolieren; und
wobei die C₁₀-C₂₄-Paraffine rektifiziert werden, um die C₁₁-C₁₈-Paraffinfraktion zur Herstellung von Kerosin und die C₁₉-C₂₄-Paraffinfraktion zur Herstellung von Diesel zu isolieren und die verbleibenden C₁₀-Paraffine mit der in Schritt 1.13 durch Rektifikation der C₆-C₂₄-Paraffine erhaltenen C₆-C₁₀-Paraffinfraktion zur Herstellung von Benzin gemischt werden.

2. Das Verfahren zur Herstellung von Motorentreibstoff aus Ethanol gemäß Anspruch 1, wobei in Schritt 1.4 das nicht umgesetzte Ethanol aus Schritt 1.1 in Gegenwart von Di-tert-butylperoxid oder Di-tert-amylperoxid unter einem Druck von P = 1,0 bis 5,0 MPa und einer Temperatur von 100 bis 130°C mit dem Ethylen umgesetzt wird, um sec-Butanol zu erhalten, welches dann mit Ethylen umgesetzt wird und in die tertiären C₆- und C₈-Alkohole umgewandelt wird.

3. Das Verfahren zur Herstellung von Motorentreibstoff aus Ethanol gemäß Anspruch 1, wobei Natriumsalze der Triphenylphosphinsulfonsäure, nämlich: vom Natriumsalz der Triphenylphosphin-3-sulfonsäure bis zum Trinatriumsalz derTriphenylphosphin-3,3',3"-trisulfonsäure in einem Gewichtsverhältnis (10-30):1 zu Rh-Metall, als ein Ligand in Schritt 1.8 bei der Hydroformylierung des Ethylens oder Propylens verwendet werden, um den wasserlöslichen Katalysator zu erhalten.

4. Das Verfahren zur Herstellung von Motorentreibstoff aus Ethanol gemäß Anspruch 3, wobei, um die Reaktionsgeschwindigkeit und die Ausbeute von Isobutanal in Schritt 1.8 zu erhöhen, die Hydroformylierung von Propylen durch Synthesegas in Gegenwart eines wasserlöslichen Rh-Katalysators, welcher gemäß Anspruch 3 hergestellt wurde, durchgeführt wird; C₂-C₃-Alkohole diesem Katalysator in einem Volumenverhältnis H₂O:(C₂-C₃) = (0,95-0,65):(0,05-0,35) zugeführt werden, während das Verhältnis von in dem Reaktionsmedium erhaltenem Butanal und Isobutanal im Bereich (n-C₄H₈O):(iso-C₄H₈O) = (2-3):1 liegt.

5. Das Verfahren zur Herstellung von Motorentreibstoff aus Ethanol gemäß Anspruch 1, wobei in Schritt 1.8 Natriumsalze der Triphenylphosphinsulfonsäure, nämlich: vom Natriumsalz der Triphenylphosphin-3-sulfonsäure bis zum Trinatriumsalz der Triphenylphosphin-3,3',3"-trisulfonsäure als ein Ligand in einem Gewichtsverhältnis (1-30):1 zu Co-Metall bei der Hydroformylierung von Ethylen oder Propylen verwendet werden, um den wasserlöslichen Co-Katalysator zu erhalten.

6. Das Verfahren zur Herstellung von Motorentreibstoff aus Ethanol gemäß Anspruch 5, wobei, um die Reaktionsgeschwindigkeit und die Ausbeute von Isobutanal in Schritt 1.8 zu erhöhen, bei der Hydroformylierung von Propylen C₂-C₃-Alkohole dem Katalysator in einem Volumenverhältnis H₂O:(C₂-C₃) = (0,95-0,5):(0,05-0,5) zugeführt werden, wobei das Verhältnis von in dem Reaktionsmedium erhaltenem Butanal und Isobutanal im Bereich von (n-C₄H₈O):(iso-C₄H₈O) = (2-3):1 liegt.

7. Das Verfahren zur Herstellung von Motorentreibstoff aus Ethanol gemäß einem der vorhergehenden Ansprüche, wobei in Schritt 1.6 das lonen-Austauscher-Harz in Form eines Kationentauschers Amberlite 15 ist.

8. Das Verfahren zur Herstellung von Motorentreibstoff aus Ethanol gemäß Anspruch 1, wobei in Schritt 1.13, um Kerosin zu erhalten, das den Anforderungen der geltenden Norm Jet A-1 vollständig entspricht, C₁₁-C₁₈-Paraffine aus dem in Schritt 1.10 erhaltenen Gemisch aus C₆-C₂₄-Paraffinen isoliert werden, C₁₁-C₁₈-Paraffine aus dem in Schritt 1.7 erhaltenen Gemisch aus C₁₀-C₂₄-Paraffinen isoliert werden und aromatische C₉-C₁₂-Kohlenwasserstoffe aus dem in Schritt 1.11 erhaltenen aromatischen C₇-C₁₂-Kohlenwasserstoffgemisch isoliert werden, und die isolierten C₁₁-C₁₈-Paraffine und die isolierten aromatischen C₉-C₁₂-Kohlenwasserstoffe gemischt werden, damit die Konzentration von aromatischen C₉-C₁₂-Kohlenwasserstoffen im Bereich von 8 bis 25 Vol.-% liegt und damit das resultierende Kerosin mindestens 100 verschiedene Kohlenwasserstoffe, und vorzugsweise 150verschiedene Kohlenwasserstoffe, enthalten wird und einen Rauchpunkt von mindestens 30 mm und einen Gefrierpunkt von maximal minus 80°C aufweisen wird.

## Revendications

1. Procédé de production à partir d'éthanol d'un carburant moteur sélectionné parmi l'essence, le kérosène et le diésel, comprenant les étapes interconnectées suivantes :
étape 1.1 conversion d'un mélange d'éthanol et de 25 à 35 % en volume d'eau dudit mélange en :
- isopropanol et 3-pentanol ;
- acétaldéhyde ;
- un mélange de paraffines en C₁ à C₄ et d'oléfines en C₂ à C₄ ;
- un mélange de dioxyde de carbone et d'hydrogène ;
dans lequel le mélange d'éthanol et d'eau à une pression de 0,5 à 1,5 MPa et une température de 500 à 515 °C est mis en contact avec un catalyseur hétérogène constitué des oxydes de métaux suivants : ZnO 60 à 63 % en masse, CeOz 1 à 6 % en masse, MgO 12 à 18 % en masse ; et Al₂O₃ 13 à 23 % en masse, dans lequel le mélange d'éthanol et d'eau est fourni au catalyseur à une vitesse spatiale de 0,5 à 0,9 hr⁻¹, produisant ainsi, de l'acétone, et de la diéthyl cétone, dans lequel l'acétone et la diéthyl cétone obtenues sont isolées du mélange réactionnel et hydrogénées à une température de 100 à 150 °C et une pression de 0,5 à 0,9 MPa en présence d'un catalyseur comprenant du CuO et du Cr₂O₃ en un rapport molaire de 1/1, par l'hydrogène obtenu du mélange d'éthanol et d'eau, résultant ainsi en l'isopropanol et le 3-pentanol ;
étape 1.2 conversion du mélange de dioxyde de carbone et d'hydrogène, obtenu à l'étape 1-1, d'hydrogène supplémentaire, et d'un mélange de paraffines en C₁ à C₄ en gaz de synthèse ; dans lequel le mélange de dioxyde de carbone et d'hydrogène est converti en eau et en monoxyde de carbone en présence d'un catalyseur contenant de 5 à 10 % de Cu supporté par de l'Al₂O₃, à une température de 550 à 600 °C et une pression de 1,0 à 5,0 MPa, ou, en un mélange de monoxyde de carbone et d'hydrogène en utilisant un catalyseur contenant du NiO, du CuO et du Cr₂O₃ en un rapport molaire de 1/1/1, à une température de 900 à 1000 °C et une pression de 0,1 à 0,5 MPa ;
étape 1.3 conversion par déshydratation en présence de gamma Al₂O₃ dans un réacteur à écoulement continu à une température de 350 à 450 °C et à une pression de 0,5 à 3,5 MPa d'éthanol, et d'alcools en C₃ à C₈, y compris un alcool en C₅ obtenu de l'étape 1.1 en :
- oléfines en C₂ à C₈, y compris de l'éthylène et du propylène ;
étape 1.4 conversion à une pression de P = 1,0 à 5,0 MPa et une température de 100 à 130 °C d'un mélange d'éthanol n'ayant pas réagi de l'étape 1.1, d'isopropanol obtenu à l'étape 1.1, d'éthylène obtenu à l'étape 1.3, en utilisant une réaction de télomérisation dans du butanol secondaire et des alcools tertiaires en C₅ à C₈, les alcools tertiaires en C₅ et C₇ étant obtenus de l'isopropanol, et les alcools tertiaires en C₆, et C₈ étant obtenus de l'éthanol, dans lequel le butanol secondaire résultant est envoyé à l'étape 1.3, et dans lequel l'isopropanol réagit en présence de peroxyde de di-tert-amyle avec l'éthylène et est ainsi converti en les alcools tertiaires en C₅ et C₇ ;
étape 1.5 conversion des alcools tertiaires en C₅ à C₈, obtenus à l'étape 1.4 par déshydratation en présence d'un catalyseur hétérogène gamma Al₂O₃ à une température de 100 à 150 °C et une pression de 0,5 à 1,5 MPa jusqu'à atteindre le rendement en oléfines en C₅ à C₈ d'au moins 99 % de la source d'alcools en C₅ à C₈ en oléfines en C₅ à C₈ ;
étape 1.6 conversion d'une première partie des oléfines en C₅ à C₈, obtenues à l'étape 1.5, par oligomérisation en oléfines en C₁₀ à C₂₄, dans lequel les oléfines en C₅ à C₈ sont oligomérisées en utilisant une résine échangeuse d'ions sous la forme d'un échangeur de cations en tant que catalyseur, à une température de 70 à 120 °C et une pression de 1,0 à 2,0 MPa ;
étape 1.7 conversion des oléfines en C₁₀ à C₂₄, obtenues à l'étape 1.6, par hydrogénation en utilisant l'hydrogène obtenu à l'étape 1.1, en paraffines en C₁₀ à C₂₄, dans lequel les oléfines en C₁₀ à C₂₄ sont hydrogénées en présence d'un catalyseur hétérogène contenant du NiO, du CuO et du Cr₂O₃ en un rapport molaire de 1/1/1 à une température de 150 à 200 °C et une pression de 4,5 à 5,0 MPa ;
étape 1.8 conversion du gaz de synthèse, obtenu à l'étape 1.2, de l'éthylène obtenu à l'étape 1.3, ou du propylène obtenu à l'étape 1.3, et de l'acétaldéhyde, obtenu à l'étape 1.1, par hydroformylation et condensation d'aldols en un mélange d'aldéhydes en C₃ à C₄ et d'aldols en C₅ à C₈, ledit mélange d'aldéhydes en C₃ à C₄ et d'aldols en C₅ à C₈ est après cela hydrogéné pour obtenir des alcools en C₃ à C₈, lesquels alcools sont envoyés à l'étape 1.3 pour obtenir des oléfines en C₃ à C₈, dans lequel l'acétaldéhyde produit un alcool en C₅, l'éthylène de l'étape 1.3 produit des alcools en C₃ et C₆, et le propylène de l'étape 1.3 produit des alcools en C₄ et C₈ ; et
dans lequel l'éthylène et le propylène de l'étape 1.3 produisent un alcool en C₇ ;
dans lequel l'éthylène ou le propylène est converti dans la réaction d'hydroformylation à une température de 70 à 90 °C et une pression de 1,0 à 5,0 MPa en présence un catalyseur au Rh soluble dans l'eau, avec une concentration de métal par rapport à la phase aqueuse de 30 ppm à 50 ppm, en propanal ou n-butanal et isobutanal,
après cela un mélange de propanal, de n-butanal, et d'acétaldéhyde obtenu à l'étape 1.1, est converti par condensation d'aldols croisée à une température de 100 à 150 °C et une pression de 0,5 à 1,0 MPa en présence d'un catalyseur hétérogène contenant au moins 93 % de zéolite ZSM-5 modifiée par 3,5 à 7,0 % de Zn, ou un catalyseur granulaire contenant au moins 95 % de zéolite ZSM-5 modifiée par 3,5 à 5,0 % de Zn et 0,1 à 1,5 % de Ce en un mélange d'aldéhydes en C₃ à C₄ et d'aldols en C₅ à C₈, après cela le mélange d'aldéhydes en C₃ à C₄ et d'aldols en C₅ à C₈ est hydrogéné à une température de 150 à 200 °C et une pression de 4,5 à 5,0 MPa en présence d'un catalyseur hétérogène contenant du Nio, du CuO et du CR₂O₃ en un rapport molaire de 1/1/1 pour obtenir le mélange d'alcools en C₃ à C₈,
dans lequel l'éthylène ou le propylène est converti dans la réaction d'hydroformylation à une température de 120 à 140 °C et une pression de 2,0 à 5,0 MPa en présence d'un catalyseur au Co soluble dans l'eau, avec une concentration de métal Co par rapport à la phase aqueuse de 0,1 % à 1,0 %, en propanal, en 2-méthylpanténal et 2-méthylpantanal ou en n-butanal ,2-éthylhexenal, 2-éthylhexanal et isobutanal, et
après cela le mélange d'aldéhydes en C₃ à C₄ et d'aldols en C₆ à C₈ à une température de 150 à 200 °C et une pression de 4,5 à 5,0 MPa est en présence d'un catalyseur hétérogène contenant du Nio, du CuO et du CR₂O₃ en un rapport molaire de 1/1/1 hydrogéné pour obtenir le mélange d'alcools en C₃ à C₈ ;
étape 1.9 conversion des oléfines en C₂ à C₈ de l'étape 1.3 par oligomérisation en oléfines en C₆ à C₂₄ en présence d'un catalyseur hétérogène contenant au moins 93 % de zéolite ZSM-5 modifiée par 3,5 à 7,0 % de Zn, ou un catalyseur hétérogène contenant au moins 95 % de zéolite ZSM-5 modifiée par 3,5 à 5,0 % de Zn et 0,1 à 1,5 % de Ce à une température de 250 à 350 °C et une pression de 4,5 à 5,0 MPa pour obtenir des oléfines en C₆ à C₂₄, dans lequel les oléfines en C₂ à C₅ de la réaction d'oligomérisation sont séparées par rectification à partir des oléfines en C₆ à C₂₄ et envoyées à l'étape 1.11 ;
étape 1.10 conversion des oléfines en C₆ à C₂₄, obtenues à l'étape 1.9, et d'hydrogène, par hydrogénation en paraffines en C₆ à C₂₄ ;
dans lequel l'hydrogénation des oléfines en C₆ à C₂₄ est réalisée en présence d'un catalyseur hétérogène contenant du Nio, du CuO et du CR₂O₃ en un rapport molaire de 1/1/1, à une température de 150 à 200 °C et une pression de 4,5 à 5,0 MPa pour obtenir les paraffines en C₆ à C₂₄ ;
étape 1.11 conversion des oléfines en C₂ à C₅ n'ayant pas réagi de l'étape 1.9, et du mélange d'oléfines en C₂ à C₄ et de paraffines en C₂ à C₄, obtenu à l'étape 1.1, par aromatisation en hydrocarbures aromatiques en C₇ à C₁₂, en hydrogène et en un mélange de paraffines en C₁ à C₄, dans lequel les oléfines en C₂ à C₅ n'ayant pas réagi sont aromatisées conjointement à un mélange avec des oléfines en C₂ à C₄ et des paraffines en C₁ à C₄, en présence d'un catalyseur hétérogène contenant au moins 93 % de zéolite ZSM-5 modifiée par 3,5 à 7,0 % de Zn à une température de 350 à 450 °C et une pression de 0,5 à 5,0 MPa, dans lequel une première partie de l'hydrogène produit est envoyée à l'étape 1.10, et la deuxième partie restante de l'hydrogène produit et du mélange de paraffines en C₁ à C₄ sont envoyés à l'étape 1.2 ;
étape 1.12 conversion de la deuxième partie restante du mélange d'oléfines en C₅ à C₈, obtenu à l'étape 1.5, et d'une partie des alcools en C₂ à C₈ de l'étape 1.3, en éthers en C₇ à 16 par éthérification réalisée sur un mélange des oléfines en C₅ à C₈ et des alcools en C₂ à C₈ en utilisant en tant que catalyseur une résine échangeuse d'ions sous la forme d'un échangeur de cations à une température de 70 à 120 °C et une pression de 1,5 à 2,0 MPa pour donner les éthers en C₇ à C₁₆ ; et
étape 1.13 séparation par rectification des paraffines en C₁₀ à C₂₄, obtenues à l'étape 1.7, et des paraffines en C₆ à C₂₄, obtenues à l'étape 1.10, en une fraction paraffines en C₆ à C₁₀ pour produire de l'essence, une fraction paraffine en C₁₁ à C₁₈ pour produire du kérosène, et une fraction paraffine en C₁₉ à C₂₄ pour produire du dièse! ;
séparation par rectification des hydrocarbures aromatiques en C₇ à C₁₂, obtenus à l'étape 1.11, en une fraction hydrocarbures aromatiques en C₇ à C₈ pour produire de l'essence et une fraction hydrocarbures aromatiques en C₉ à C₁₂ pour produire du kérosène ; et
séparation par rectification des éthers en C₇ à C₁₆, obtenus à l'étape 1.12, en une fraction éther en C₇ à C₁₀ pour produire de l'essence et une fraction éther en C₁₁ à C₁₆ pour produire du dièse! ;
et mélange de fractions sélectionnées de celui-ci, dans un carburant moteur sélectionné parmi l'essence, le kérosène, et le dièse! ;
dans lequel les paraffines en C₆ à C₂₄ sont rectifiées pour isoler la fraction paraffines en C₆ à C₁₀ pour produire de l'essence, la fraction paraffines en C₁₁ à C₁₈ pour produire du kérosène, et la fraction paraffines en C₁₉ à C₂₄ pour produire du diesel ; et,
dans lequel les paraffines en C₁₀ à C₂₄ sont rectifiées pour isoler la fraction paraffines en C₁₁ à C₁₈ pour produire du kérosène, et la fraction paraffines en C₁₉ à C₂₄ pour produire du diesel, et les paraffines restantes en C₁₀ sont mélangées avec la fraction paraffines en C₆ à C₁₀ pour produire de l'essence, obtenue à l'étape 1.13 par rectification des paraffines en C₆ à C₂₄.

2. Procédé de production d'un carburant moteur à partir d'éthanol selon la revendication 1, dans lequel, à l'étape 1.4, l'éthanol n'ayant pas réagi de l'étape 1.1 réagit en présence de peroxyde de di-tert-butyle ou de peroxyde de di-tert-amyle sous une pression de P = 1,0 à 5,0 MPa et à une température de 100 à 130 °C avec de l'éthylène pour obtenir du sec-butanol, qui est alors mis à réagir avec de l'éthylène et est converti en alcools tertiaires en C₆ et C₈.

3. Procédé de production de carburant moteur à partir d'éthanol selon la revendication 1, dans lequel des sels de sodium de l'acide triphénylphosphine-sulfonique, précisément : du sel de sodium de l'acide triphénylphosphine-3-sulfonique au sel de trisodium de l'acide triphénylphosphine-3,3',3"-trisulfonique en un rapport de poids de (10 à 30)/1 sur le Rh métallique sont utilisés en tant que ligand à l'étape 1.8 dans l'hydroformylation de l'éthylène ou du propylène pour obtenir le catalyseur soluble dans l'eau.

4. Procédé de production de carburant moteur à partir d'éthanol selon la revendication 3, dans lequel, de manière à augmenter la vitesse de réaction et le rendement en isobutanal à l'étape 1.8, l'hydroformylation du propylène par le gaz de synthèse est réalisée en présence d'un catalyseur au Rh soluble dans l'eau préparé selon la revendication 3 ; des alcools en C₂ à C₃ sont ajoutés audit catalyseur en un rapport de volume H₂O/(C₂ à C₃) = (0,95 à 0,65)/(0,05 à 0,35), alors que le rapport du butanal sur l'isobutanal obtenu dans le milieu de réaction se trouve dans la plage (n-C₄H₈O)/(iso-C₄H₈O) = (2-3)/1.

5. Procédé de production de carburant moteur à partir d'éthanol selon la revendication 1, dans lequel, à l'étape 1.8, des sels de sodium de l'acide triphénylphosphine-sulfonique, précisément : du sel de sodium de l'acide triphénylphosphine-3-sulfonique au sel de trisodium de l'acide triphénylphosphine-3,3',3"-trisulfonique sont utilisés en tant que ligand en un rapport de poids de (1à 30)/1 sur le métal Co dans l'hydroformylation de l'éthylène ou du propylène pour obtenir le catalyseur au Co soluble dans l'eau.

6. Procédé de production d'un carburant moteur à partir d'éthanol selon la revendication 5, dans lequel, de manière à augmenter la vitesse de réaction et le rendement en isobutanal à l'étape 1.8, dans l'hydroformylation du propylène, des alcools en C₂ à C₃ sont ajoutés au catalyseur en un rapport de volume H₂O/(C₂ à C₃) = (0,95 à 0,5)/(0,05 à 0,5), dans lequel que le rapport du butanal sur l'isobutanal obtenu dans le milieu de réaction se trouve dans la plage (n-C₄H₈O)/(iso-C₄H₈O) = (2-3)/1.

7. Procédé de production d'un carburant moteur à partir d'éthanol selon l'une quelconque des revendications précédentes, dans lequel à l'étape 1.6 la résine échangeuse d'ions sous la forme d'une résine échangeuse de cations est Amberlite 15.

8. Procédé de production d'un carburant moteur à partir d'éthanol selon la revendication 1, dans lequel, à l'étape 1.13, de manière à obtenir du kérosène complètement conforme aux exigences de la norme Jet A-1 actuelle, les paraffines en C₁₁ à C₁₈ sont isolées du mélange de paraffines en C₆ à C₂₄ obtenu à l'étape 1.10, les paraffines en C₁₁ à C₁₈ sont isolées du mélange de paraffines en C₁₀ à C₂₄ obtenu à l'étape 1.7, et les hydrocarbures aromatiques en C₉ à C₁₂ sont isolés du mélange d'hydrocarbures aromatiques en C₇ à C₁₂ obtenu à l'étape 1.11, et les paraffines isolées en C₁₁ à C₁₈ et les hydrocarbures aromatiques isolés en C₉ à C₁₂ sont mélangés de manière à ce que la concentration en hydrocarbures aromatiques en C₉ à C₁₂ se trouve dans la plage de 8 à 25 % en volume, et ainsi à ce que le kérosène résultant contienne au moins 100 hydrocarbures différents, et de préférence 150 hydrocarbures différents, ait un point de fumée d'un minimum de 30 mm, et un point de congélation d'un maximum de -80 °C.
